(19) Europäisches Patentamt
European Patent Office
Office européen des brevets

(11) EP 2 330 158 A1

(12) **EUROPEAN PATENT APPLICATION**
published in accordance with Art. 153(4) EPC

(43) Date of publication:
**08.06.2011 Bulletin 2011/23**

(21) Application number: **09813072.7**

(22) Date of filing: **08.09.2009**

(51) Int Cl.:
***C08L 101/00*** *(2006.01)* ***C08K 5/00*** *(2006.01)*
***C08K 5/357*** *(2006.01)* ***C08K 5/45*** *(2006.01)*
***C09K 3/00*** *(2006.01)* ***F21V 3/04*** *(2006.01)*
***F21V 9/06*** *(2006.01)* ***C07D 249/20*** *(2006.01)*
***C07D 251/22*** *(2006.01)* ***C07D 265/22*** *(2006.01)*
***C07D 413/14*** *(2006.01)* ***F21Y 101/00*** *(2006.01)*
***F21Y 103/00*** *(2006.01)*

(86) International application number:
**PCT/JP2009/065691**

(87) International publication number:
**WO 2010/029926 (18.03.2010 Gazette 2010/11)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **10.09.2008 JP 2008232472**

(71) Applicant: **FUJIFILM Corporation**
**Tokyo 106-0031 (JP)**

(72) Inventors:
- **FURUKAWA, Kazushi**
**Odawara-shi**
**Kanagawa 250-0001 (JP)**
- **TAKESHIMA, Youichiro**
**Odawara-shi**
**Kanagawa 250-0001 (JP)**
- **KIMURA, Keizo**
**Odawara-shi**
**Kanagawa 250-0001 (JP)**

(74) Representative: **HOFFMANN EITLE**
**Patent- und Rechtsanwälte**
**Arabellastraße 4**
**81925 München (DE)**

(54) **LIGHTING COVER**

(57) A lighting unit cover, having a resin composition including ultraviolet absorbent A, the ultraviolet absorbent A being a compound represented by formula (1):

Formula (1)

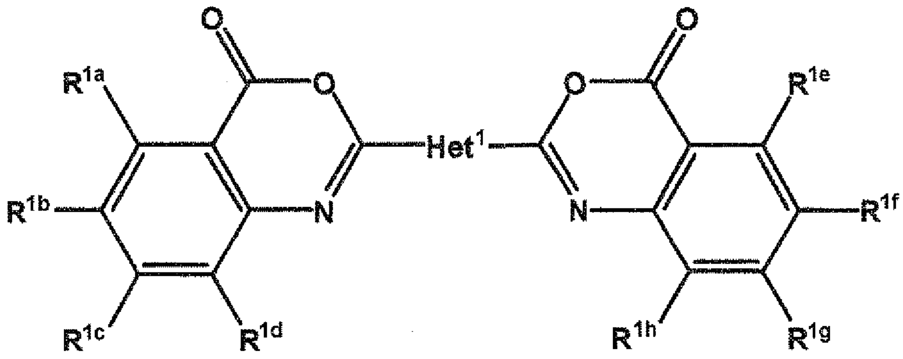

in which, Het[1] represents a bivalent five- or six-membered aromatic heterocyclic residue; the aromatic heterocyclic

EP 2 330 158 A1

residue may have a substitutent; and $R^{1a}$, $R^{1b}$, $R^{1c}$, $R^{1d}$, $R^{1e}$, $R^{1f}$, $R^{1g}$ and $R^{1h}$ each independently represent a hydrogen atom or a monovalent substituent.

{Fig. 1}

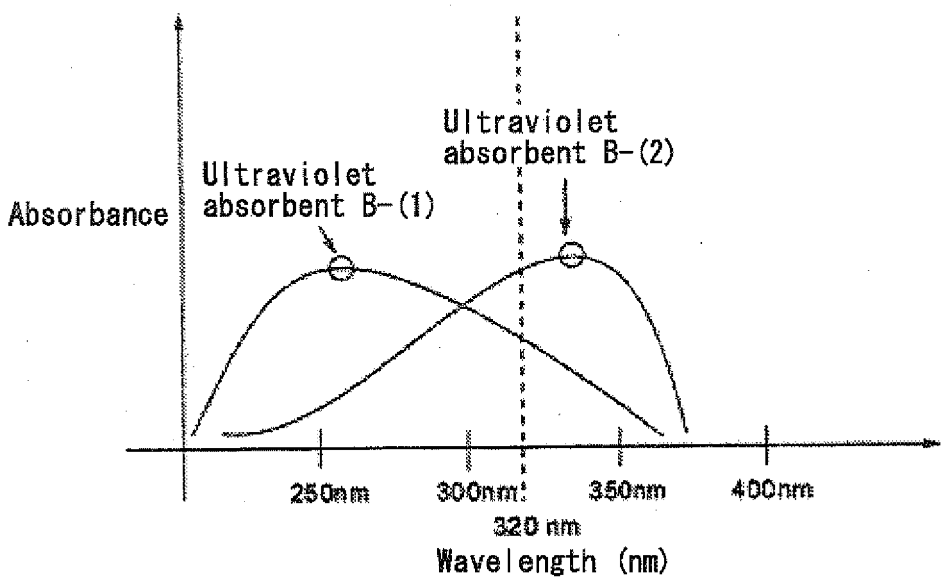

**Description**

TECHNICAL FIELD

The present invention relates to a lighting unit cover for shielding ultraviolet light.

BACKGROUND ART

A lighting unit cover has been conventionally provided, which prevents a lighting unit from attracting an insect by cutting off light with a particular wavelength. For example, since a spectral distribution of light which is apt to attract an insect such as a flying insect is generally considered to have a peak in an ultraviolet region, a lighting unit cover which cuts off light with wavelength up to approximately 380 nm (ultraviolet light) and a lighting unit cover which cuts off light with wavelength within the range from an ultraviolet region to the short-wavelength side of a visible region (up to 450 nm, approximately) are provided (for example, see Patent Literature 1).

In order to provide a capability of cutting off ultraviolet light that is needed for these lighting apparatuses, various ultraviolet absorbents are used, and an inorganic ultraviolet absorbent or an organic ultraviolet absorbent may be used. The inorganic ultraviolet absorbent (see, for example, Patent Literatures 1 to 3) is excellent in durability such as weather resistance and heat resistance. However, an absorption wavelength thereof is determined by a band gap of the compound, and hence it is low in the degree of freedom for selecting compounds. There is hence no inorganic absorbent available that can absorb light in a long-wavelength ultraviolet (UV-A) range of 320 to 400 nm.

In contrast, the organic ultraviolet absorbent is high in the degree of freedom for designing the structure of absorbents, and thus it is possible to obtain ones having various absorption wavelengths by modifying the structure of them. For absorption in the long-wavelength ultraviolet range, it is conceivable either to use an absorbent having the wavelength of maximal absorption in the long-wavelength ultraviolet range or to increase the concentration of absorbent. However, the absorbents, described in, for example, Patent Literatures 4 and 5, have the wavelength of maximal absorption in the long-wavelength ultraviolet range, and they have a defect that they are poor in light fastness, and their absorption capability declines with time. On the other hand, benzophenone- and benzotriazole-based ultraviolet absorbents are relatively excellent in light fastness, and increase in an amount to be used leads to relatively clean blocking of light in the long-wavelength range (see, for example, Patent Literatures 6 and 7). However, there is a problem that coloration of yellow becomes visible as the absorbance in the visible range increases.

Among the lighting apparatuses described above, the lighting unit cover which cuts off light with wavelength up to approximately 380 nm (ultraviolet light) can prevent the lighting unit form attracting the insect by reducing ultraviolet light. However, ultraviolet light in the longer-wavelength region has the effect of attracting the insect, thus it is not satisfactory in terms of the effect of lowering an insect attracting property. In addition, in the lighting unit cover which cuts off light with wavelength within the range from an ultraviolet region to the short-wavelength side of a visible region (up to 450 nm, approximately), an insect's approach is difficult, compared to the lighting apparatus which cuts off ultraviolet light. However, as since the light surely looks yellow, there is a problem that the appearance of the lighting apparatus is unfavorable at the time of lighting, in the case where the lighting unit cover is used for a usual light.

Further, a resin molded article is known, which prevents it from attracting flying insects and has high transparent feeling without yellow tint by effectively cutting off ultraviolet light up to 410 nm, which is a longer wavelength than 380 nm (for example, see Patent Literature 8). However, there is a problem that coloration of yellow occurs by long-term use, and thus, an improvement has been needed.

CITATION LIST

PATENT LITERATURE

Patent Literature 1: JP-A-2001-161253 (Pages 3 to 5, and Fig.1) ("JP-A" means unexamined published Japanese patent application)
Patent Literature 2: JP-A-5-339033
Patent Literature 3: JP-A-5-345639
Patent Literature 4: JP-A-6-56466
Patent Literature 5: JP-A-2003-177235
Patent Literature 6: JP-T-2005-517787 ("JP-T" means published Japanese translation of PCT application)
Patent Literature 7: JP-A-7-285927
Patent Literature 8: JP-A-2005-206830

DISCLOSURE OF INVENTION

TECHNICAL PROBLEM

The present invention addresses to the provision of a lighting unit cover which prevents the gathering of flying insects without damaging the appearance of the lighting unit cover at the time of the lighting unit turned on, and is excellent in light fastness.

The present invention provides the following means:

<1> A lighting unit cover, comprising a resin composition including ultraviolet absorbent A, the ultraviolet absorbent A being a compound represented by formula (1):

Formula (1)

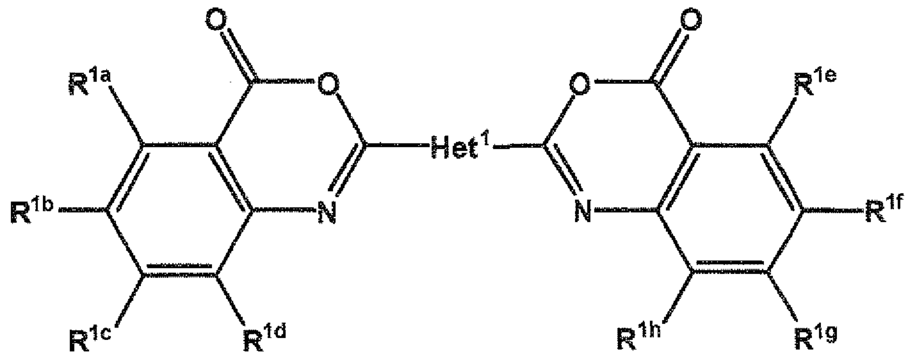

wherein, $Het^1$ represents a bivalent five- or six-membered aromatic heterocyclic residue; the aromatic heterocyclic residue may have a substituent; and $R^{1a}$, $R^{1b}$, $R^{1c}$, $R^{1d}$, $R^{1e}$, $R^{1f}$, $R^{1g}$ and $R^{1h}$ each independently represent a hydrogen atom or a monovalent substituent.

<2> The lighting unit cover described in the above item <1>, wherein the compound represented by formula (1) is a compound represented by formula (2):

Formula (2)

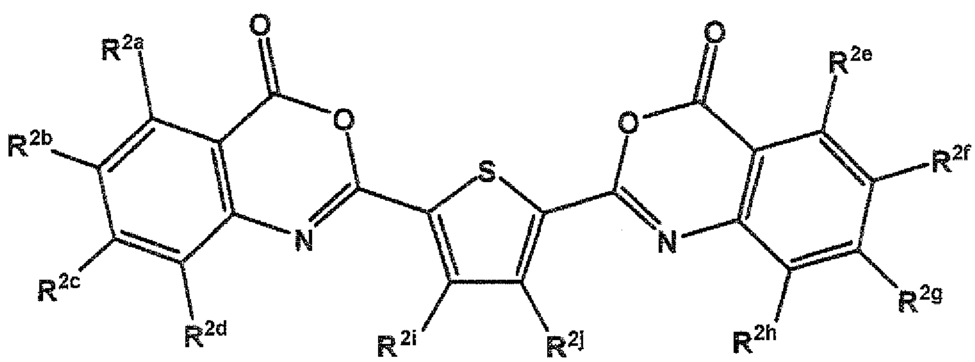

wherein, $R^{2a}$, $R^{2b}$, $R^{2c}$, $R^{2d}$, $R^{2e}$, $R^{2f}$, $R^{2g}$ and $R^{2h}$ have the same meanings as those of $R^{1a}$, $R^{1b}$, $R^{1c}$, $R^{1d}$, $R^{1e}$, $R^{1f}$, $R^{1g}$ and $R^{1h}$ in formula (1), respectively, and $R^{2i}$ and $R^{2j}$ each independently represent a hydrogen atom or a monovalent substituent.

<3> The lighting unit cover described in the above item <1> or <2>,

wherein the resin composition includes ultraviolet absorbent B, and
wherein the ultraviolet absorbent B is a compound, in which an absorbance at 320 nm is 20% or more of an absorbance at a maximum absorption wavelength within a range from 270 to 400 nm and the maximum absorption wavelength is 380 nm or less;
<4> The lighting unit cover described in any one of the above items <1> to <3>, wherein the resin composition contains 0.00001 to 1 mass part of bluing agent D, relative to 100 mass parts of the ultraviolet absorbent A.

<5> The lighting unit cover described in any one of the above items <1> to <4>, wherein the resin composition includes at least one kind of compound C represented by any one of formulae (TS-I) to (TS-V):

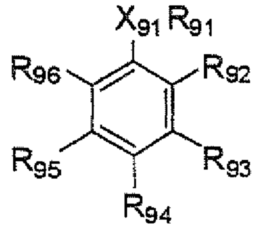
(TS-I)

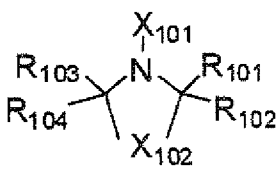
(TS-II)

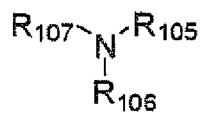
(TS-III)

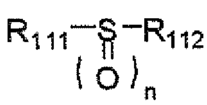
(TS-IV)

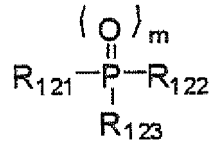
(TS-V)

wherein, in formula (TS-I), $R_{91}$ represents a hydrogen atom, an alkyl group, an alkenyl group, an aryl group, an heterocyclic group, an acyl group, an alkyloxcarbonyl group, an alkenyloxycarbonyl group, an aryloxycarbonyl group, an alkyl sulfonyl group, an arylsulfonyl group, a phosphinotolyl group, a phosphinyl group, or -Si($R_{97}$)($R_{98}$)($R_{99}$), in which $R_{97}$, $R_{98}$ and $R_{99}$, which may be the same as or different from each other, each independently represent an alkyl group, an alkenyl group, an aryl group, an alkoxy group, an alkenyloxy group, or an aryloxy group; -$X_{91}$- represents -O-, -S-, or -N(-$R_{100}$)-, in which $R_{100}$ has the same meaning as that of $R_{91}$; $R_{92}$, $R_{93}$, $R_{94}$, $R_{95}$ and $R_{96}$, which may be the same as or different from each other, each independently represent a hydrogen atom or a substituent; $R_{91}$ and $R_{92}$, $R_{100}$ and $R_{96}$, and $R_{91}$ and $R_{100}$, respectively, may bind to each other to form any of 5- to 7-membered rings; $R_{92}$ and $R_{93}$, and $R_{93}$ and $R_{94}$, respectively, may bind together with each other to form any of 5- to 7-membered rings, a spiro ring, or a bicyclo ring; and all of $R_{91}$, $R_{92}$, $R_{93}$, $R_{94}$, $R_{95}$, $R_{96}$ and $R_{100}$ cannot simultaneously represent a hydrogen atom, respectively, and the total number of carbon atoms of $R_{91}$, $R_{92}$, $R_{93}$, $R_{94}$, $R_{95}$, $R_{96}$ and $R_{100}$ is 10 or more;
wherein, in formula (TS-II), $R_{101}$, $R_{102}$, $R_{103}$ and $R_{104}$ each independently represent a hydrogen atom, an alkyl group, or an alkenyl group; $R_{101}$ and $R_{102}$, and $R_{103}$ and $R_{104}$, respectively, may bind to each other to form any of 5- to 7-membered rings; $X_{101}$ represents a hydrogen atom, an alkyl group, an alkenyl group, an alkyloxy group, an alkenyloxy group, an alkyloxycarbonyl group, an alkenyloxycarbonyl group, an aryloxycarbonyl group, an acyl group, an acyloxy group, an alkyloxycarbonyloxy group, an alkenyloxycarbonyloxy group, an arylaxycarbanyloxy group, an alkylsulfonyl group, an alkenylsulfonyl group, an arylsulfonyl group, an alkylsulfinyl group, an alkenylsulfinyl group, an arylsulfinyl group, a sulfamoyl group, a carbamoyl group, a hydroxy group, or an oxy radical group; and $X_{102}$ represents a group of non-metal atoms necessary for forming any of 5- to 7-membered rings;

wherein, in formula (TS-III), $R_{105}$ and $R_{106}$ each independently represent a hydrogen atom, an aliphatic group, an acyl group, an aliphatic oxycarbonyl group, an aromatic oxycarbonyl group, an aliphatic sulfonyl group, or an aromatic sulfonyl group; $R_{107}$ represents an aliphatic group, an aliphatic oxy group, an aromatic oxy group, an aliphatic thio group, an aromatic thio group, an acyloxy group, an aliphatic oxycarbonyloxy group, an aromatic oxycarbonyloxy group, a substituted amino group, a heterocyclic group, or a hydroxyl group; $R_{105}$ and $R_{106}$, $R_{106}$ and $R_{107}$, and $R_{105}$ and $R_{107}$, respectively, may bind to each other to form any of 5- to 7-membered rings except of forming a 2,2,6,6-tetraalkylpiperidine residue; and both $R_{105}$ and $R_{106}$ are not hydrogen atoms at the same time, and the total number of carbon atoms of $R_{105}$ and $R_{106}$ is 7 or more;
wherein, in formula (TS-IV), $R_{111}$ and $R_{112}$ each independently represent an aliphatic group; $R_{111}$ and $R_{112}$ may bind to each other to form any of 5- to 7-membered rings; n represents 0, 1 or 2; and the total number of carbon atoms of $R_{111}$ and $R_{112}$ is 10 or more; and
wherein, in formula (TS-V), $R_{121}$ and $R_{122}$ each independently represent an aliphatic oxy group or an aromatic oxy group; $R_{123}$ represents an aliphatic group, an aromatic group, an aliphatic oxy group, or an aromatic oxy group; m represents 0 or 1; $R_{121}$ and $R_{122}$, and $R_{121}$ and $R_{123}$, respectively, may bind to each other to form any of 5-to 8-membered rings; and the total number of carbon atoms of $R_{121}$, $R_{122}$ and $R_{123}$ is 10 or more.
<6> The lighting unit cover described in any one of the above items <1> to <5>, wherein a content of the ultraviolet absorbent A in the resin composition is 0.01 to 20 mass parts, relative to 100 mass parts of the resin.
<7> The lighting unit cover described in any one of the above items <3> to <6>, wherein the molar ratio of the ultraviolet absorbent A and the ultraviolet absorbent B in the resin composition is within a range of 1:10 to 10:1.
<8> The lighting unit cover described in any one of Claims 1 to 7, wherein the resin composition includes at least one kind of an acrylic resin, a polycarbonate resin, a polyester resin, and a polyolefin resin.
<9> The lighting unit cover described in any one of Claims 1 to 8, wherein a value obtained by subtracting a smallest transmittance value in 550 nm to 600 nm from a largest transmittance value in 430 nm to 480 nm is 15% or less of the largest transmittance value in 430 nm to 480 nm.
<10> The lighting unit cover described in any one of the above items <1> to <9>, wherein an entire light transmittance is 40% or more.
<11> The lighting unit cover described in any one of the above items <1> to <10>, wherein the cover is adapted to an application for removing light defined in the wavelength that is apt to attract an insect.

ADVANTAGEOUS EFFECTS OF INVENTION

The ultraviolet absorbent used in the present invention can absorb ultraviolet light in the broad wavelength range, and can cut off ultraviolet light up to the long-wavelength range. Further, the ultraviolet absorbent is excellent in the colorless property. Thus, the lighting unit cover of the present invention can prevent the lighting unit from gathering flying insects, without deteriorating the appearance of the lighting cover at the lighting unit turned on, besides excellent in light fastness.
Other and further features and advantages of the invention will appear more fully from the following description, taking the accompanying drawing into consideration.

BRIEF DESCRIPTION OF DRAWINGS

{Fig. 1} Fig. 1 shows preferred absorption spectra of the ultraviolet absorbents B used in the present invention.
{Fig. 2} Fig. 2 is a transmittance spectrum of sample plate 200 produced in Example 2.
{Fig. 3} Fig. 3 is a transmittance spectrum of sample plate 201 produced in Example 2.

DESCRIPTION OF EMBODIMENTS

The lighting unit cover of the present invention characteristically comprises a resin composition containing the ultraviolet absorbent A. The ultraviolet absorbent A is a compound represented by formula (1).
In formula (1), $Het^1$ represents a bivalent five- or six-membered aromatic heterocyclic residue having at least one hetero atom. $Het^1$ may be a condensed ring.
Examples of the hetero atoms include a boron atom, a nitrogen atom, an oxygen atom, a silicon atom, a phosphorus atom, a sulfur atom, a selenium atom, and a tellurium atom, preferably, a nitrogen atom, an oxygen atom, and a sulfur atom, more preferably a nitrogen atom and a sulfur atom, and particularly preferably a sulfur atom. If the ring has two

or more hetero atoms, the hetero atoms may be the same as or different from each other.
Examples of the aromatic heterocycles prepared by adding two hydrogen atoms to a bivalent aromatic heterocyclic residue include pyrrole, pyrazole, imidazole, 1,2,3-triazole, 1,2,4-triazole, pyridine, pyridazine, pyrimidine, pyrazine, 1,3,5-triazine, furan, thiophene, oxazole, isoxazole, thiazole, isothiazole, 1,2,3-oxadiazole, 1,3,4-thiadiazole, and the like. The aromatic heterocycle is preferably pyrrole, pyridine, furan, or thiophene, more preferably pyridine or thiophene, and particularly preferably thiophene. The site of the aromatic heterocycle where the hydrogen atom is abstracted is arbitrary. For example, in the case of a five-membered heterocyclic compound of pyrrole, the binding sites are, for example, 2- and 3-sites, 2- and 4-sites, 2- and 5-sites, 3- and 4-sites, and 3- and 5-sites. In the case of a six-membered heterocyclic compound of pyridine, the binding sites are 2- and 3-sites, 2- and 4-sites, 2- and 5-sites, 2- and 6-sites, 3- and 4-sites, 3- and 5-sites, and 3- and 6-sites.

The aromatic heterocyclic residue may have a substituent. The substituent is, for example, a monovalent substituent (hereinafter, also referred to as substituent R). Examples of the monovalent substituent include a halogen atom (e.g., fluorine atom, chlorine atom, bromine atom, and iodine atom), an alkyl group having 1 to 20 carbon atoms (e.g., methyl and ethyl), an aryl group having 6 to 20 carbon atoms (e.g., phenyl and naphthyl), a cyano group, a carboxyl group, an alkoxycarbonyl group (e.g., methoxycarbonyl), an aryloxycarbonyl group (e.g., phenoxycarbonyl), a substituted or unsubstituted carbamoyl group (e.g., carbamoyl, N-pheylcarbamoyl, and N,N-dimethylcarbamoyl), an alkylcarbonyl group (e.g., acetyl), an arylcarbonyl group (e.g., benzoyl), a nitro group, a substituted or unsubstituted amino group (e.g., amino, dimethylamino, and anilino), an acylamino group (e.g., acetamido and ethoxycarbonylamino), a sulfonamido group (e.g., methane sulfonamide), an imido group (e.g., succinimido and phthalimido), an imino group (e.g., benzylideneamino), a hydroxy group, an alkoxy group having 1 to 20 carbon atoms (e.g., methoxy), an aryloxy group (e.g., phenoxy), an acyloxy group (e.g., acetoxy), an alkylsulfonyloxy group (e.g., methanesulfonyloxy), an arylsulfonyloxy group (e.g., benzenesulfonyloxy), a sulfo group, a substituted or unsubstituted sulfamoyl group (e.g., sulfamoyl and N-phenylsulfamoyl), an alkylthio group (e.g., methylthio), an arylthio group (e.g., phenylthio), an alkylsulfonyl group (e.g., methanesulfonyl), an arylsulfonyl group (e.g., benzenesulfonyl), a heterocyclic group having 6 to 20 carbon atoms (e.g., pyridyl, morpholino), and the like. The substituent may be further substituted, and the multiple substituents, if present, may be the same as or different from each other. The substituents then are, for example, the monovalent substituent R described above. The substituents may bind to each other to form a ring. The substituent is preferably an alkyl group, an alkoxy group, or an aryl group, more preferably an alkyl group or an aryl group, and particularly preferably an alkyl group.

$R^{1a}$, $R^{1b}$, $R^{1c}$, $R^{1d}$, $R^{1e}$, $R^{1f}$, $R^{1g}$ and $R^{1h}$ each independently represent a hydrogen atom or the monovalent substituent R. Any two substituents among $R^{1a}$ to $R^{1d}$ and $R^{1e}$ to $R^{1h}$, respectively, may bind to each other to form a ring, which may further form a condensed ring. $R^{1a}$ to $R^{1h}$ each preferably represent a hydrogen atom, an alkyl group having 10 or less carbon atoms, an alkoxy group having 10 or less carbon atoms, or a hydroxy group, more preferably a hydrogen atom or an alkoxy group having 10 or less carbon atoms, furthermore preferably a hydrogen atom, and particularly preferably, $R^{1a}$ to $R^{1h}$ are all hydrogen atoms.

Further, the compound represented by formula (1) is preferably a compound represented by formula (2). Hereinafter, the compound represented by formula (2) is described in detail.

$R^{2a}$, $R^{2b}$, $R^{2c}$, $R^{2d}$, $R^{2e}$, $R^{2f}$, $R^{2g}$ and $R^{2h}$ in formula (2) each have the same meanings as those of $R^{1a}$, $R^{1b}$, $R^{1c}$, $R^{1d}$, $R^{1e}$, $R^{1f}$, $R^{1g}$ and $R^{1h}$ in formula (1), respectively, and the preferable examples thereof are also the same. $R^{2i}$ and $R^{2j}$ each independently represent a hydrogen atom or a monovalent substituent. The substituent includes the examples of the monovalent substituent R described above. $R^{2i}$ and $R^{2j}$ may bind to each other to form a ring, which may further form a condensed ring. $R^{2j}$ and $R^{2j}$ each preferably represent a hydrogen atom, an alkyl group having 10 or less carbon atoms, an alkoxy group having 10 or less carbon atoms, or a hydroxy group, more preferably a hydrogen atom or an alkoxy group having 10 or less carbon atoms, furthermore preferably a hydrogen atom, and particularly preferably, $R^{2i}$ and $R^{2j}$ both are hydrogen atoms.

The compound represented by formula (1) or (2) may be prepared by an arbitrary method. For example, the compound represented by formula (1) or (2) can be synthesized with reference to the methods described in known patent literatures and non-patent literatures, for example, those described in the Examples of JP-A-2000-264879, p.4, line 43 on left column to line 8 on right column; in the Examples of JP-A-2003-155375, p.4, lines 5 to 30 on right column; "Bioorganic & Medicinal Chemistry", 2000, vol. 8, pp. 2095-2103, "Bioorganic & Medicinal Chemistry Letters", 2003, vol. 13, pp. 4077-4080. For example, exemplified compound (15) can be synthesized by the reaction of 3,5-pyrazole dicarbonyl dichloride with anthranilic acid. Exemplified compound (32) can be synthesized by the reaction of 2,5-thiophenedicarbonyl dichloride with 4,5-dimethoxyanthranilic acid.

Hereinafter, specific examples of the compound represented by formula (1) or (2) are described, but the present invention is not restricted thereby.

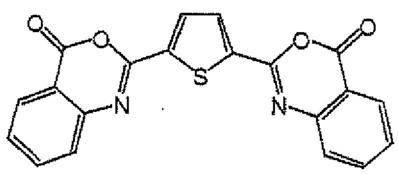
(1)

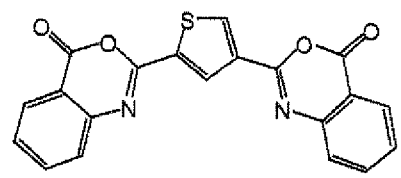
(2)

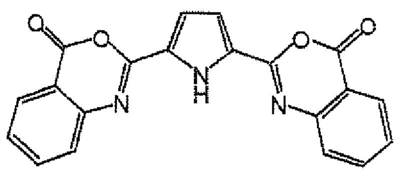
(3)

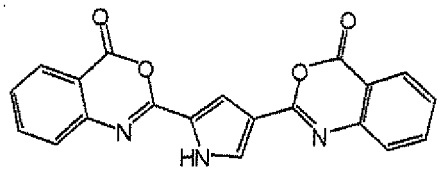
(4)

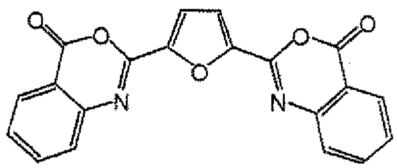
(5)

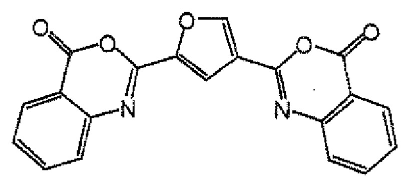
(6)

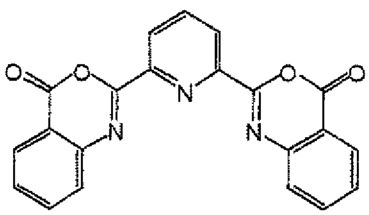
(7)

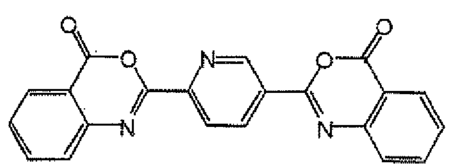
(8)

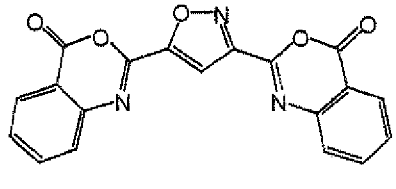
(9)

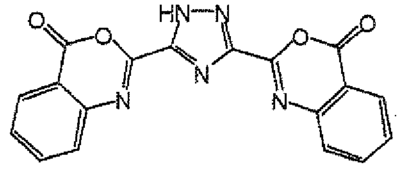
(10)

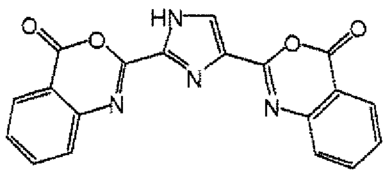

(11)

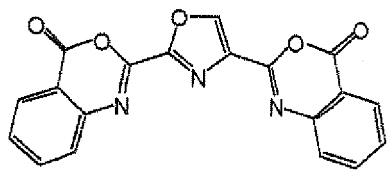

(12)

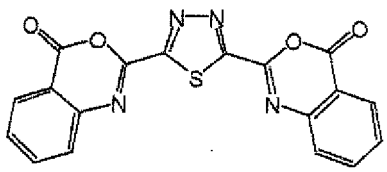

(13)

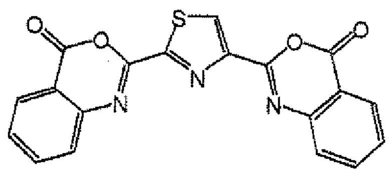

(14)

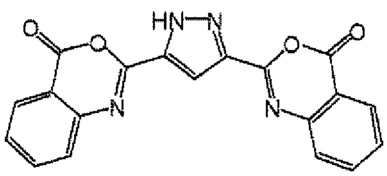

(15)

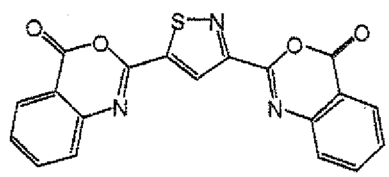

(16)

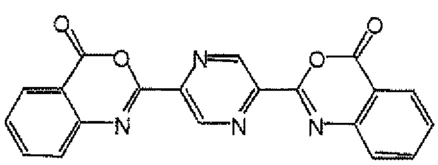

(17)

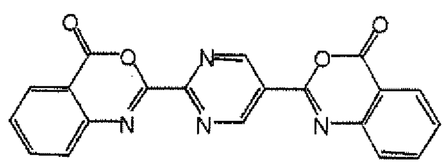

(18)

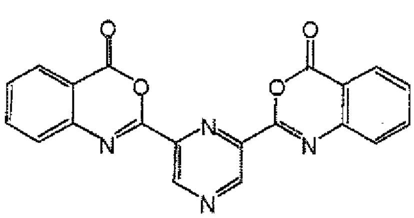

(19)

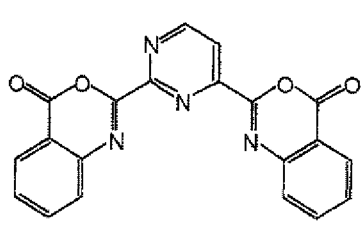

(20)

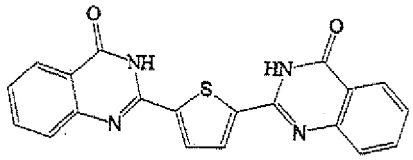 (21) 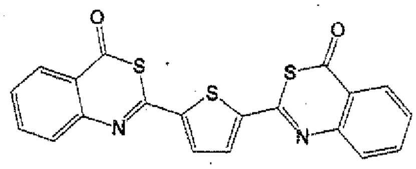 (22)

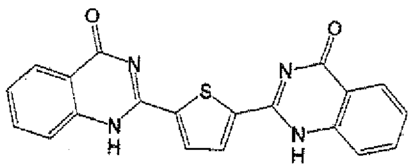 (23) 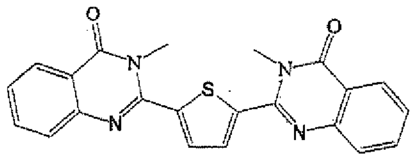 (24)

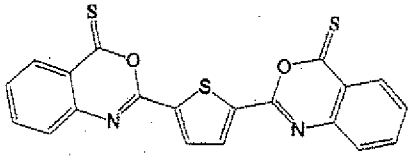 (25) 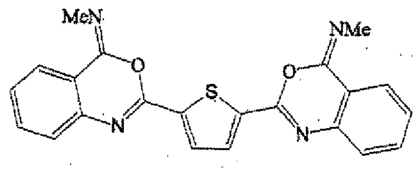 (26)

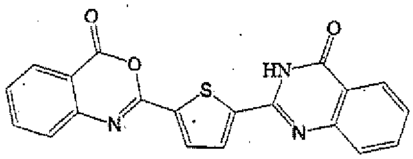 (27) 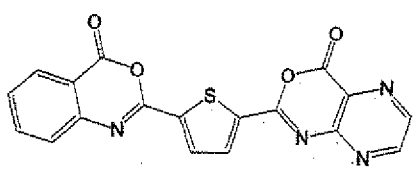 (28)

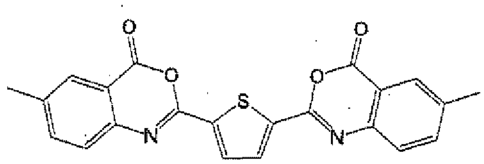 (29) 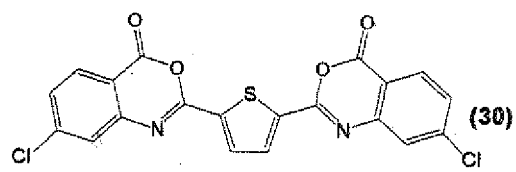 (30)

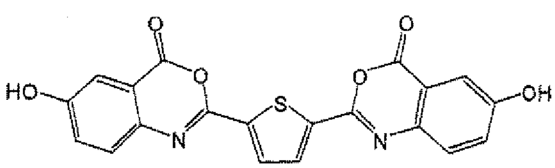 (31)

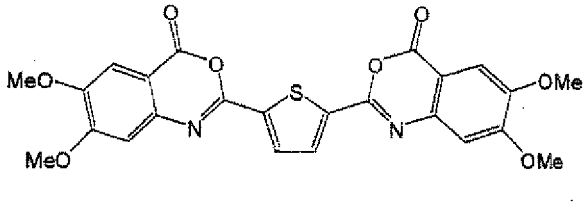

(32)

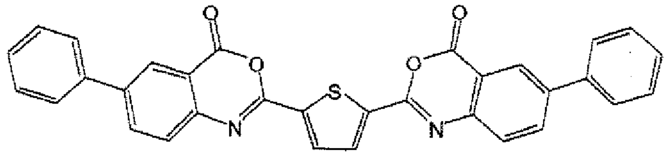

(33)

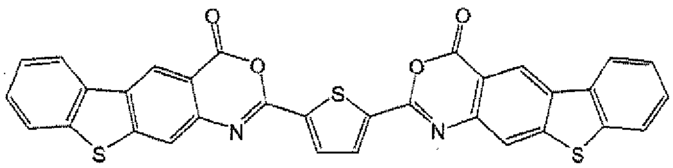

(34)

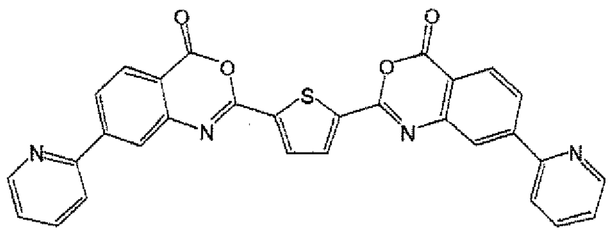

(35)

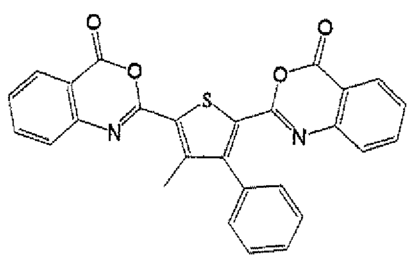

(37)

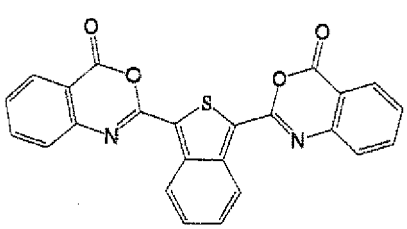

(38)

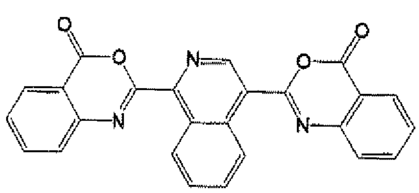

(39)

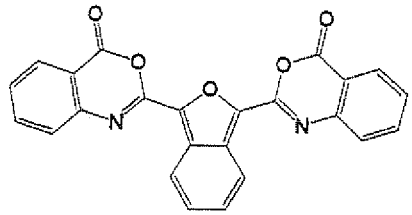 (40) 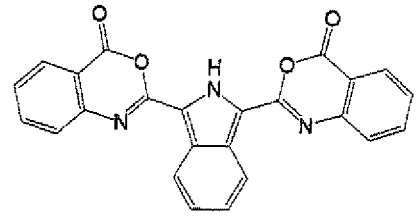 (41)

The compound represented by formula (1) or (2) may have tautomers depending on the structure thereof and the environment where the compound is located. In the present invention, a typical form thereof is described, but the tautomers different from that described in the present invention are also included in the compound used in the present invention.

The compound represented by formula (1) or (2) may contain an isotopic element (for example, $^{2}H$, $^{3}H$, $^{13}C$, $^{15}N$, $^{17}O$, or $^{18}O$).

A polymer having the structure of the compound represented by formula (1) or (2) in its repeating unit can also be favorably used in the present invention. The polymer may be a homopolymer or a copolymer having two or more kinds of repeating units. It may be a copolymer having another repeating unit additionally. Examples of the polymer having an ultraviolet absorbent structure in the repeating unit are described, for example, in JP-B-1-5345 ("JP-B" means examined Japanese patent publication), JP-A-61-189530 and EP Patent No. 27242. The polymer can be prepared with reference to the methods described in these patent literatures.

The ultraviolet absorbent A, which is a compound represented by formula (1) or (2), is preferably used as a long-wavelength ultraviolet absorbent. The wavelength of the largest absorption is preferably within the range from 450 to 350 nm, more preferably 410 to 350 nm, particularly preferably 390 to 350nm. According to the lighting unit cover of the present invention, light within the wavelength from 410 to 350 nm are cut off, so that the attraction of a flying insect or the like can be prevented.

The ultraviolet absorbent B in the present invention is explained.

The ultraviolet absorbent B is characterized in that the absorbance at 320 nm is 20% or more of the absorbance at the wavelength of maximum absorption within the range from 270 nm to 400 nm, and the wavelength of maximum absorption is 380 nm or less. Especially, the absorbance of the ultraviolet absorbent B at 320 nm is preferably 30% or more of the absorbance at the wavelength of maximum absorption, more preferably 40% or more, and most preferably 50% or more. If the absorbance at 320 nm is less than 20% of the absorbance at the maximum absorption wavelength, a wavelength range that cannot be covered by both the ultraviolet absorbent A and the ultraviolet absorbent B generates within the range from 310 to 330 nm. Further, the wavelength of maximum absorption is preferably 380 nm or less, more preferably 370 nm or less, furthermore preferably 365 nm or less, and most preferably 350 nm or less.

The ultraviolet absorbent B represents an ultraviolet absorbent, in which the absorbance at 320 nm is 20% or more of the absorbance at the wavelength of maximum absorption and the wavelength of maximum absorption is 380 nm or less. As is shown in Fig. 1, the ultraviolet absorbent B can be classified into ultraviolet absorbent B-(1) in which the wavelength of maximum absorption is less than 320 nm and ultraviolet absorbent B-(2) in which the wavelength of maximum absorption is within the range from 320 nm to 380 nm, which can be properly selected in accordance with their intended use.

For example, it is particularly preferable that the ultraviolet absorbent B-(1) is used in the case where any other short-wavelength ultraviolet-absorbing element is not present at the time of, for example, kneading the ultraviolet absorbent into a molded product of plastic or a polymer. On account that any other element capable of absorbing a short-wavelength ultraviolet light of 300 nm or less is not present at the time of kneading the ultraviolet absorbent into the molded product of plastic or the polymer, usage of the ultraviolet absorbent B-(1), which is capable of effectively absorbing light in the short-wavelength ultraviolet range, enables to prevent the molded product of plastic itself and its content from ultraviolet light without another short-wavelength ultraviolet range-absorbing filter. Further, unexpected effects, that both compatibility with the polymer and light fastness are improved, are obtained, by using the ultraviolet absorbent B-(1) in combination with the ultraviolet absorbent A that is used in the present invention.

It is particularly preferable that the ultraviolet absorbent B-(2) is used in the case where another short-wavelength ultraviolet-absorbing element is present at the time of, for example, coating the ultraviolet absorbent on a glass film or dissolving the ultraviolet absorbent with a polymer to coat on a substrate. The ultraviolet absorbent B-(2) is excellent in capability of shielding light of around 320 nm, and is capable of efficiently absorbing light in the short-wavelength ultraviolet range of 300 nm or less. However, it is sometimes difficult for the ultraviolet absorbent B-(2) to absorb light in the short-wavelength ultraviolet range. Thus, it is preferable that the ultraviolet absorbent B-(2) is used by being coated on a polymer or a glass substrate used as a filter shielding light in the short-wavelength ultraviolet range. Further, in a

solvent-coating process, improvement of both solubility to the solvent (for example, ethyl acetate, methylethyl ketone, toluene) and light fastness when a coating film is used are unexpectedly achieved by using the ultraviolet absorbent B-(2) in combination with the ultraviolet absorbent A that is used in the present invention.

The ultraviolet absorbent B may have any structure, as long as the structure satisfies the condition (condition A) that the absorbance at 320 nm is 20% or more (preferably 30% to 90%) of the absorbance at the wavelength of maximum absorption and the wavelength of maximum absorption is 380 nm or less (preferably 270 nm to 370 nm). Examples of the ultraviolet absorbent B include benzotriazole-series, triazine-series, benzophenone-series, merocyanine-series, cyaine-series, dibenzoylmethane-series, cinnamic acid-series, acrylate-series, benzoic acid ester-series, oxalic acid diamide-series, formamidine-series, and benzoxadinone-series compounds, all of which are known as a structure of the ultraviolet absorbent. Among them, benzotriazole-series, triazine-series, benzophenone-series, dibenzoylmethane-seies, formamidine-series, and benzoxadinone-series compounds are preferred. Benzotriazole-series, triazine-series, benzophenone-series, formamidine-series, and benzoxadinone-series compounds are more preferable. Benzotriazole-series, triazine-series, and benzoxadinone-series compounds are most preferable. These ultraviolet absorbents are described, for example, in Fine Chemical, May in 2004, pp. 28-38, Kobunshi-yo Kinoseitenkazai no Shin Tenkai (New Developments of Functional Additives for Polymers), published by Toray Research Center, Division of Investigation Research (Toray Research Center, 1999), pp. 96-140, Kobunshi Tenkazai no Kaihatsu to kankyo Taisaku (Developments and Environmental Measures for Polymer Additives), supervised by Seiichi Okatsu (published by C M C Shuppan, 2003), pp. 54-64, and Kobunshi no Rekka · Henshoku mekanizumu to sono Anteika Gijutsu-Nohausyu-(Mechanism of Deterioration · Discoloration of Polymers and Their Stabilization Technique-Collection of Know-how), published by Kabushiki kaisha Gijutsu Jyoho Kyokai (TECHNICAL INFORMATION INSTITUTE CO., LTD., 2006).

Among these, amide-series solvents, sulfone-series solvents, sulfoxide-series solvents, ureido-series solvents, ether-series solvents, ketone-series solvents, halogen-containing solvents, alcohol-series solvents, ester-series solvents, and nitrile-series solvents are preferable from the viewpoint of solubility of the ultraviolet absorbent.

In the present invention, the concentration of the ultraviolet absorbents A and B and the compound C as well as the bluing agent D for the measurement of a wavelength of spectroscopic absorption maximum can be a concentration which can measure a wavelength of maximum spectroscopic absorption. Preferably, it is within the range of $1\times10^{-7}$ to $1\times10^{-2}$ mol/L. A temperature for the measurement is not specifically limited, however, preferably it is from 0C˚ to 80C˚. Unless otherwise specified, as an apparatus for measuring the spectroscopic absorption, a common spectroscopic absorption measurement apparatus (e.g., U-4100 spectrophotometer, trade name, manufactured by Hitachi High-Technologies Corp.) is used.

The maximum absorption wavelength and the half value width of the dye in the present invention are determined, unless otherwise specified, by preparing a solution using ethyl acetate as a solvent at a concentration of approximately $5 \times 10^{-5}$ $mol.dm^{-3}$ and conducting a measurement using a quartz cell having an optical path length of 10 mm.

A solution for measuring the spectroscopic absorption maximum wavelength in the present invention is explained. The solution for measuring the spectroscopic absorption maximum wavelength is obtained by dissolving the ultraviolet absorbents A and B and the compound C as well as the bluing agent D in an organic or inorganic solvent or water, either singly or as a mixture.

Examples of the organic solvent include amide-series solvents (e.g., N,N-dimethylformamide, N,N-dimethylacetamide, and 1-methyl-2-pyrrolidone), sulfone-series solvents (e.g., sulfolane), sulfoxide-series solvents (e.g., dimethyl sulfoxide), ureido-series solvents (e.g., tetramethylurea), ether-series solvents (e.g., dioxane, tetrahydrofuran, and cyclopentyl methyl ether), ketone-series solvents (e.g., acetone and cyclohexanone), hydrocarbon-series solvents (e.g., toluene, xylene, and n-decane), halogen-containing solvents (e.g., tetrachloroethane, chlorobenzene, and chloronaphthalene), alcohol-series solvents (e.g., methanol, ethanol, isopropyl alcohol, ethylene glycol, cyclohexanol, and phenol), pyridine-series solvents (e.g., pyridine, γ-picoline, and 2,6-lutidine), ester-series solvents (e.g., ethyl acetate and butyl acetate), carboxylic acid-series solvents (e.g., acetic acid and propionic acid), nitrile-series solvents (e.g., acetonitrile), sulfonic acid-series solvents (e.g., methanesulfonic acid), and amine-series solvents (e.g., triethylamine and tributylamine). Examples of the inorganic solvent include sulfuric acid and phosphoric acid.

The benzotriazole-series compounds have an effective absorption wavelength of approximately 270 to 380 nm, and are preferably represented by formula (IIa) or (IIb). Hereinafter, (IIa) and (IIb) are described deteil.

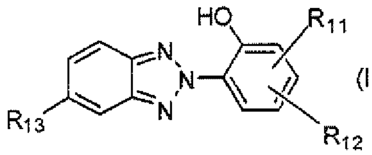
(IIa)
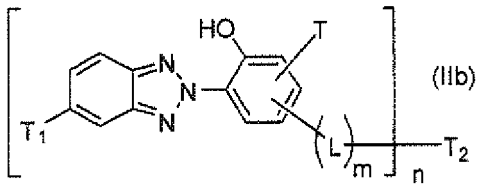
(IIb)

[In formula (IIa),

$R_{11}$ represents a hydrogen atom, a substituted or unsubstituted alkyl group, a substituted or unsubstituted cycloalkyl group, or a substituted or unsubstituted aryl group;

$R_{12}$ represents a hydrogen atom, a halogen atom, a substituted or unsubstituted alkyl group, or a substituted or unsubstituted aryl group; and

$R_{13}$ represents a hydrogen atom, a halogen atom, a substituted or unsubstituted alkyl group, a substituted or unsubstituted alkoxy group, or -COOR$_{14}$ group (herein, $R_{14}$ represents a hydrogen atom, a substituted or unsubstituted alkyl group, or a substituted or unsubstituted aryl group.)]

[In formula (IIb),

T represents a hydrogen atom or a substituted or unsubstituted alkyl group;

$T_1$ represents a hydrogen atom, a halogen atom, a substituted or unsubstituted alkyl group, a substituted or unsubstituted aryl group, or a substituted or unsubstituted alkoxy group;

L represents a divalent linking group or a single bond;

m represents 0 or 1;

n represents an integer of 1 to 4; and

when n is 1, $T_2$ represents a halogen atom, a substituted or unsubstituted alkyl group, or a substituted or unsubstituted aryl group; when n is 2, $T_2$ represents a divalent substituent; when n is 3, $T_2$ represents a trivalent substituent; and when n is 4, $T_2$ represents a tetravalent substituent.]

(Formula (IIa))

$R_{11}$ represents a hydrogen atom, a substituted or unsubstituted alkyl group, a substituted or unsubstituted cycloalkyl group, or a substituted or unsubstituted aryl group.

$R_{11}$ is preferably a substituted or unsubstituted alkyl group having 1 to 18 carbon atoms, a substituted or unsubstituted cycloalkyl group having 5 to 18 carbon atoms, or a substituted or unsubstituted aryl group having 6 to 24 carbon atoms; and particularly preferably a substituted or unsubstituted alkyl group having 1 to 18 carbon atoms, or a substituted or unsubstituted alkyl group having 1 to 18 carbon atoms.

The substituted alkyl group, the substituted cycloalkyl group, and the substituted aryl group each are referred to as an alkyl group, a cycloalkyl group, and an aryl group, each of which has a monovalent substituent at an arbitrary position thereof, respectively. Examples of the monovalent substituent include a halogen atom (e.g., a fluorine atom, a chlorine atom, a bromine atom, and an iodine atom), a straight-chain or branched alkyl group having 1 to 20 carbon atoms (preferably 1 to 10 carbon atoms) (e.g., methyl, ethyl), an aryl group having 6 to 20 carbon atoms (preferably 6 to 10 carbon atoms) (e.g., phenyl, naphthyl), a cyano group, a carboxyl group, an alkoxycarbonyl group having 1 to 20 carbon atoms (preferably 1 to 10 carbon atoms) (e.g., methoxycarbonyl), an aryloxycarbonyl group having 6 to 20 carbon atoms (preferably 6 to 10 carbon atoms) (e.g., phenoxycarbonyl), a substituted or unsubstituted carbamoyl group having 0 to 20 carbon atoms (preferably 0 to 10 carbon atoms) (e.g., carbamoyl, N-phenylcarbamoyl, N,N-dimethylcarbamoyl), an alkylcarbonyl group having 1 to 20 carbon atoms (preferably 1 to 10 carbon atoms) (e.g., acetyl), an arylcarbonyl group having 6 to 20 carbon atoms (preferably 6 to 10 carbon atoms) (e.g., benzoyl), a nitro group, a substituted or unsubstituted amino group having 0 to 20 carbon atoms (preferably 0 to 10 carbon atoms) (e.g., amino, dimethylamino, anilino), an acylamino group having 1 to 20 carbon atoms (preferably 1 to 10 carbon atoms) (e.g., acetamido, ethoxycarbonylamino),

a sulfonamido group having 0 to 20 carbon atoms (preferably 0 to 10 carbon atoms) (e.g., methanesulfanamido), an imido group having 2 to 20 carbon atoms (preferably 2 to 10 carbon atoms) (e.g., succinimido, phthalimido), an imino group having 1 to 20 carbon atoms (preferably 1 to 10 carbon atoms) (e.g., benzylideneamino), a hydroxy group, an alkoxy group having 1 to 20 carbon atoms (preferably 1 to 10 carbon atoms) (e.g., methoxy), an aryloxy group having 6 to 20 carbon atoms (preferably 6 to 10 carbon atoms) (e.g., phenoxy), an acyloxy group having 1 to 20 carbon atoms (preferably 1 to 10 carbon atoms) (e.g., acetoxy), an alkylsulfonyloxy group having 1 to 20 carbon atoms (preferably 1 to 10 carbon atoms) (e.g., methanesulfonyloxy), an arylsulfonyloxy group having 6 to 20 carbon atoms (preferably 6 to 10 carbon atoms) (e.g., benzenesulfonyloxy), a sulfo group, a substituted or unsubstituted sulfamoyl group having 0 to 20 carbon atoms (preferably 0 to 10 carbon atoms) (e.g., sulfamoyl, N-phenylsulfamoyl), an alkylthio group having 1 to 20 carbon atoms (preferably 1 to 10 carbon atoms) (e.g., methylthio), an arylthio group having 6 to 20 carbon atoms

(preferably 6 to 10 carbon atoms) (e.g., phenylthio), an alkylsulfonyl group having 1 to 20 carbon atoms (preferably 1 to 10 carbon atoms) (e.g., methansulfonyl), an arylsulfonyl group having 6 to 20 carbon atoms (preferably 6 to 10 carbon atoms) (e.g., benzenesulfonyl), and a four- to seven-membered (preferably five- to six-membered) heterocyclic group (e.g., pyridyl, morpholino).

$R_{12}$ represents a hydrogen atom, a halogen atom, a substituted or unsubstituted alkyl group, or a substituted or unsubstituted aryl group. $R_{12}$ is preferably a hydrogen atom, a chlorine atom, a substituted or unsubstituted alkyl group having 1 to 18 carbon atoms, a substituted or unsubstituted cycloalkyl group having 5 to 18 carbon atoms, or a substituted or unsubstituted aryl group having 6 to 24 carbon atoms; and particularly preferably a hydrogen atom, a chlorine atom, a substituted or unsubstituted alkyl group having 1 to 18 carbon atoms, or a substituted or unsubstituted aryl group having 6 to 24 carbon atoms.

$R_{13}$ represents a hydrogen atom, a halogen atom, a substituted or unsubstituted alkyl group, a substituted or unsubstituted alkoxy group, or -COOR$_{14}$ group (herein, $R_{14}$ represents a hydrogen atom, a substituted or unsubstituted alkyl group, or a substituted or unsubstituted aryl group). $R_{13}$ is preferably a hydrogen atom, a chlorine atom, a substituted or unsubstituted alkyl group having 1 to 18 carbon atoms, a substituted or unsubstituted alkoxy group having 1 to 18 carbon atoms, or -COOR$_{14}$ group (herein, $R_{14}$ represents a hydrogen atom, a substituted or unsubstituted alkyl group having 1 to 18 carbon atoms, or a substituted or unsubstituted aryl group having 6 to 24 carbon atoms).

$R_{11}$ and $R_{12}$ may be substituted at arbitrary positions of the benzene ring. The substitution at 2- or 4- position to the hydroxyl group is preferable.

(Formula (IIb))

T represents a hydrogen atom or a substituted or unsubstituted alkyl group. T is preferably a hydrogen atom or a substituted or unsubstituted alkyl group having 1 to 18 carbon atoms.

$T_1$ represents a hydrogen atom, a halogen atom, a substituted or unsubstituted alkyl group, a substituted or unsubstituted aryl group, or a substituted or unsubstituted alkoxy group. $T_1$ is preferably a hydrogen atom, a chlorine atom, a substituted or unsubstituted alkyl group having 1 to 18 carbon atoms, an aryl group having 6 to 24 carbon atoms, or an alkoxy group having 1 to 18 carbon atoms.

-L- represents a divalent linking group or a single bond. m represents 0 or 1.

The case where m is 0 (zero) means that $T_2$ directly bonds with the benzene ring without involving L, that is, -L- represents a single bond.

The divalent linking group -L- is explained. -L- is a divalent substituent represented by the following formula (a).

Formula (a) $$-(L_1)_{m1}-(L_2)_{m2}-(L_3)_{m3}-(L_4)_{m4}-(L_5)_{m5}-$$

In formula (a), m1 to m5 each represent an integer of 0 to 2.

$L_1$ to $L_5$ each independently represent -CO-, -O-, -SO$_2$-, -SO-, -NR$_L$-, a substituted or unsubstituted divalent alkyl group, a substituted or unsubstituted divalent alkenyl group, or a substituted or unsubstituted divalent aryl group. $R_L$ represents a hydrogen atom, a substituted or unsubstituted alkyl group, or a substituted or unsubstituted aryl group.

Specific examples of $R_L$ include a hydrogen atom, a methyl group, an ethyl group, a propyl group, a hexyl group, an octyl group, a phenyl group, and a naphthyl group. The group may be substituted with one or more monovalent substituents at any position of the alkyl group or the aryl group. Examples of the monovalent substituent include the examples of the monovalent substituents described above. $R_L$ is preferably a substituted or unsubstituted alkyl group having 3 to 20 carbon atoms or a substituted or unsubstituted aryl group having 6 to 14 carbon atoms; and more preferably a substituted or unsubstituted alkyl group having 6 to 12 carbon atoms or a substituted or unsubstituted aryl group having 6 to 10 carbon atoms.

Preferred examples of the divalent substituent -L- include $-O-CO-C_2H_4-CO-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$ and $-NH-SO_2-C_3H_6-$.

In formula (IIb), n represents an integer of 1 to 4.

When n is 1, $T_2$ represents a halogen atom, a substituted or unsubstituted alkyl group, or a substituted or unsubstituted aryl group. When n is 1, $T_2$ is preferably a chlorine atom, a substituted or unsubstituted alkyl group having 1 to 18 carbon atoms, or a substituted or unsubstituted aryl group having 6 to 24 carbon atoms.

When n is 2, $T_2$ represents a divalent substituent. When n is 2, specific examples of $T_2$ include the same examples as those of the above-described divalent substituent -L-. When n is 2, $T_2$ is preferably $-CH_2-$, $-O-CO-C_2H_4-CO-O-$, or $-NH-CO-C_3H_6-CO-NH-$.

When n is 3, $T_2$ represents a trivalent substituent. The trivalent substituent is explained. The trivalent substituent is a trivalent alkyl group, a trivalent aryl group, or a substituent represented by the following formula.

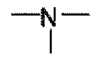

The trivalent substituent is preferably a trivalent alkyl group having 1 to 8 carbon atoms, a trivalent aryl group having 6 to 14 carbon atoms, or a substituent represented by the following formula.

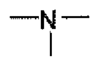

When n is 4, $T_2$ represents a tetravalent substituent. The tetravalent substituent is explained. The tetravalent substituent is a tetravalent alkyl group or a tetravalent aryl group. Among the tetravalent substituents, a tetravalent alkyl group having 1 to 8 carbon atoms and a tetravalent aryl group having 6 to 14 carbon atoms are preferable.
In formula (IIb), it is particularly preferable that n is 1 or 2.
Specifically, the components of formula (IIb) are preferably combined as follows:

when n is 1, T is a hydrogen atom, or a substituted or unsubstituted alkyl group having 1 to 18 carbon atoms; $T_1$ is a hydrogen atom, a chlorine atom, a substituted or unsubstituted alkyl group having 1 to 18 carbon atoms, an aryl group having 6 to 24 carbon atoms, or an alkoxy group having 1 to 18 carbon atoms; L is $-O-CO-C_3H_6-$, $-CH_2-$, $-C_3H_6-$, $-C_5H_{10}-$, $-C_8H_{16}-$, $-NH-CO-C_4H_8-$ or a single bond; and $T_2$ is a chlorine atom, a substituted or unsubstituted alkyl group having 1 to 18 carbon atoms, or a substituted or unsubstituted aryl group having 6 to 24 carbon atoms;
when n is 2, a preferable combination is that T is a hydrogen atom, or a substituted or unsubstituted alkyl group having 1 to 18 carbon atoms; $T_1$ is a hydrogen atom, a chlorine atom, a substituted or unsubstituted alkyl group having 1 to 18 carbon atoms, an aryl group having 6 to 24 carbon atoms, or an alkoxy group having 1 to 18 carbon atoms; L is $-CH_2-$ or a single bond; and $T_2$ is $-CH_2-$, $-O-CO-C_2H_4-CO-O-$ or $NH-CO-C_3H_6-CO-NH-$; and
when n is 2, a preferable combination is that m is 0 and T is a hydrogen atom or substituted or unsubstituted alkyl group having 1 to 18 carbon atoms; $T_1$ is a hydrogen atom, a chlorine atom, a substituted or unsubstituted alkyl group having 1 to 18 carbon atoms, an aryl group having 6 to 24 carbon atoms, or an alkoxy group having 1 to 18 carbon atoms; and $T_2$ is $-CH_2-$, $-O-CO-C_2H_4-CO-O-$ or $-NH-CO-C_3H_6-CO-NH-$.

Typical examples of the compound represented by formula (IIa) or (IIb) include 2-(2'-hydroxy-5'-methylphenyl) benzotriazole, 2-(2'-hydroxy-5'-t-butylphenyl)benzotriazole, 2-(2'-hydroxy-3'-t-butyl-5'-methylphenyl)-5-chlorobenzotriazole, 2-(2'-hydroxy-3',5'-di-t-butylphenyl)-5-chlorohenzotriazole, 2-(2'-hydroxy-3'-dodecyl-5'-methylphenyl)-5-chlorobenzotriazole, 2-(2'-hydroxy-3',5'-di-t-amylphenyl)benzotriazole, 2-(2'-hydroxy-5'-(1,1,3,3-tetramethylbutyl)phenyl) benzotriazole, 2-(2'-hydroxy-4'-octyloxyphenyl)benzotriazole, 2-(2'-hydroxy-3'-(3,4,5,6-tetrahydrophthalimidylmethyl)-5'-methylbenzyl)phenyl)benzotriazole, 2-(3'-sec-butyl-5'-t-butyl-2'-hydroxyphenyl)benzotriazole, 2-(3',5'-bis-(α,α-dimethylbenzyl)-2'-hydroxyphenyl)benzotriazole, 2-(3'-t-butyl-2'-hydroxy-5'-(2-octyloxycarbonylethyl)phenyl)-5-chloro-benzotriazole, 2-(3'-t-butyl-5'-[2-(2-ethylhexyloxy)-carbonylethyl]-2'-hydroxyphenyl)-5-chloro-benzotriazole, 2-(3'-t-butyl-2'-hydroxy-5'-(2-methoxycarbonylethyl)phenyl)-5-chloro-benzotriazole, 2-(3'-t-butyl-2'-hydroxy-5'-(2-methoxycarbonylethyl)phenyl)benzotriazole, 2-(3'-t-butyl-2'-hydroxy-5'-(2-octyloxycarbonylethyl)phenyl)benzotriazole, 2-(3'-t-butyl-5'-[2-(2-ethylhexyloxy)carbonylethyl]-2'-hydroxyphenyl)benzotriazole, 2-(3'-dodecyl-2'-hydroxy-5'-methylphenyl) benzotriazole, 2-(3'-t-butyl-2'-hydroxy-5'-(2-isooctyloxycarbonylethyl)phenylbenzotriazole, 2,2'-methylene-bis [4-(1,1,3,3-tetramethylbutyl)-6-benzotriazole-2-ylphenol], ester exchange products of 2-[3'-t-butyl-5'-(2-methoxycarbonylethyl)-2'-hydroxyphenyl]-2H-benzotriazole and polyethylene glycol 300; and the compound represented by the following formula:

$$[R-CH_2CH_2-COO-CH_2CH_2]_2$$

(wherein, R represents 3'-tert-butyl-4'-hydroxy-5'-2H-benzotriazol-2-ylphenyl, 2-[2'-hydroxy-3'-(α,α-dimethylbenzyl)-5'-(1,1,3,3-tetramethylbutyl)-pbenyl]bezazotriazole; 2-[2'-hydroxy-3'-(1,1,3,3-tetramethylbutyl)-5'-(α,α-dimethylbenzyl)-phenyl]benzotriazole and the like).
The triazine-based compound is preferably a compound having an effective absorption wavelength of approximately 270 to 380 nm that is represented by the following formula (III).

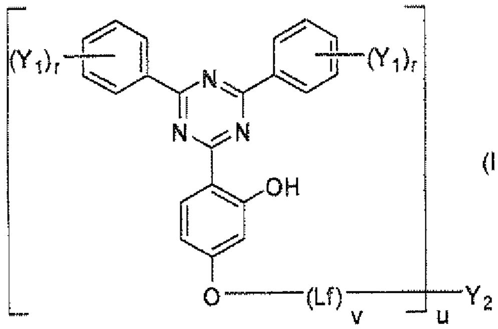

(III)

[In formula (III),
the substituent $Y_1$s each independently represent a hydrogen atom, a hydroxyl group, a substituted or unsubstituted alkyl group, a substituted or unsubstituted aryl group, or a substituted or unsubstituted alkoxy group;
Lf represents a divalent linking group or a single bond;
u represents 1 or 2;
v represents 0 or 1;
r represents an integer of 1 to 3; and
when u is 1, $Y_2$ represents a hydrogen atom, a substituted or unsubstituted alkyl group, or a substituted or unsubstituted aryl group; and when u is 2, $Y_2$ represents a divalent substituent.

$Y_1$s each independently represent a hydrogen atom, a hydroxyl group, a substituted or unsubstituted alkyl group, a substituted or unsubstituted aryl group, or a substituted or unsubstituted alkoxy group. $Y_1$ is preferably a hydrogen atom, a hydroxyl group, a substituted or unsubstituted alkyl group having 1 to 18 carbon atoms, a substituted or unsubstituted aryl group having 6 to 24 carbon atoms, or a substituted or unsubstituted alkoxy group having 1 to 18 carbon atoms.

Lf represents a divalent linking group or a single bond. u represents 1 or 2. r represents an integer of 1 to 3. v represents 0 or 1. When v is 0, Lf represents a single bond.
The divalent linking group -Lf- is explained. The divalent substituent -Lf- is a divalent substituent represented by the following formula (b).

Formula (b) $-(Lf_1)_{mf1}-(Lf_2)_{mf2}-(Lf_3)_{mf3}-(Lf_4)_{mf4}-(Lf_5)_{mf5}-$

In formula (b), mf1 to mf5 each represents an integer of 0 to 2.
$Lf_1$ to $Lf_5$ each independently represent -CO-, -O- $-SO_2-$, -SO-, $-NRf_L-$, a substituted or unsubstituted divalent alkyl group, a substituted or unsubstituted divalent alkenyl group, or a substituted or unsubstituted divalent aryl group. $Rf_L$ represents a hydrogen atom, a substituted or unsubstituted alkyl group, or a substituted or unsubstituted aryl group.

Specific examples of $Rf_L$ include a hydrogen atom, a methyl group, an ethyl group, a propyl group, a hexyl group, an octyl group, a phenyl group, and a naphthyl group. The group may be substituted with one or more monovalent substituents at any position of the alkyl group or the aryl group. Examples of the monovalent substituent include the examples of monovalent substituent described above. $Rf_L$ is preferably a substituted or unsubstituted alkyl group having 3 to 20 carbon atoms or a substituted or unsubstituted aryl group having 6 to 14 carbon atoms; and more preferably a substituted or unsubstituted alkyl group having 6 to 12 carbon atoms or a substituted or unsubstituted aryl group having 6 to 10 carbon atoms.

Preferred examples of the divalent substituent -Lf include $-O-CO-C_2H_4-CO-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$ and $-NH-SO_2-C_3H_6-$.

When u is 1, $Y_2$ represents a hydrogen atom, a substituted or unsubstituted alkyl group, or a substituted or unsubstituted aryl group. When u is 1, $Y_2$ is preferably a hydrogen atom, a substituted or unsubstituted alkyl group having 1 to 18 carbon atoms, or a substituted or unsubstituted aryl group having 6 to 24 carbon atoms.

When u is 2, $Y^2$ represents a divalent substituent. Examples of the divalent substituent include the same examples as those of the aforementioned divalent substituent -L-. $Y_2$ is preferably a substituted or unsubstituted divalent alkyl group, a substituted or unsubstituted divalent alkenyl group, a substituted or unsubstituted divalent aryl group, $-CH_2CH(OH)CH_2-O-Y_{11}-OCH_2CH(OH)CH_2$, $-CO-Y_{12}-CO-$, $-CO-NH-Y_{13}-NH-CO-$, or $-(CH_2)_t-CO_2-Y_{14}-OCO-(CH_2)_t$.
Herein, t is 1, 2 or 3;

$Y_{11}$ represents a substituted or unsubstituted alkylene, phenylene, or - phenylene-M-phenylene- (wherein, M represents -O-, -S-, $-SO_2-$, $-CH_2-$ or $-C(CH_3)_2-$);

$Y_{12}$ represents a substituted or unsubstituted divalent alkyl group, a substituted or unsubstituted divalent alkenyl group, or a substituted or unsubstituted divalent aryl group;

$Y_{13}$ represents a substituted or unsubstituted divalent alkyl group, or a substituted or unsubstituted divalent aryl group; and $Y_{14}$ represents a substituted or unsubstituted divalent alkyl group, or a substituted or unsubstituted divalent aryl group.

When u is 2, $Y_2$ is preferably a substituted or unsubstituted divalent alkyl group having 1 to 18 carbon atoms, a substituted or unsubstituted divalent aryl group having 6 to 24 carbon atoms, $-CH_2CH(OH)CH_2-O-CH_2-OCH_2CH(OH)CH_2-$, $-CH_2CH(OH)CH_2-O-C(CH_3)_2-OC_8H_{16}-$, or $-(CH_2)_2-CO_2-C_2H_4-OCO-(CH_2)_2-$.

Typical examples of the compound represented by formula (III) include 2-(4-butoxy-2-hydroxyphenyl)-4,6-di(4-butoxyphenyl)-1,3,5-triazine, 2-(4-butoxy-2-hydroxyphenyl)-4,6-di(2,4-dibutoxyphenyl)-1,3,5-triazine, 2,4-di(4-butoxy-2-hydroxyphenyl)-6-(4-butoxyphenyl)-1,3,5-triazine, 2,4-di(4-butoxy-2-hydroxyphenyl)-6-(2,4-dibutoxyphenyl)-1,3,5-triazine, 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-oetyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-(2-hydroxy-4-tridecyloxyphenyl)-4,6-bis(2,4-dimethylphenyl)-1-3,5-triazine, 2-[2-hydroxy-4-(2-hydroxy-3-butyloxypropoxy)phenyl]-4,6-bis(2,4-dimethyl)-1,3,5-triazine, 2-[2-hydroxy-4-(2-hydroxy-3-octyloxypropyloxy)phenyl]-4,6-bis(2,4-dimsthyl)-1,3,5-triazine, 2-[4-(dodecyloxy/tridecyloxy-2-hydroxypropoxy)-2-hydroxyphenyl]-4,6-bis(2,4-dimethylphenyl)-1,3,5-triazine, 2-[2-hydroxy-4-(2-hydroxy-3-dodecyloxypropoxy)phenyl]-4,6-bis(2,4-dimethylphenyl)-1,3,5-triazine, 2-(2-hydroxy-4-hexyloxy)phenyl-4,6-diphenyl-1,3,5-triazine, 2-(2-hydroxy-4-methoxyphenyl)-4,6-diphanyl-1,3,5-triazine, 2,4,6-tris(2-hydroxy-4-(3-butoxy-2-hydroxypropoxy)phenyl)-1,3,5-triazine, 2-(2-hydroxyphenyl)-4-(4-methoxyphenyl)-6-phenyl-1,3,5-triazine, 2-{2-hydroxy-4-[3-(2-ethylhexyl-1-oxy)-2-hydroxy-propyloxy]phenyl}-4,6-bis(2,4-dimethylphenyl)-1,3,5-triazine, and 2-(2-hydroxy-4-{2-ethylhexyl}oxy)phenyl-4,6-di(4-phenyl)phenyl-1,3,5-triazine.

The benzophenone-based compound is preferably a compound having an effective absorption wavelength of approximately 270 to 380 nm that is represented by the following formula (IVa) or (IVb).

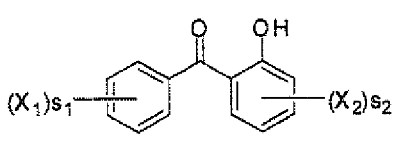 (IVa) 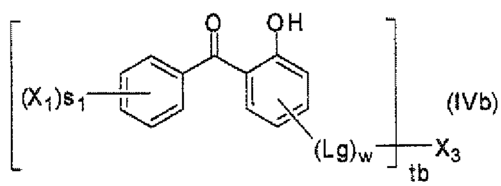 (IVb)

[In formula (IVa), $X_1$ and $X_2$ each independently represent a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group; and s1 and s2 each independently represent an integer of 1 to 3.]

[In formula (IVb), $X_1$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group; s1 represents an integer of 1 to 3;

Lg represents a divalent substituent or a single bond; w represents 0 or 1;

tb represents 1 or 2; and when tb is 1, $X_3$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group; and when tb is 2, $X_3$ represents a divalent substituent.]

(Formula (IVa))

$X_1$ and $X_2$ each independently represent a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group.

$X_1$ and $X_2$ each are preferably a hydrogen atom, a chlorine atom, a hydroxyl group, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms; and particularly preferably a hydrogen atom, a hydroxyl group, a substituted or unsubstituted alkoxy group having 1 to 18 carbon atoms, a sulfonic acid group, or a substituted or unsubstituted amino group having 1 to 16 carbon atoms.

(Formula (IVb))

tb is 1 or 2, w is 0 or 1, and s1 is an integer of 1 to 3.

The substituent $X_1$ represents a hydrogen atom, a halogen atom, a hydroxyl group, a substituted or unsubstituted alkyl group, a substituted or unsubstituted phenyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group.

$X_1$ is preferably a hydrogen atom, a chlorine atom, a hydroxyl group, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms; and particularly preferably a hydrogen atom, a hydroxyl group, a substituted or unsubstituted alkoxy group having 1 to 18 carbon atoms, a sulfonic acid group, or a substituted or unsubstituted amino group having 1 to 16 carbon atoms.

-Lg- represents a divalent linking group or a single bond. w represents an integer of 0 to 1. The case where w is 0 (zero) means that $X_3$ directly bonds with the benzene ring without involving Lg, namely, -Lg- represents a single bond. The divalent linking group -Lg- is explained. The divalent substituent Lg is a divalent substituent represented by the following formula (c).

Formula (c) $-(Lg_1)_{mg1}-(Lg_2)_{mg2}-(Lg_3)_{mg3}-(Lg_4)_{mg4}-(Lg_5)_{mg5}-$

In formula (c), mg1 to mg5 each represent an integer of 0 to 2.

$Lg_1$ to $Lg_5$ each independently represent -CO-, -O-, $-SO_2-$, -SO-, $-NRg_L-$, a substituted or unsubstituted divalent alkyl group, a substituted or unsubstituted divalent alkenyl group, or a substituted or unsubstituted divalent aryl group. $Rg_L$ represents a hydrogen atom, a substituted or unsubstituted alkyl group, or a substituted or unsubstituted aryl group.

Specific examples of $Rg_L$ include a hydrogen atom, a methyl group, an ethyl group, a propyl group, a hexyl group, an octyl group, a phenyl group, and a naphthyl group. The group may be substituted with one or more monovalent substituents at any position of the alkyl group or the aryl group. Examples of the monovalent substituent include the examples of the monovalent substituent described above. $Rg_L$ is preferably a substituted or unsubstituted alkyl group having 3 to 20 carbon atoms, or a substituted or unsubstituted aryl group having 6 to 14 carbon atoms; and more preferably a substituted or unsubstituted alkyl group having 6 to 12 carbon atoms, or a substituted or unsubstituted aryl group having 6 to 10 carbon atoms.

Preferred examples of the divalent substituent -Lg- include -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, and $-NH-SO_2-C_3H_6-$.

When tb is 1, $X_3$ represents a hydrogen atom, a halogen atom, a hydroxyl group, a substituted or unsubstituted phenyl group, a substituted or unsubstituted alkyl group, a substituted or unsubstituted alkoxy group, a substituted or unsubstituted alkylsulfonyl group, a substituted or unsubstituted arylsulfonyl group, a sulfonic acid group, a substituted or unsubstituted alkyloxycarbonyl group, a substituted or unsubstituted aryloxycarbonyl group, or a substituted or unsubstituted amino group.

When tb is 1, $X_3$ is preferably a hydrogen atom, a hydroxyl group, a chlorine atom, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon, atoms, an alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms.

$X_3$ is particularly preferably a hydrogen atom, a hydroxyl group, a substituted or unsubstituted alkoxy group having 1 to 18 carbon atoms, a sulfonic acid group, or a substituted or unsubstituted amino group having 1 to 16 carbon atoms.

When tb is 2, $X_3$ represents a divalent substituent.

When tb is 2, specific examples of $X_3$ include the same examples as those of the above-described divalent substituent -L-. When tb is 2, $X_3$ is preferably $-CH_2-$, $-C_4H_8-$, $-O-C_4H_8-O-$, $-O-CO-C_2H_4-CO-O-$, or $-NH-CO-C_3H_6-CO-NH-$.

In formula (IVb), tb is particularly preferably 1.

The component of formula (IVb) is preferably combined as follows.
Specifically, when tb is 1, a preferable combination is that $X_1$ is a hydrogen atom, a hydroxyl group, a substituted or unsubstituted alkoxy group having 1 to 18 carbon atoms, a sulfonic acid group, or a substituted or unsubstituted amino group having 1 to 16 carbon atoms;
Lg is -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, $-NH-SO_2-C_3H_6-$, or a single bond; and
$X_3$ is a hydrogen atom, a hydroxyl group, a chlorine atom, a substituted or unsubstituted alkyl group having 1 to 18 carbon atoms, a substituted or unsubstituted aryl group having 6 to 24 carbon atoms, a substituted or unsubstituted alkoxy group having 1 to 18 carbon atoms, an alkyloxycarbonyl group having 2 to 18 carbon atoms, an aryloxycarbonyl group having 7 to 24 carbon atoms, a sulfonic acid group, or a substituted or unsubstituted amino group having 1 to 16 carbon atoms.

When tb is 2, a preferable combination is that
$X_1$ is a hydrogen atom, a hydroxyl group, a substituted or unsubstituted alkoxy group having 1 to 18 carbon atoms, a sulfonic acid group, or a substituted or unsubstituted amino group having 1 to 16 carbon atoms;
Lg is -O-, $-O-CO-C_2H_4-CO-O-$, $-O-C_4H_8-O-$, $-O-CO-C_3H_6-$, $-NH-CO-C_3H_6-CO-NH-$, $-NH-CO-C_4H_8-$, $-CH_2-$, $-C_2H_4-$, $-C_3H_6-$, $-C_4H_8-$, $-C_5H_{10}-$, $-C_8H_{16}-$, $-C_4H_8-CO-O-$, $-C_6H_4-C_6H_4-$, $-NH-SO_2-C_3H_6-$, or a single bond; and
$X_3$ is $-CH_2-$, $-C_4H_8-$, $-O-C_4H_8-O-$, $-O-CO-C_2H_4-CO-O-$, or $-NH-CO-C_3H_6-CO-NH-$.

Typical examples of the benzophenone-series compound include 2,4-dihydroxybenzophenone, 2-hydroxy-4-methoxybenzophenone, 2-hydroxy-4-octyloxybenzophenone, 2-hydroxy-4-decyloxybenzophenone, 2-hydroxy-4-dodecyloxybenzophenone, 2-hydroxy-4-benzyloxybenzophenone, 2-hydroxy-4-(2-hydroxy-3-methacryloxypropoxy)benzophenone, 2-hydroxy-4-methoxy-5-sulfobenzophenone, 2-hydroxy-4-methoxy-5-sulfobenzophenone trihydrate, 2-hydroxy-4-methoxy-2'-carboxybenzophenone, 2-hydroxy-4-octadecyloxybenzophenone, 2-hydroxy-4-diethylamino-2'-hexyloxycarbonylbenzophenone, 2,2'-dihydroxy-4-methoxybenzophenone, 2,2',4,4'-tetrahydroxybenzophenone, 2,2'-dihydroxy-4,4'-dimethoxybenzophenone, and 1,4-bis(4-benzyloxy-3 -hydroxyphenoxy)butane.

The benzoxazinone-series compound is preferably a compound having an effective absorption wavelength of approximately 270 to 380 nm that is represented by the following formula (V).

Formula (V)

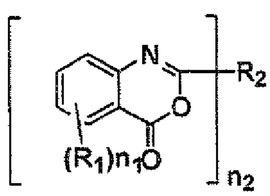

(In formula (V), $R_1$ represents a substituent; $n_1$ is an integer of 0 to 4; $R_2$ represents a $n_2$-valent substituent or linking group; and $n_2$ is an integer of 1 to 4.)

In formula (V), $R_1$ represents a substituent. Examples of the substituent include the same as those recited as examples of the substituent involved in the above-described substituted alkyl group, substituted alkenyl group, substituted alkynyl group, and substituent of the alkyl moiety of the above-described substituted aralkyl group. $R_1$ is preferably a halogen atom, an alkyl group, an alkenyl group, an alkynyl group, an aryl group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group, an acylamino group, an aminocarbonylamino group, an alkoxycarbonylamino group, an aryloxycarbonylamino group, a sulfamoylamino group, an alkyl- or aryl-sulfanylamino group, a mercapto group, an alkylthio group, an arylthio group, a heterocyclic thio group, a sulfamoyl group, a sulfo group, an alkyl- or aryl-sulfonyl group, an alkyl- or aryl-sulfonyl group, an acyl group, an aryloxycarbonyl group, an alkoxycarbonyl group, a carbamoyl group, an imido group, a phosphino group, a phosphinyl group, a phosphinyloxy group, a phosphinylamino group, or a silyl group; more preferably a halogen atom, an alkyl group, an aryl group, a cyano group, a hydroxyl group, a nitro group, a carboxyl group, an alkoxy group, an aryloxy group, a silyloxy group, a heterocyclic oxy group, an acyloxy group, a carbamoyloxy group, an amino group, an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, a carbamoyl group, an imido group, a phosphino group, a phosphinyl group, a phosphinyloxy group, a phosphinylamino group, or a silyl group; further preferably a halogen atom, an alkyl group, an aryl group, a hydroxyl group, an alkoxy group, an

aryloxy group, an amino group, a mercapto group, an alkylthio group, an arylthio group, a sulfamoyl group, a sulfo group, an alkyl- or aryl-sulfinyl group, or an alkyl- or aryl-sulfonyl group; further preferably a halogen atom, an alkyl group, an aryl group, an alkoxy group, an aryloxy group, an alkylthio group, or an arylthio group; further preferably a halogen atom, an alkyl group having 1 to 20 carbon atoms, an aryl group having 6 to 20 carbon atoms, an alkoxy group having 1 to 20 carbon atoms, an aryloxy group having 6 to 20 carbon atoms, an alkylthio group having 1 to 20 carbon atoms, or an arylthio group having 6 to 20 carbon atoms; further preferably a chlorine atom, a fluorine atom, a bromine atom, an alkyl group having 1 to 8 carbon atoms, an aryl group having 6 to 10 carbon atoms, an alkoxy group having 1 to 8 carbon atoms, an aryloxy group having 6 to 10 carbon atoms, an alkylthio group having 1 to 8 carbon atoms, or an arylthio group having 6 to 10 carbon atoms; and further preferably a chlorine atom, a fluorine atom, an alkyl group having 1 to 4 carbon atoms, or an alkoxy group having 1 to 4 carbon atoms.

$n_1$ is preferably an integer of 0 to 3, more preferably an integer of 0 to 2, and further more preferably 0 or 1. $n_1$ is most preferably 0, which means that the benzene ring has no substituent.

$R_2$ represents a $n_2$-valent substituent or a linking group. Examples of the substituent include the same as those recited as examples of the substituent involved in the above-described substituted alkyl group, substituted alkenyl group, substituted alkynyl group, and substituent of the alkyl moiety of the above-described substituted aralkyl group. The term "linking group" means a substituent further having one or more of linking bond. $R_2$ is preferably an aliphatic group, an aromatic group, or a linking group in which the aliphatic group or the aromatic group has an additional linking bond. $R_2$ is more preferably an alkyl group, an alkenyl group, an alkynyl group, an aryl group, or a divalent, trivalent, or tetravalent linking group each derived from these groups, still more preferably an alkyl group, an alkenyl group, an aryl group, and a divalent or trivalent linking group each derived from these groups, still more preferably an alkyl group having 1 to 20 carbon atoms, an alkenyl group having 2 to 20 carbon atoms, an aryl group having 6 to 20 carbon atoms, and a divalent or trivalent linking group each derived from these groups, still more preferably an alkyl group having 1 to 8 carbon atoms, an alkenyl group having 2 to 8 carbon atoms, an aryl group having 6 to 12 carbon atoms, and a divalent or trivalent linking group each derived from these groups, still more preferably an alkyl group having 1 to 8 carbon atoms, an aryl group having 6 to 12 carbon atoms, and a divalent or trivalent linking group each derived from these groups, still more preferably methyl, ethyl, propyl, butyl, isopropyl, 2-butyl, benzyl, phenyl, 2-naphthyl, ethylene, trimethylene, 1,2-propylene, tetramethylene, 1,2-phenylene, 1,3-phenylene, 1,4-phenylene, 2,6-naphthylene, and benzene-1,3,5,-yl, still more preferably methyl, ethyl, benzyl, phenyl, ethylene, trimethylene, 1,3-phenylene, 1,4-phenylene, and benzene-1,3,5,-yl, still more preferably ethylene, trimethylene, 1,3-phenylene, 1,4-phenylene, and benzene-1,3,5,-yl, and most preferably 1,4-phenylene.

$n_2$ is preferably an integer of 1 to 3, more preferably 2 or 3, and most preferably 2.

Typical examples of the above-described benzoxadinone-series compound include 2,2'-(p-phenylene)di-3,1-benzoxadine-4-on.

The salicylic acid-series compound above is preferably a compound having an effective absorption wavelength of approximately 290 to 330 nm, and typical examples thereof include phenyl salicylate, 4-t-butylphenyl salicylate, 4-octylphenyl salicylate, dibenzoylresorcinol, bis(4-t-butylbenzoyl)resorcinol, benzoylresorcinol, 2,4-di-t-butylphenyl 3,5-di-t-butyl-4-hydroxysalicylate, and hexadecyl 3,5-di-t-butyl-4-hydroxysalicylate.

The acrylate-series compound above is preferably a compound having an effective absorption wavelength of approximately 270 to 350 nm, and typical examples thereof include 2-ethylhexyl 2-cyano-3,3-diphenylacrylate, ethyl 2-cyano-3,3-diphenylacrylate, isooctyl 2-cyano-3,3-diphenylacrylate, hexadecyl 2-cyano-3-(4-methylphenyl)acrylate, methyl 2-cyano-3-methyl-3-(4-methoxyphenyl)cinnamate, butyl 2-cyano-3-methyl-3-(4-methoxyphenyl)cinnamate, methyl 2-carbomethoxy-3-(4-methoxyphenyl)cinnamate 2-cyano-3-(4-methylphenyl)acrylate salt, 1,3-bis(2'-cyano-3,3'-diphenylacryloyl)oxy)-2,2-bis(((2'-cyano-3,3'-diphenylacryloyl)oxy)methyl)propane, and N-(2-carbomethoxy-2-cyanovinyl)-2-methylindoline.

The oxalic diamide-series compound above is preferably a compound having an effective absorption wavelength of approximately 250 to 350 nm, and typical examples thereof include 4,4'-dioctyloxyoxanilide, 2,2'-dioctyloxy-5,5'-di-t-butyloxanilide, 2,2'-didodecyloxy-5,5'-di-t-butyloxanilide, 2-ethoxy-2'-ethyloxanilide, N,N'-bis(3-dimethylaminopropyl) oxamide, 2-ethoxy-5-t-butyl-2'-ethyloxanilide, and 2-ethoxy-2'-ethyl-5,4'-di-t-butyloxanilide.

The ultraviolet absorbent B is particular preferably a compound selected from the following compound group (B). The compound group (B) is a group composed of the following compounds (II-1) to (V-1).

[1] Compound represented by formula (IIa)

(II-1) 2-(2'-hydroxy-3',5'-di-t-amylphenyl)benzotriazole
(II-2) 2-(3-t-butyl-5-methyl-2-hydroxyphenyl)-5-chlorobenzotriazole
(II-3) 2-(2-hydroxy-5-t-octylphenyl)benzotriazole
(II-4) 2-ethylhexyl-3-[3-t-butyl-4-hydroxy-5-(5-chloro-2H-benzotriazole-2-yl)phenyl] propionate
(II-5) 2-(2H-benzotriazole-2-yl)-6-dodecyl-4-methyl-phenol
(II-6) 2-(2H-benzotriazole-2-yl)-3-t-butylphenol

(II-7) 2-(2H-benzotriazale-2-yl)-6-(1-methyl-1-phenylethyl)-4-(1,1-3,3-tetramethylbutyl)phenol
(II-8) 2-(2H-benzotriazole-2-yl)-3-methylphenol
(II-9) 2-(2H-benzotriazole-2-yl)-6-dodecyl-4-methyl-phenol

[2] Compound represented by formula (IIb)

(II-10) 2,2'-methylene-bis[6-(2H-benzotriazole-2-yl)-4-(1,1,3,3-tetramethylbutyl)phenol]

[3] Compound represented by formula (III)

(III-1) 2,4-bis(2-hydroxy-4-butoxyphenyl)-6-(2,4-dibutoxyphenyl)-1,3,5-triazine
(III-2) 2-[4-[(2-hydroxy-3-(2'-ethyl)hexyl)oxy]-2-hydroxyphenyl]-4,6-bis(2,4-dimethylphenyl)-1,3,5-triazine
(III-3) 2-[4-[(2-hydroxy-3-(2'-ethyl)hexyl)oxy]-2-hydroxyphenyl-4,6-bis(2,4-dimethylphenyl)-1,3,5-triazine
(III-4) 2-(4,6-diphenyl-1,3,5-triazin-2-yl)-5-hexyloxyphenol
(III-5) bisethylhexyloxyphenol methoxyphenyltriazine

[4] Compound represented by formula (IV)

(IV-1) hexyl 2-(4-diethylamino-2-hydroxybenzoyl)benzoate
(IV-2) 2,2'-hydroxy-4,4'-dimethoxybenzophenone
(IV-3) 2-hydroxy-4-methoxybenzophenone
(IV-4) 1,4-bis(4-benzoyl-3-hydroxyphenoxy)butane
(IV-5) 2-hydroxy-4-octoxybenzophenone
(IV-6) 2-hydroxy-4-methoxybenzophenone-5-sulfonic acid
(IV-7) 2,2',4,4'-tetrahydroxybenzophenone

[5] Compound represented by formula (V)

(V-1) 2,2'-(p-phenylene)di-3,1 -benzoxazinone-4-on

The compound (II-1) has the following structure, and is commercially available as trade name Tinuvin 328 (manufactured by Ciba Specialty Chemicals).
The compound (II-2) has the following structure, and is commercially available as trade name Tinuvin 326 (manufactured by Ciba Specialty Chemicals).
The compound (II-3) has the following structure, and is commercially available as trade name Tinuvin 329 (manufactured by Ciba Specialty Chemicals).
The compound (II-4) has the following structure, and is commercially available as trade name Tinuvin 109 (manufactured by Ciba Specialty Chemicals).
The compound (II-5) has the following structure, and is commercially available as trade name Tinuvin 171 (manufactured by Ciba Specialty Chemicals).
The compound (II-6) has the following structure, and is commercially available as trade name Tinuvin PS (manufactured by Ciba Specialty Chemicals).
The compound (II-7) has the following structure, and is commercially available as trade name Tinuvin 928 (manufactured by Ciba Specialty Chemicals).
The compound (II-8) has the following structure, and is commercially available as trade name Tinuvin P (manufactured by Ciba Specialty Chemicals).
The compound (II-9) has the following structure, and is commercially available as trade name Tinuvin 234 (manufactured by Ciba Specialty Chemicals).
The compound (II-10) has the following structure, and is commercially available as trade name Tinuvin 360 (manufactured by Ciba Specialty Chemicals).

The compound (III-1) has the following structure, and is commercially available as trade name Tinuvin 460 (manufactured by Ciba Specialty Chemicals).
The compound (III-2) has the following structure, and is commercially available as trade name Cyasorb UV-116

(manufactured by CYTEC Company Ltd.).
The compound (III-3) has the following structure, and is commercially available as trade name Tinuvin 405 (manufactured by Ciba Specialty Chemicals).
The compound (III-4) has the following structure, and is commercially available as trade name Tinuvin 1577 (manufactured by Ciba Specialty Chemicals).
The compound (III-5) has the following structure, and is commercially available as trade name Tinosorb S (manufactured by Ciba Specialty Chemicals).

The compound (IV-1) has the following structure, and is commercially available as trade name Uvinul A plus (manufactured by BASF).
The compound (IV-2) has the following structure, and is commercially available as trade name Uvinul 3049 (manufactured by BASF).
The compound (IV-3) has the following structure, and is commercially available as trade name Visorb 114 (manufactured by KYODO CHEMICAL CO., LTD.).
The compound (IV-4) has the following structure, and is commercially available as trade name Seesorb 151 (manufactured by SHIPRO KASEI KAISHA LTD.).
The compound (IV-5) has the following structure, and is commercially available as trade name Chimassorb 81 (manufactured by Ciba Specialty Chemicals).
The compound (IV-6) has the following structure, and is commercially available as trade name Uvinul MS40 (manufactured by BASF).
The compound (IV-7) has the following structure, and is commercially available as trade name Uvinul 3050 (manufactured by BASF).
The compound (V-1) has the following structure, and is commercially available as trade name Cyasorb UV-3638 (manufactured by Cytec Industries Inc.).

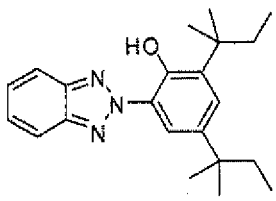
(II-1)

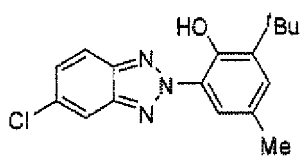
(II-2)

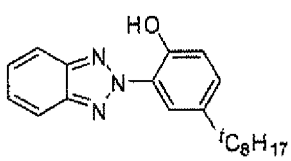
(II-3)

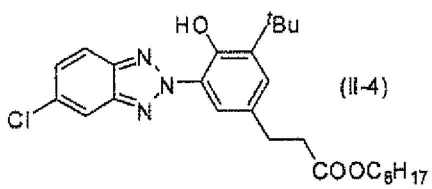
(II-4)

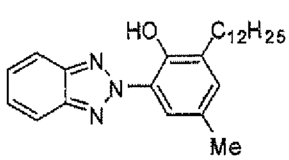
(II-5)

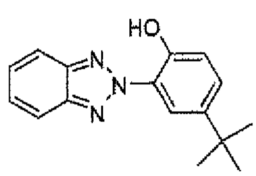
(II-6)

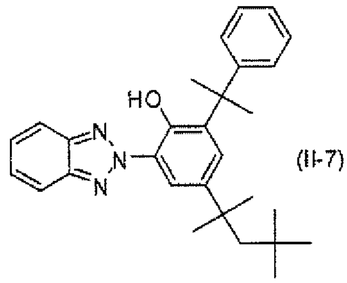
(II-7)

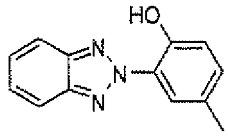
(II-8)

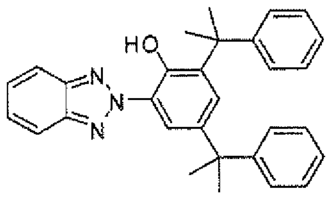
(II-9)

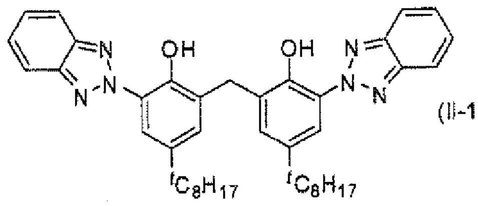
(II-10)

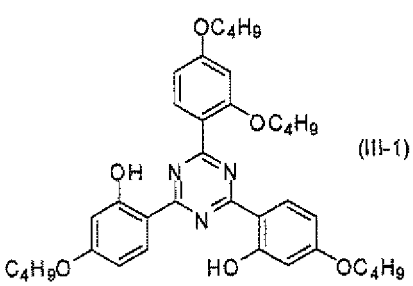
(III-1)

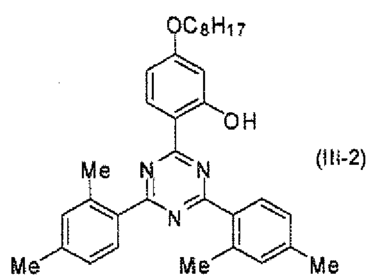
(III-2)

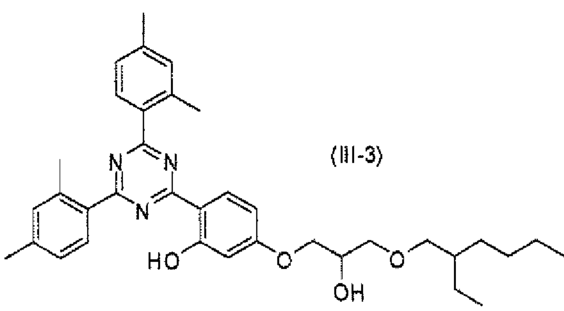
(III-3)

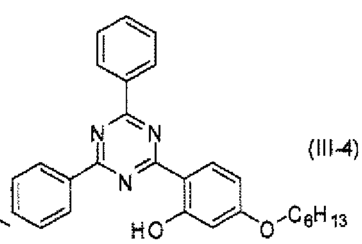
(III-4)

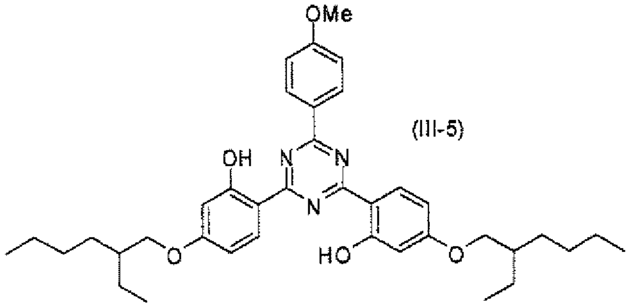
(III-5)

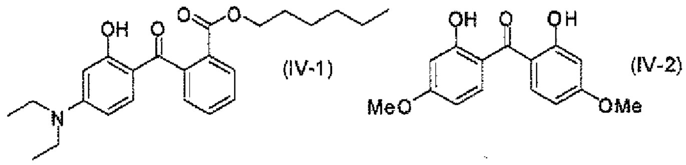

(IV-1) (IV-2)

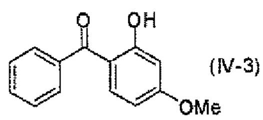

(IV-3)

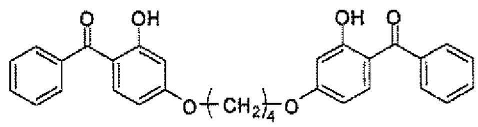

(IV-4)

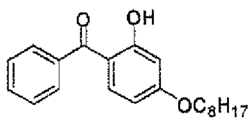

(IV-5)

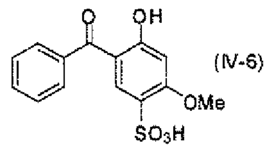

(IV-6)

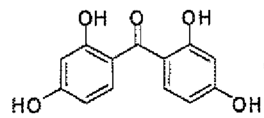

(IV-7)

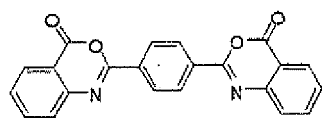

(V-1)

The ultraviolet absorbents A and B used in the present invention may be individually present, or may be connected to each other previously or by binding together with each other in the composition. Further, a polymerizable group may be bound with each of the ultraviolet absorbents A and B to form a polymerizable monomer, followed by polymerization of these monomers to form a copolymer including these monomers as a unit structure. Alternatively, these monomers may be used together with other monomers free of these ultraviolet absorbents A and B to form a copolymer. A preferable embodiment is that a composition is constructed by monomers, and a copolymer is formed by polymerization of the monomers at a desired stage.

Hereinafter, the compound C used in the present invention is explained. The compound C is represented by any one of the following formulae (TS-I) to (TS-V).

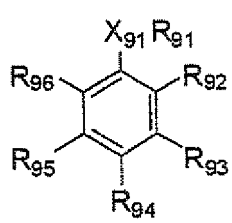

(TS-I)

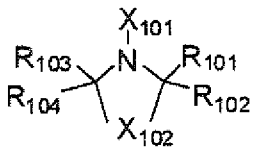 (TS-II)

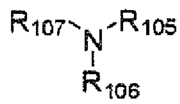 (TS-III)

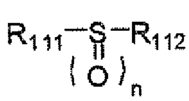 (TS-IV)

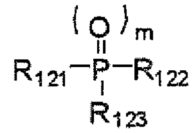 (TS-V)

In formula (TS-I), $R_{91}$ represents a hydrogen atom, an alkyl group (including a cyclic alkyl group such as a cycloalkyl group, a bicycloalkyl group, and a tricycloalkyl group), an alkenyl group (including a cyclic alkenyl group such as a cycloalkenyl group, a bicycloalkenyl group, and a tricycloalkenyl group), an aryl group, an heterocyclic group, an acyl group, an alkoxycarbonyl group, an aryloxycarbonyl group, an alkyl sulfonyl group (including a cyclic alkyl sulfonyl group such as a cycloalklyl sulfonyl group, a bicycloalklyl sulfonyl group, and a tricycloalklyl sulfonyl group), an aryl sulfonyl group, a phosphino group, a phosphinyl group, or -Si($R_{97}$)($R_{98}$)($R_{99}$). $R_{97}$, $R_{98}$, and $R_{99}$, which may be the same as or different from each other, each independently represent an alkyl group, an alkenyl group, an aryl group, an alkoxy group, an alkenyloxy group, or an aryloxy group. -$X_{91}$- represents -O-, -S-, or -N(-$R_{100}$)-, in which $R_{100}$ has the same meaning as that of $R_{91}$. $R_{92}$, $R_{93}$, $R_{94}$, $R_{95}$ and $R_{96}$, which may be the same as or different from each other, each independently represent a hydrogen atom or a substituent. $R_{91}$ and $R_{92}$, $R_{100}$ and $R_{96}$, and $R_{91}$ and $R_{100}$, respectively, may bind together with each other to form a 5- to 7-membered ring. Further, $R_{92}$ and $R_{93}$, and $R_{93}$ and $R_{94}$, respectively, may bind together with each other to form any of 5- to 7-membered rings, a spiro ring, or a bicyclo ring. However, all of $R_{91}$, $R_{92}$, $R_{93}$, $R_{94}$, $R_{95}$, $R_{96}$ and $R_{100}$ cannot simultaneously represent a hydrogen atom, respectively, and the total number of carbon atoms is 10 or more.

In the case where the group in the present specification contains an aliphatic moiety, the aliphatic moiety may be straight chain-like, branched chain-like, or cyclic, and saturated or unsaturated. Examples of the aliphatic moiety include alkyl, alkenyl, cycloalkyl, and cycloalkenyl moieties, each of which may be unsubstituted or substituted with a substituent. Further, in the case where the group contains an aryl moiety, the aryl moiety may be a single ring or a condensed ring, each of which may be unsubstituted or substituted with a substituent. Further, in the case where the group contains a heterocyclic moiety, the heterocyclic moiety contains a hetero atom (for example, a nitrogen atom, a sulfur atom, an oxygen atom) in the ring, and may be a saturated ring or an unsaturated ring, and may be a single ring or a condensed ring, each of which may be unsubstituted or substituted with a substituent. The substituent may bind with a hetero atom or a carbon atom in the ring.

The substituent used in the present invention is not particularly limited, as long as it is a replaceable group. Examples thereof include an aliphatic group, an aryl group, a heterocyclic group, an acyl group, an acyloxy group, an acyl amino group, an aliphatic oxy group, an aryloxy group, a heterocyclic oxy group, an aliphatic oxycarbonyl group, an aryloxycarbonyl group, a heterocyclic oxycarbonyl group, a carbamoyl group, an aliphatic sulfonyl group, an arylsulfonyl group, a heterocyclic sulfonyl group, an aliphatic sulfonyloxy group, an arylsulfonyloxy group, a heterocyclic sulfonyloxy group, a sulfamoyl group, an aliphatic sulfonamido group, an arylsulfonamido group, a heterocyclic sulfonamido group, an aliphatic amino group, an arylamino group, a heterocyclic amino group, an aliphatic oxycarbonylamino group, an aryloxycarbonylamino group, a heterocyclic oxycarbonylamino group, an aliphatic sulfinyl group, an arylsulfinyl group, an aliphatic thio group, an arylthio group, a hydroxy group, a cyano group, a sulfo group, a carboxyl group, an aliphatic oxyamino group, an aryloxyamino group, a carbamoylamino group, a sulfamoylamino group, a halogen atom, a sulfamoyl carbamoyl group, a carbamoyl sulfamoyl group, a phosphinyl group, and a phosphoryl group.

The compound represented by formula (TS-I) is described in detail below.

$R_{91}$ represents a hydrogen atom, an alkyl group (including a cyclic alkyl group such as a cycloalkyl group, a bicycloalkyl group, and a tricycloalkyl group, e.g., a methyl group, an i-propyl group, a s-butyl group, a dodecyl group, a methoxyethoxy group, and a benzyl group), an alkenyl group (including a cycloalkenyl group and a bicycloalkenyl group, e.g., an allyl group), an aryl group (e.g., a phenyl group, a p-methoxyphenyl group), a heterocyclic group (e.g., a 2-tetrahydfuryl group, a pyranyl group), an acyl group (e.g., an acetyl group, a pivaloyl group, a benzoyl group, an acryloyl group), an alkyl- or alkenyl-oxycarbonyl group (e.g., a methoxycarbonyl group, a hexadecyloxycarbonyl group), an aryloxycarbonyl

group (e.g., a phenoxycarbonyl group, a p-methoxyphenoxy carbonyl group), an alkyl sulfonyl group (e.g., a methanesulfonyl group, a butanesulfonyl group), an arylsulfonyl group (e.g., a benzene sulfonyl group, a p-toluenesulfonyl group), a phosphinotolyl group (e.g., a dimethoxyphosphino group, a diphenoxyphosphino group), a phosphinyl group (e.g., a diethylphosphinyl group, a diphenylphosphinyl group), or -Si($R_{97}$)($R_{98}$)($R_{99}$). $R_{97}$, $R_{98}$, and $R_{99}$ may be the same as or different from each other. $R_{97}$, $R_{98}$, and $R_{99}$ each represent an alkyl group (e.g., a methyl group, an ethyl group, a t-butyl group, a benzyl group), an alkenyl group (e.g., an allyl group), an aryl group (e.g., a phenyl group), an alkoxy group (e.g., a methoxy group, a butoxy group), an alkenyloxy group (e.g., an allyloxy group), or an aryloxy group (e.g., a phenoxy group).

-$X_{91}$- represents -O-, -S- or -N(-$R_{100}$)-. $R_{100}$ has the same meaning as that of $R_{91}$, and preferable ranges thereof are also the same. $R_{92}$, $R_{93}$, $R_{94}$, $R_{95}$ and $R_{96}$, which may be the same as or different from each other, each independently represent a hydrogen atom or a substituent. Examples of the substituent include a hydrogen atom, an alkyl group (including a cyclic alkyl group such as a cycloalkyl group, a bicycloalkyl group, and a tricycloalkyl group), an alkenyl group (including a cyclic alkenyl group such as a cycloalkenyl group, a bicycloalkenyl group, and a tricycloalkenyl group), an alkynyl group, an aryl group, a heterocyclic group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an anilino group), an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an aryl- or heterocyclic-azo group, an imido group, a phosphino group, a phophinyl group, a phosphinyloxy group, a phosphinylamino group, and a silyl group.

Among these, preferred examples of the substituent include an alkyl group (e.g., a methyl group, a t-butyl group, a t-hexyl group, a benzyl group), an alkenyl group (e.g., an allyl group), an aryl group (e.g., a phenyl group), an alkoxycarbonyl group (e.g., a methoxycarbonyl group, a dodecyloxycarbonyl group), an aryl oxycarbonyl group (e.g., a phenoxycarbonyl group), an alkyl- or alkenyl-sulfonyl group (e.g., a methanesulfonyl group, a butanesulfonyl group), an arylsulfonyl group (e.g., a benzene sulfonyl group, a p-hydroxybenzenesulfonyl group), or -$X_{91}$-$R_{91}$.

$R_{91}$ and $R_{92}$, $R_{100}$ and $R_{96}$, and $R_{91}$ and $R_{100}$, respectively, may bind together with each other to form a 5- to 7-membered ring (for example, a chromane ring, a morpholine ring). Further, $R_{92}$ and $R_{93}$, and $R_{93}$ and $R_{94}$, respectively, may bind together with each other to form a 5- to 7-membered ring (for example, a chromane ring, an indane ring), a spiro ring, or a bicyclo ring. However, all of $R_{91}$, $R_{92}$, $R_{93}$, $R_{94}$, $R_{95}$, $R_{96}$ and $R_{100}$ are not a hydrogen atom at the same time; and the total number of carbon atoms is 10 or more, and preferably 16 or more.

The compound represented by formula (TS-I) used in the present invention includes those compounds represented by any of, for example, formula (I) of JP-B-63-50691, formula (IIIa), (IIIb), or (IIIc) of JP-B-2-37575, formula of JP-B-2-50457, formula of JP-B-5-67220, formula (IX) of JP-B-5-70809, formula of JP-B-6-19534, formula (I) of JP-A-62-227889, formula (I) or (II) of JP-A-62-244046, formula (I) or (II) of JP-A-2-66541, formula (II) or (III) of JP-A-2-139544, formula (I) of JP-A-2-194062, formula (B), (C) or (D) of JP-A-2-212836, formula (III) of JP-A-3-200758, formula (II) or (III) of JP-A-3-48845, formula (B), (C) or (D) of JP-A-3-266836, formula (I) of JP-A-3-969440, formula (I) of JP-A-4-330440, formula (I) of JP-A-5-297541, formula of JP-A-6-130602, formula (1), (2) or (3) of International Patent Application Publication WO 91111749, formula (I) of German Patent Publication DE 4,008,785A1, formula (II) of U.S. Patent No. 4,931,382, formula (a) of European Patent No. 203,746B1, and formula (I) of European Patent No. 264,730B1.

As the compound represented by formula (TS-I), compounds represented by the following formulae (TS-IA) to (TS-IG) are exemplified. In the present invention, compounds having these structures are preferable.

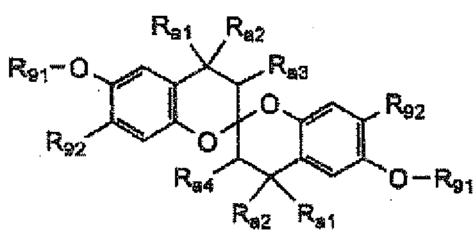

(TS-IA)

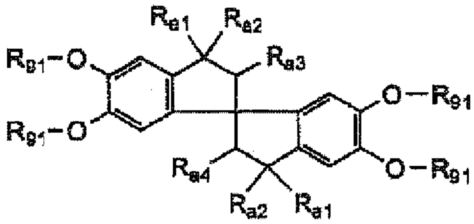
(TS-IB)

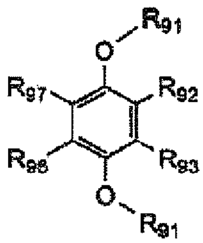
(TS-IC)

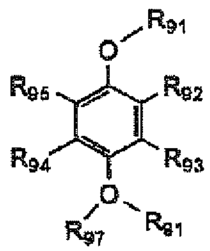
(TS-ID)

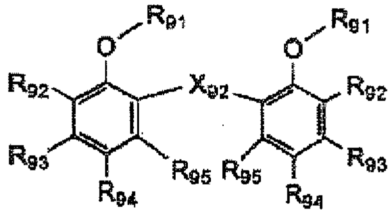
(TS-IE)

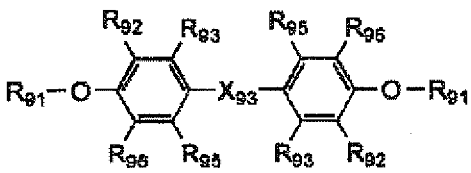
(TS-IF)

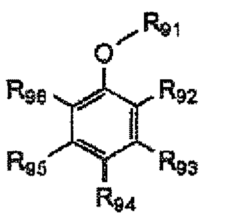
(TS-IG)

In formulae (TS-IA) to (TS-IG), $R_{91}$ to $R_{97}$ have the same meanings as those defined in formula (TS-I), and preferable ranges thereof are also the same. $R_{a1}$ to $R_{a4}$ each represent a hydrogen atom or an aliphatic group. $X_{92}$ and $X_{93}$ each represent a divalent linking group. Examples of the divalent linking group include an alkylene group, an oxy group, and a sulfonyl group. In the formulae, the same symbols in the same molecule may be the same as or different from each other.

In formula (TS-II), $R_{10}$, $R_{102}$, $R_{103}$, and $R_{104}$ each independently represent a hydrogen atom, an alkyl group (including a cyclic alkyl group such as a cycloalkyl group, a bicycloalkyl group, and a tricycloalkyl group), or an alkenyl group (including a cyclic alkenyl group such as a cycloalkenyl group, a bicycloalkenyl group, and a tricycloalkenyl group); $R_{101}$ and $R_{102}$, and $R_{103}$ and $R_{104}$, respectively, may bind to each other to form any of 5- to 7-membered rings. $X_{101}$ represents a hydrogen atom, an alkyl group (including a cyclic alkyl group such as a cycloalkyl group, a bicycloalkyl group, and a tricycloalkyl group), an alkenyl group (including a cyclic alkenyl group such as a cycloalkenyl group, a bicycloalkenyl group, and a tricycloalkenyl group), an alkoxy group, an alkenyloxy group, an alkyl- or alkenyl-oxycarbonyl group, an aryloxycarbonyl group, an acyl group, an acyloxy group, an alkyloxycarbonyloxy group, an alkenyloxycarbonyloxy group, an aryloxycarbonyloxy group, an alkyl- or alkenyl-sulfonyl group, an arylsulfonyl group, an alkyl- or alkenyl-sulfinyl group, an arylsulfinyl group, a sulfamoyl group, a carbamoyl group, a hydroxy group, or an oxy radical group.
$X_{102}$ represents a group of non-metal atoms necessary for forming any of 5- to 7-membered rings.

The compound represented by formula (TS-II) is described in more detail below.
In formula (TS-II), $R_{101}$, $R_{102}$, $R_{103}$ and $R_{104}$ each are a hydrogen atom, an alkyl group (e.g., a methyl group, an ethyl group), or an alkenyl group (e.g., an allyl group); preferably an alkyl group.
$X_{101}$ represents a hydrogen atom, an alkyl group (e.g., a methyl group, an ethyl group), an alkenyl group (e.g., an allyl group), an alkyloxy group (e.g., a methoxy group, an octyloxy group, a cyclohexyloxy group), an alkenyloxy group (e.g., an allyloxy group), an alkyloxycarbonyl group (e.g., a methoxycarbonyl group, a hexadecyloxycarbonyl group), an alkenyloxycarbonyl group (e.g., an allyloxycarbonyl group), an aryloxycarbonyl group (e.g., a phenoxycarbonyl group, a p-chlorophenoxycarbonyl group), an acyl group (e.g., an acetyl group, a pivaloyl group, a methacryloyl group), an acyloxy group (e.g., an acetoxy group, a benzoyloxy group), an alkyloxycarbonyloxy group (e.g., a methoxycarbonyloxy group, an octyloxycarbonyloxy group), an alkenyloxycarbonyloxy group (e.g., an allyloxycarbonyloxy group), an aryloxycarbonyloxy group (e.g., a phenoxycarbonyloxy group), an alkylsulfonyl group (e.g., a methanesulfonyl group, a butanesulfonyl group), an alkenylsulfonyl group (e.g., an allylsulfonyl group), an arylsulfonyl group (e.g., a benzene sulfonyl group, a p-toluenesulfonyl group), an alkylsulfinyl group (e.g., a methanesulfinyl group, an octanesulfinyl group), an alkenylsulfinyl group (e.g., an allylsulfinyl group), an arylsulfinyl group (e.g., a benzenesulfinyl group, a p-toluenesulfinyl group), a sulfamoyl group (e.g., a dimethylsulfamoyl group), a carbamoyl group (e.g., a dimethylcarbamoyl group, a diethylcarbamoyl group), a hydroxy group, or an oxy radical group.
$X_{102}$ represents a group of non-metal atoms necessary for forming a 5- to 7-membered ring (e.g., a piperidine ring, a piperazine ring).

In formula (TS-II), it is further preferable that $R_{103}$, $R_{104}$, $R_{105}$ and $R_{106}$ each are an alkyl group having 1 to 3 carbon atoms; $X_{101}$ is an oxy radical group, an alkyl group having 1 to 12 carbon atoms, an alkenyl group having 3 to 12 carbon atoms, a cycloalkyl group having 5 to 12 carbon atoms, an acyl group having 2 to 14 carbon atoms, or an aryl group having 6 to 20 carbon atoms; and $X_{102}$ forms a cyclohexane ring.
The compound represented by formula (TS-II) is particularly preferably a compound represented by formula (TS-IIa).

Formula (TS-IIa)

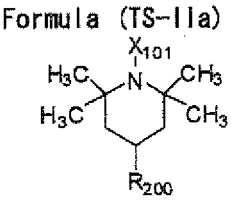

In formula (TS-IIa), $X_{101}$ has the same meaning as that of $X_{101}$ in formulae (TS-II), and preferable ranges thereof are also the same. $R_{200}$ represents a monovalent substituent. Examples of the monovalent substituent include those described above as the monovalent substituent.

The compound represented by formula (TS-II) used in the present invention include those compounds represented by, for example, formula (I) of JP-B-2-32298, formula (I) of JP-B-3-39296, formula of JP-B-3-40373, formula (I) of JP-A-2-49762, formula (II) of JP-A-2-208653, formula (III) of JP-A-2-217845, formula (B) of U.S. Patent No. 4,906,555, formula of European Patent Publication EP309,400A2, formula of European Patent Publication EP309,401A1, and formula of European Patent Publication EP309,402A1.

In formula (TS-III), $R_{105}$ and $R_{106}$ each independently represent a hydrogen atom, an aliphatic group, an acyl group, an aliphatic oxycarbonyl group, an aromatic oxycarbonyl group, an aliphatic sulfonyl group, or an aromatic sulfonyl group; $R_{107}$ represents an aliphatic group, an aliphatic oxy group, an aromatic oxy group, an aliphatic thio group, an aromatic thio group, an acyloxy group, an aliphatic oxycarbonyloxy group, an aromatic oxycarbonyloxy group, a substituted amino group, a heterocyclic group, or a hydroxyl group. If possible, $R_{105}$ and $R_{106}$, $R_{106}$ and $R_{107}$, and $R_{105}$ and $R_{107}$, respectively, may combine together to form any of 5- to 7-membered rings, but they never form a 2,2,6,6-tetraalkylpiperidine skeleton. In addition, both $R_{105}$ and $R_{106}$ are not hydrogen atoms at the same time; and the total number of carbon atoms is 7 or more.

The compound represented by formula (TS-III) is described in more detail below.
In formula (TS-III), $R_{105}$ and $R_{106}$ each independently represent a hydrogen atom, an aliphatic group (e.g., a methyl group, an ethyl group, a t-butyl group, an octyl group, a methoxyethoxy group), an acyl group (e.g., an acetyl group, a pivaloyl group, a methacryloyl group), an aliphatic oxycarbonyl group (e.g., a methoxycarbonyl group, a hexadecyl oxycarbonyl group), an aromatic oxycarbonyl group (e.g., a phenoxycarbonyl group), an aliphatic sulfonyl group (e.g.,

a methane sulfonyl group, a butane sulfonyl group), or an aromatic sulfonyl group (e.g., a phenyl sulfonyl group). $R_{107}$ represents an aliphatic group (e.g., a methyl group, an ethyl group, a t-butyl group, an octyl group, a methoxyethoxy group), an aliphatic oxy group (e.g., a methoxy group, an octyloxy group), an aromatic oxy group (e.g., a phenoxy group, a p-methoxyphenoxy group), an aliphatic thio group (e.g., a methylthio group, an octylthio group), an aromatic thio group (e.g., a phenylthio group, a p-methoxyphenylthio group), an acyloxy group (e.g., an acetoxy group, a pivaloyloxy group), an aliphatic oxycarbonyloxy group (e.g., a methoxycarbonyloxy group, an octyloxycarbonyloxy group), an aromatic oxycarbonyloxy group (e.g., a phenoxycarbonyl oxy group), a substituted amino group (the substituent may be any one that is able to substitute, e.g., an amino group substituted with a substituent such as an aliphatic group, an aromatic group, an acyl group, an aliphatic sulfonyl group, or an aromatic sulfonyl group), a heterocyclic group (e.g., a piperidine ring, a thiomorpholine ring), or a hydroxyl group. If possible, $R_{105}$ and $R_{106}$, $R_{106}$ and $R_{107}$, and $R_{105}$ and $R_{107}$, respectively, may combine together to form a 5- to 7-membered ring (e.g. a piperidine ring and a pyrazolidine ring). Both $R_{105}$ and $R_{106}$ are not hydrogen atoms at the same time; and the total number of carbon atoms is 7 or more.

The compound represented by formula (TS-III) used in the present invention include compounds represented by, for example, formula (I) of JP-B-6-97332, formula (I) of JP-B-6-97334, formula (I) of JP-A-2-148037, formula (I) of JP-A-2-150841, formula (I) of JP-A-2-181145, formula (I) of JP-A-3-266836, formula (IV) of JP-A-4-350854, and formula (I) of JP-A-5-61166.

Examples of the compound represented by formula (TS-III) include compounds represented by any one of formulae (TS-IIIA), to (TS-IIID). In the present invention, the compounds having any one of these structures are preferable.

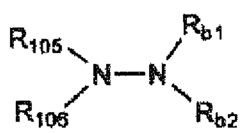 (TS-IIIA)

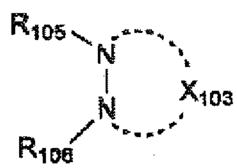 (TS-IIIB)

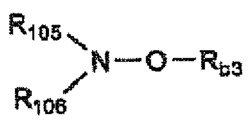 (TS-IIIC)

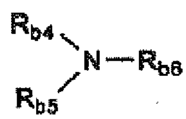 (TS-IIID)

In formulae (TS-IIIA) to (TS-IIID), $R_{105}$ and $R_{106}$ each have the same meanings as those defined in formula (TS-III), and the preferable ranges thereof are also the same. $R_{b1}$ to $R_{b3}$ each independently have the same meaning as that of $R_{105}$, and the preferable ranges thereof are also the same. $R_{b4}$, $R_{b5}$ and $R_{b6}$ each represent an aliphatic group. $X_{103}$ represents a group of non-metal atoms necessary to form any of 5- to 7-membered rings.

In formula (TS-IV), $R_{111}$ and $R_{112}$ each independently represent an aliphatic group. $R_{111}$ and $R_{112}$ may combine with each other to form any of 5- to 7-membered rings. n represents 0, 1, or 2. In the above, the total number of carbon atoms of $R_{111}$ and $R_{112}$ is 10 or more.

The compound represented by formula (TS-IV) is described in more detail below.

In formula (TS-IV), $R_{111}$ and $R_{112}$ each independently represent an aliphatic group (e.g., a methyl group, a methoxycarbonylethyl group, a dodecyloxycarbonyl ethyl group). $R_{111}$ and $R_{112}$ may combine with each other to form a 5- to 7-membered ring (such as a tetrahydrothiophene ring and a thiomorpholine ring), n represents 0, 1, or 2. In the above, the total number of carbon atoms of $R_{111}$ and $R_{112}$ is 10 or more.

The compound represented by formula (TS-IV) used in the present invention include compounds represented by, for example, formula (I) of JP-B-2-44052, formula (T) of JP-A-3-48242, formula (A) of JP-A-3-266836, formula (I), (II) or (III) of JP-A-5-323545, formula (I) of JP-A-6-148837, and formula (I) of U.S. Patent No. 4,933,271.

In formula (TS-V), $R_{121}$ and $R_{122}$ each independently represent an aliphatic oxy group or an aromatic oxy group; $R_{123}$ represents an aliphatic group, an aromatic group, an aliphatic oxy group, or an aromatic oxy group; and m represents 0 or 1. $R_{121}$ and $R_{122}$, and $R_{121}$ and $R_{123}$, respectively, may combine together to form any of 5- to 8-membered rings. The number of total carbon atoms of $R_{121}$, $R_{122}$, and $R_{123}$ is 10 or more.

The compound represented by formula (TS-V) is described in more detail below.

In formulae (TS-V), $R_{121}$ and $R_{122}$ each independently represent an aliphatic oxy group (e.g., a methoxy group, a t-octyloxy group), or an aromatic oxy group (e.g., a phenoxy group, a 2,4-di-t-butylphenoxy group). $R_{123}$ represents an

aliphatic group (e.g., a methyl group, an ethyl group, a t-octyl group), an aromatic group (e.g., a phenyl group, a 4-t-butylphenyl group), an aliphatic oxy group (e.g., a methoxy group, a t-octyloxy group), or an aromatic oxy group (e.g., a phenoxy group, a 4- t-butylphenoxy group). m represents 0 or 1. $R_{121}$ and $R_{122}$, and $R_{121}$ and $R_{123}$, respectively, may combine together to form any of 5- to 8-membered rings. The number of total carbon atoms of $R_{121}$, $R_{122}$, and $R_{123}$ is 10 or more.

The compound represented by formula (TS-V) used in the present invention include compounds represented by, for example, formula (I) of JP-A-3-25437, formula (I) of JP-A-3-142444, formula of U.S. Patent No. 4,749,645, and formula of U.S. Patent No. 4,980,275.

The compound represented by any one of formulae (TS-1) to (TS-V) is preferably selected from compounds represented by any one of formulae (TS-I), (TS-II) and (TS-V); more preferably compounds represented by formula (TS-I) or (TS-II); and particularly preferably compounds represented by formula (TS-II). Particularly preferred examples of the compound represented by formula (TS-II) include those having a 2,2,6,6-tetraalkylpiperidine skeleton (residue).

Hereinafter, specific examples of the compound represented by any one of formulae (TS-I) to (TS-V) are shown, but the present invention is not limited thereto. In addition, TI-1 to TI-57 are appended to the compounds that fall within formula (TS-I), TII-1 to TII-36 are appended to the compounds that fall within formula (TS-II), TIII-1 to TIII-13 are appended to the compounds that fall within formula (TS-III), TIV-1 to TIV-6 are appended to the compounds that fall within formula (TS-IV), and TV-1 to TV-8 are appended to the compounds that fall within formula (TS-V).

(TI-1)

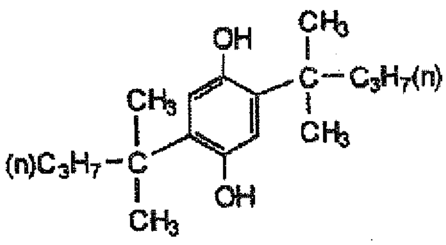

(TI-2)

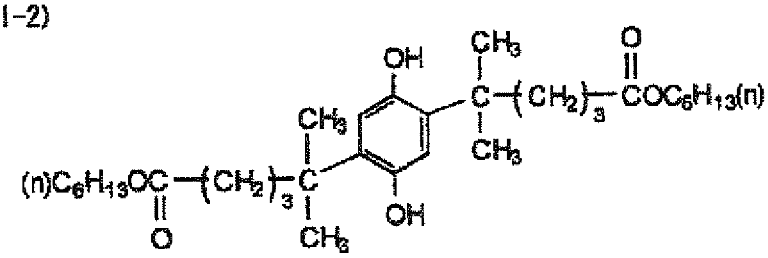

(TI-3)

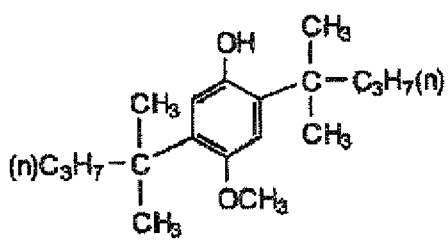

(TI-4)

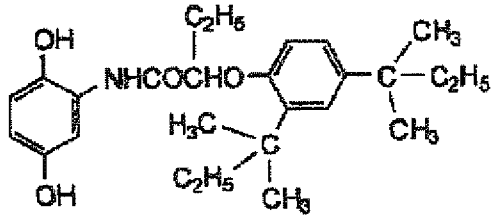

(TI-5)

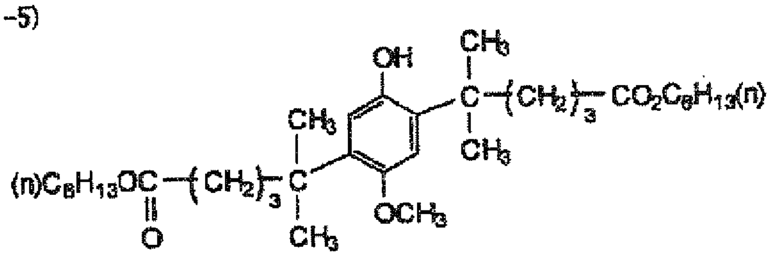

(TI-6)

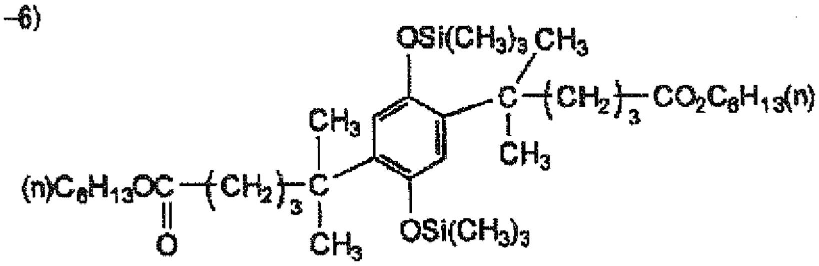

(TI-7)

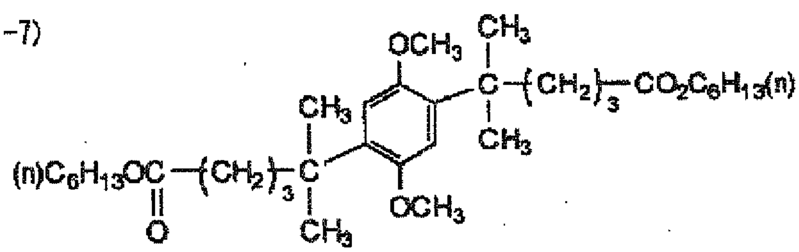

(TI-8)

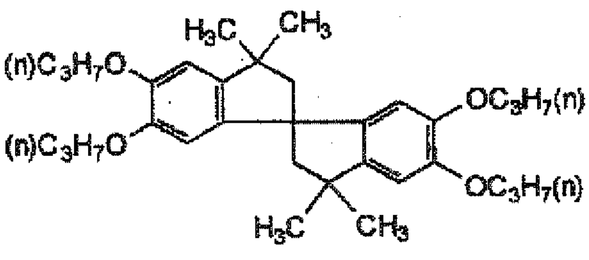

(TI-9)

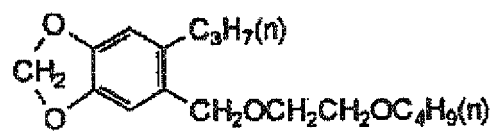

(TI-10)

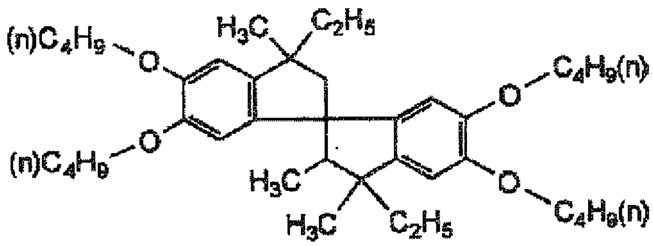

(TI-11)

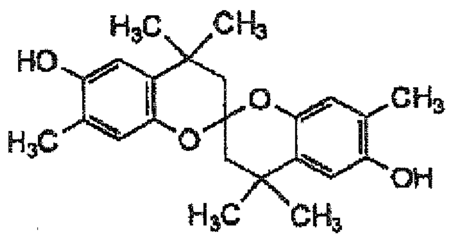

(TI-12)

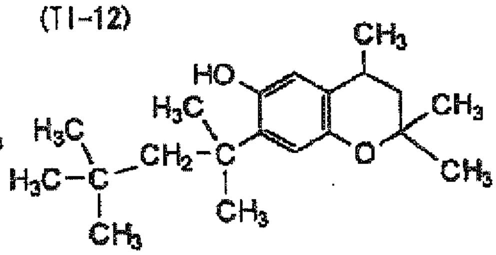

(TI-13)

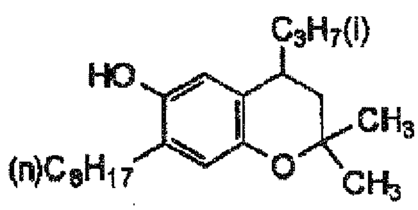

(TI-14)

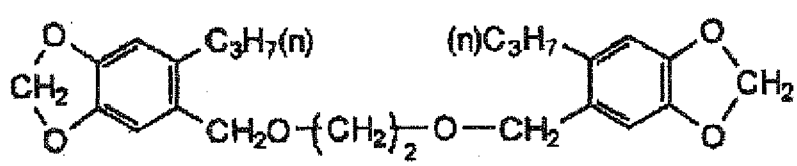

(TI-15)

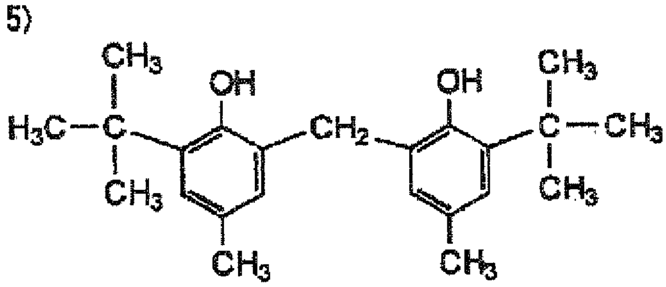

(TI-16)

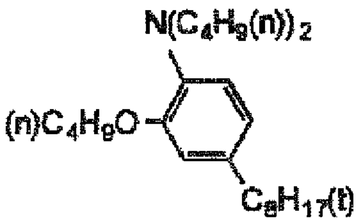

(TI-17)

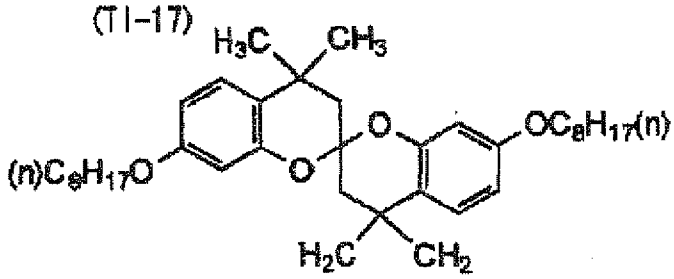

(TI-18)

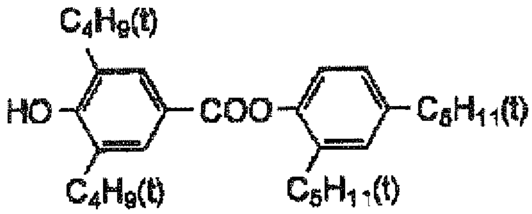

(TI-19)

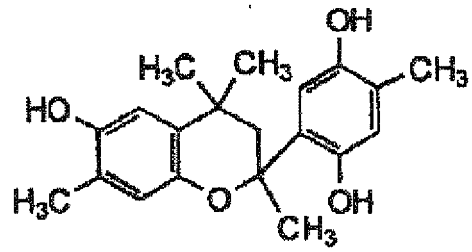

(TI-20)

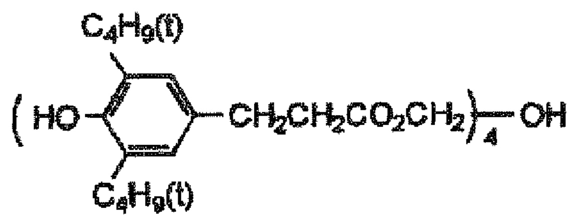

(TI-21)

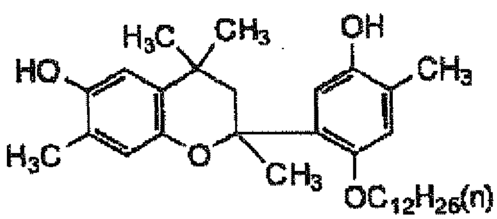

(TI-22)

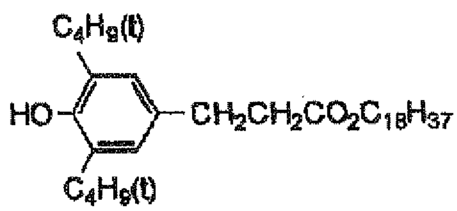

(TI-23)

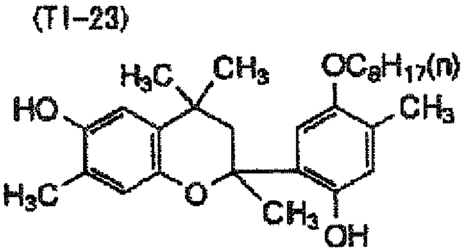

(TI-24)

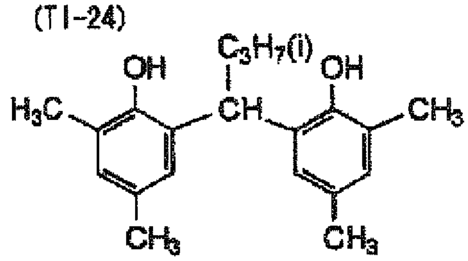

(TI-25)

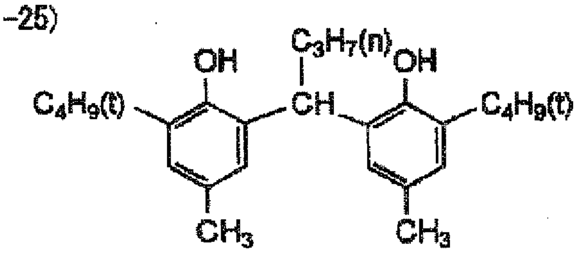

(TI-26)

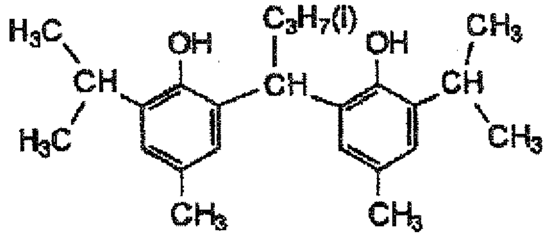

(TI-27)

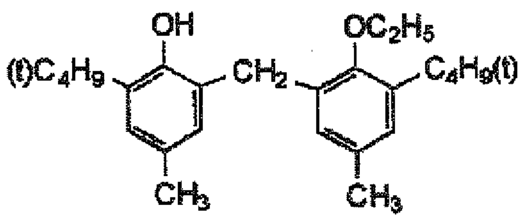

(TI-28)

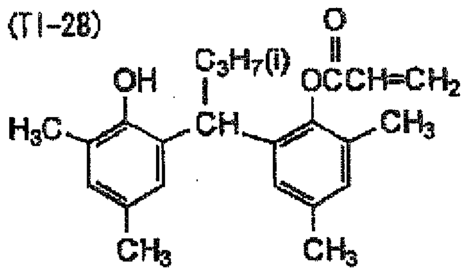

(TI-29)

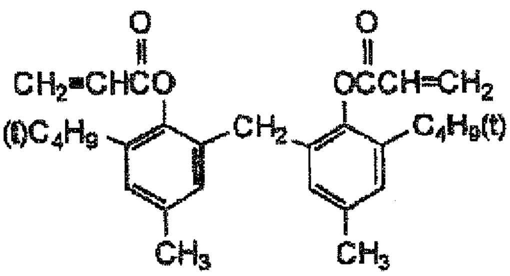

(TI-30)

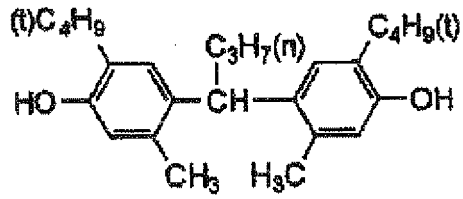

(TI-31)

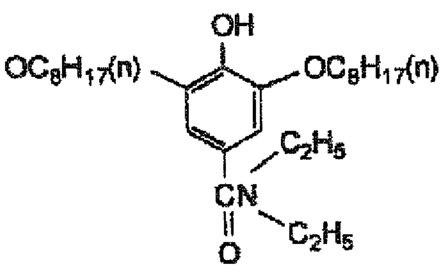

(TI-32)

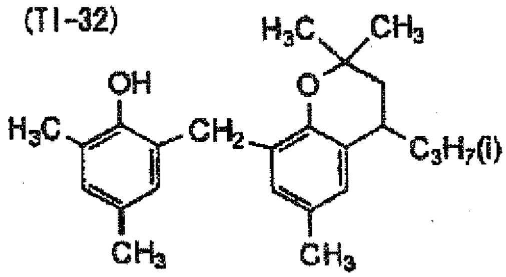

(TI-33)

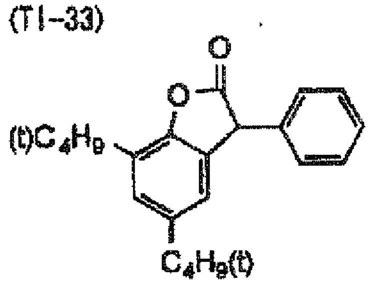

(TI-34)

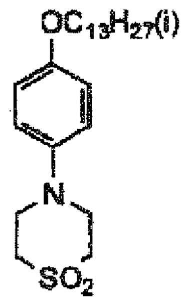

(TI-35)

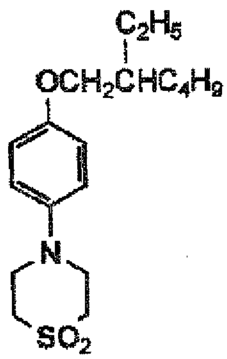

(TI-36)

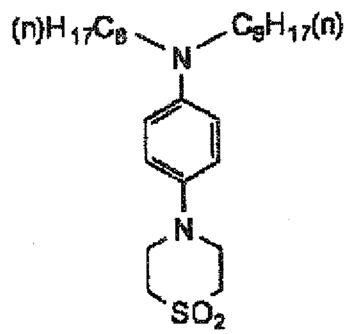

(TI-37)

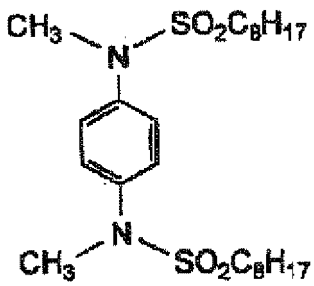

(TI-38)

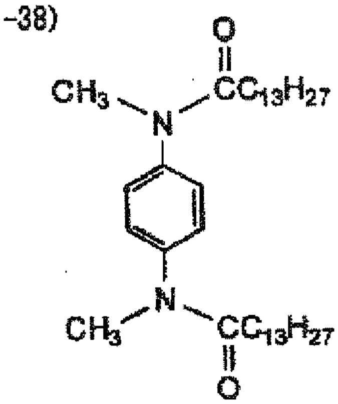

(TI-39)

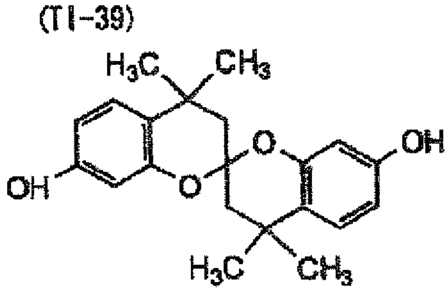

(TI-40)

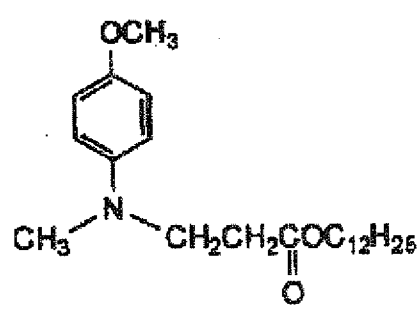

(TI-41)

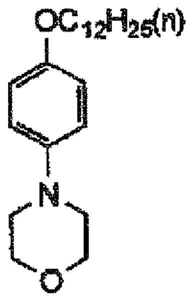

(TI-42)

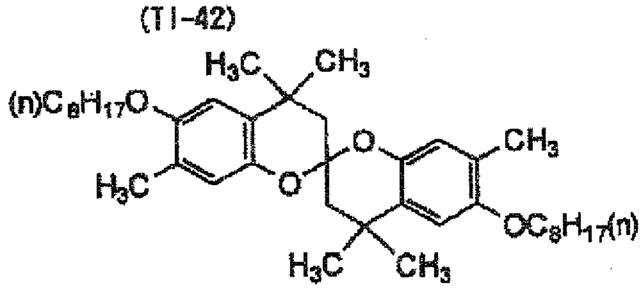

(TI-43)

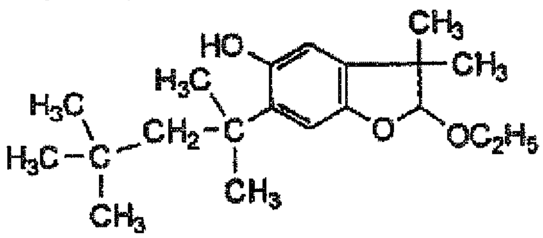

(TI-44)

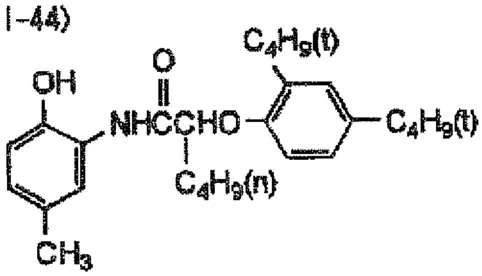

(TI-45)

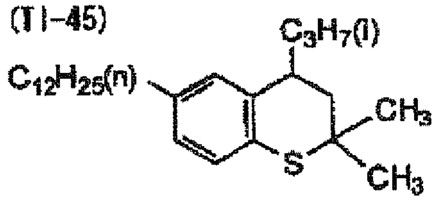

(TI-46)

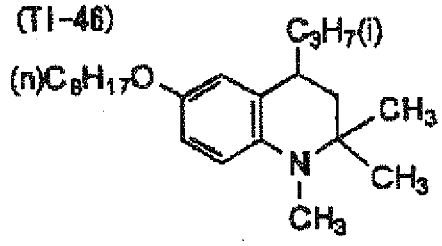

(TI-47)

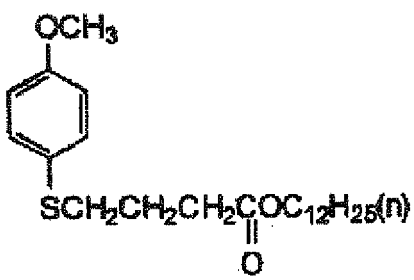

(TI-48)

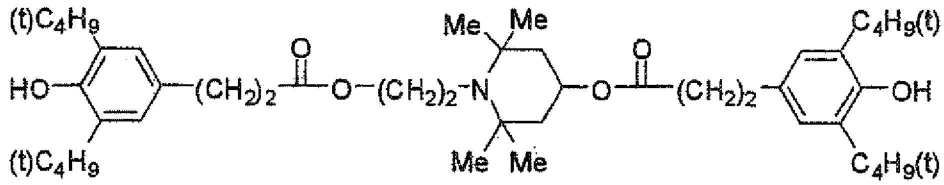

(TI-49)

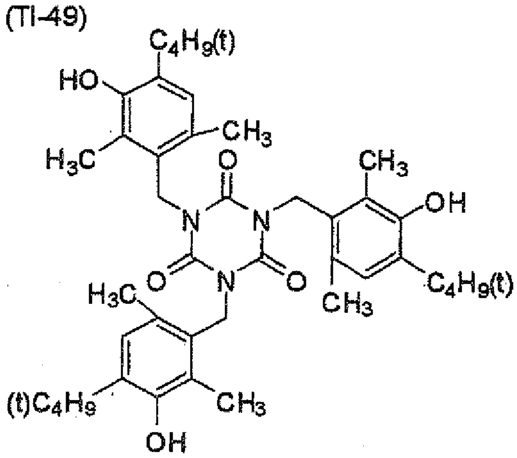

(TI-50)

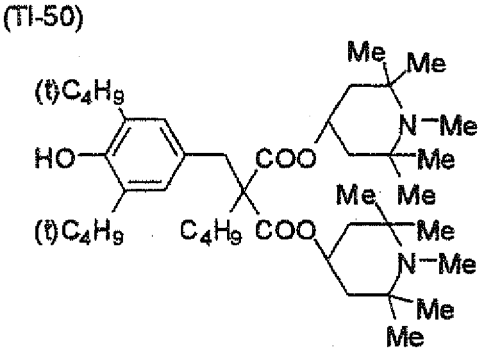

(TI-51)

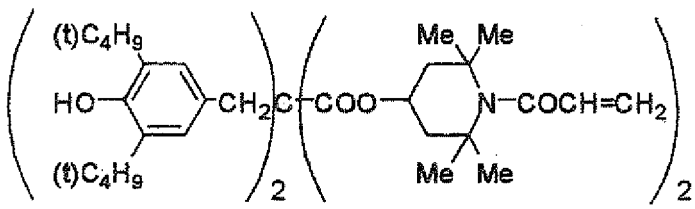

(TI-52)

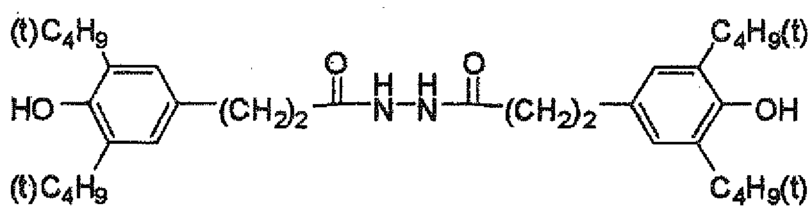

(TI-53)

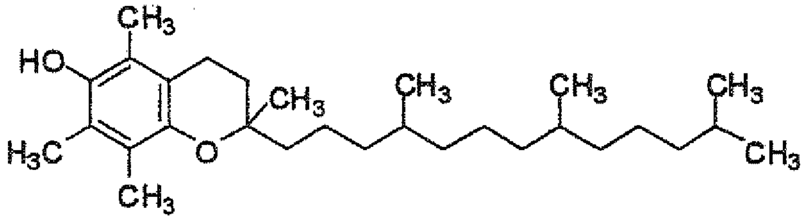

(TI-54)

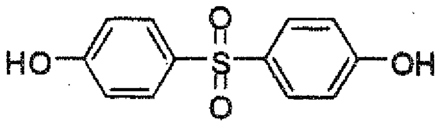

(TI-55)

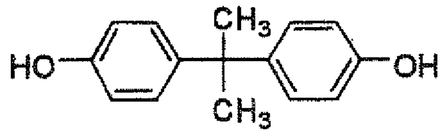

(TI-56)

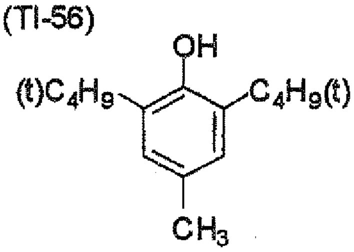

(TI-57)

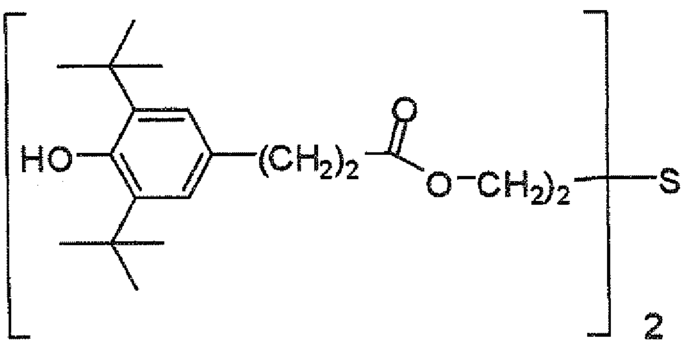

(TII-1)

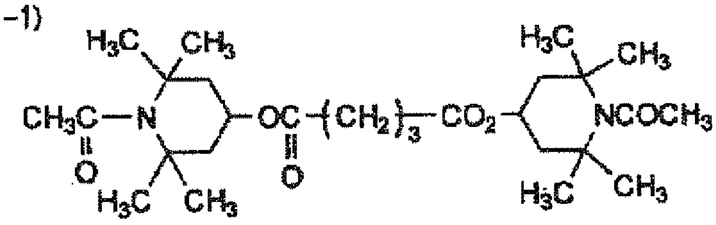

(TII-2)

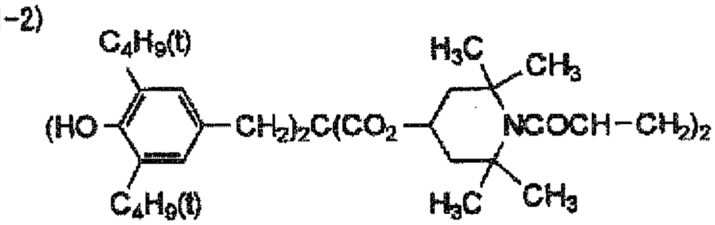

(TII-3)

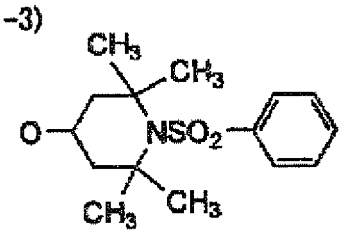

(TII-4)

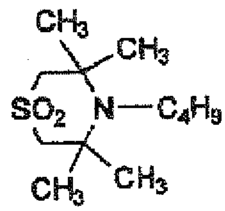

(TII-5)

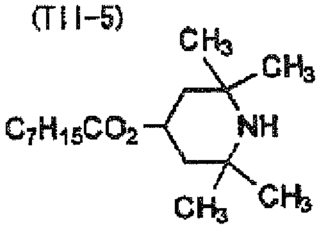

(TII-6)

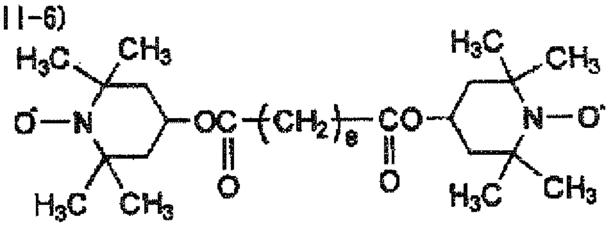

(TII-7)

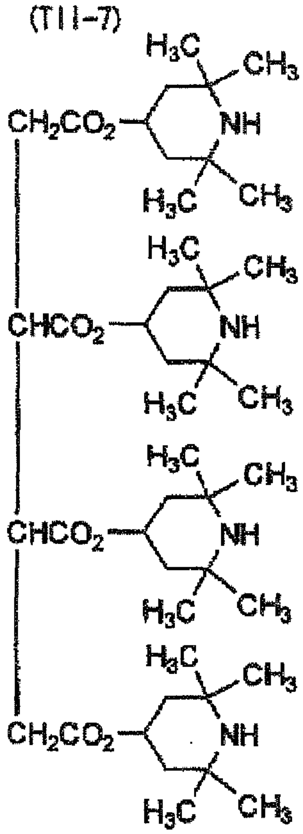

(TII-8)

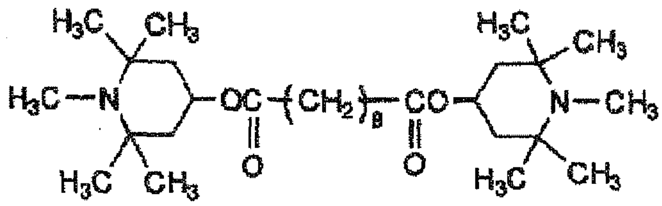

(TII-9)

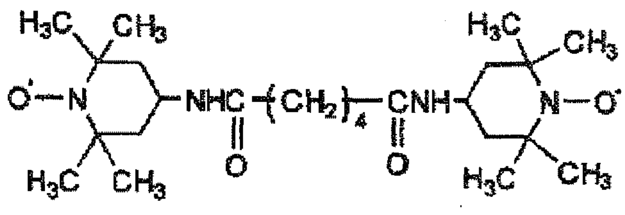

(TII-10)

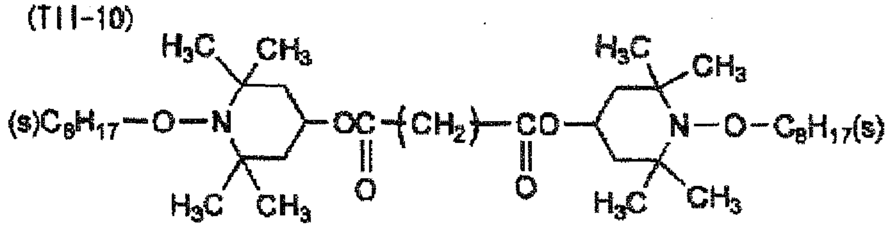

(TII-11)

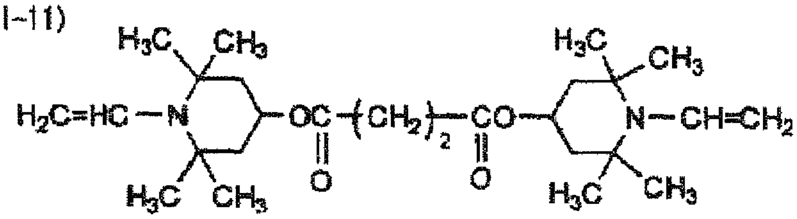

(TII-12)

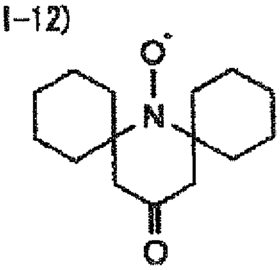

(TII-13)

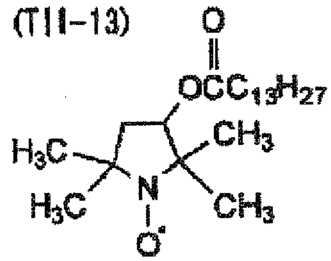

(TII-14)

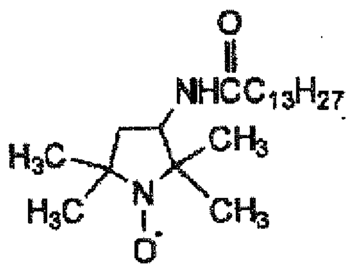

(TII-15)

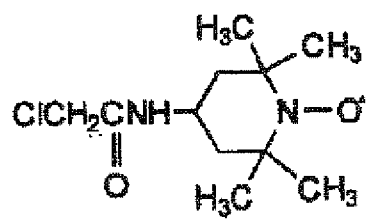

(TII-16)

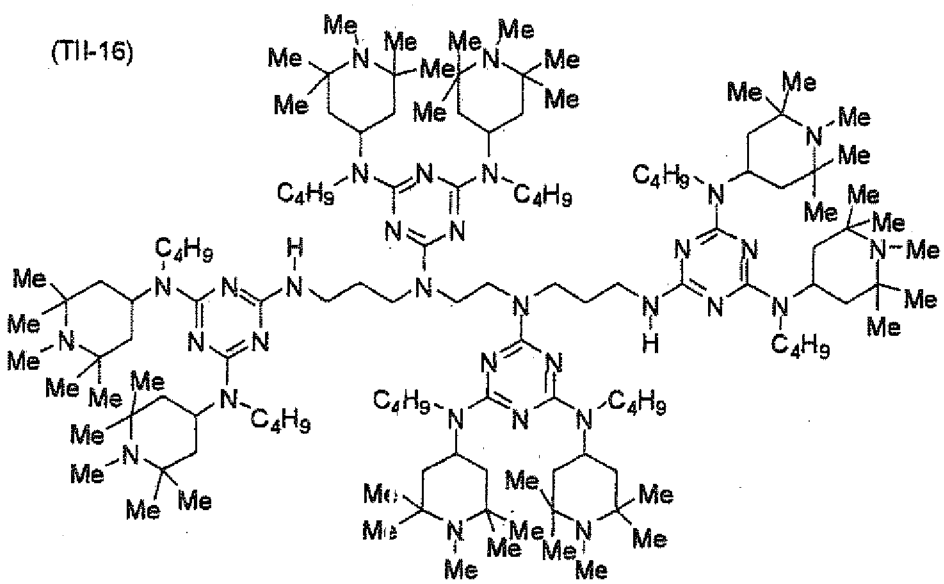

(TII-17)

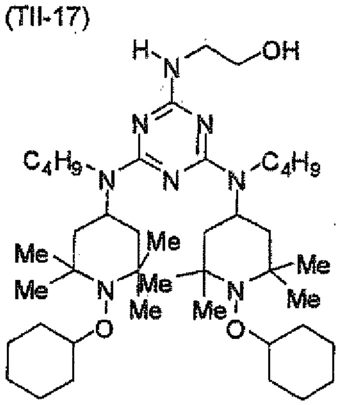

(TII-18)

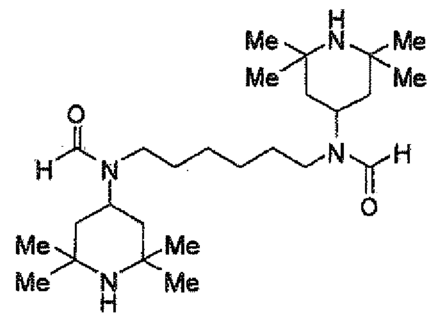

(TII-19)

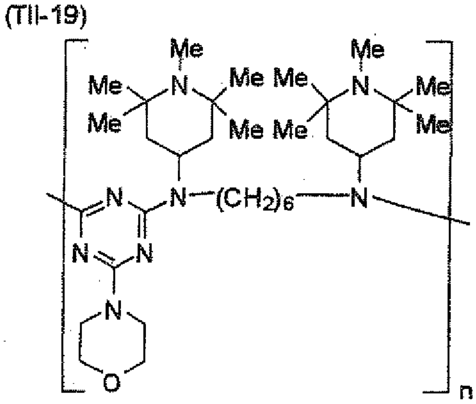

(TII-20)

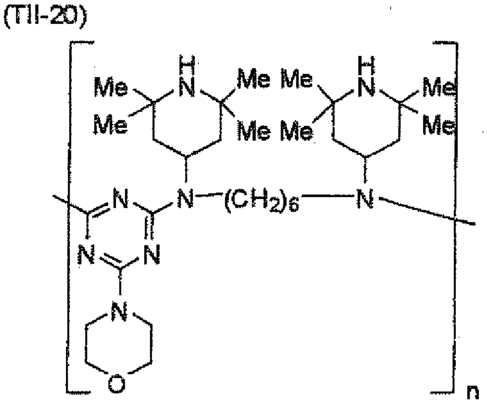

(TII-21)

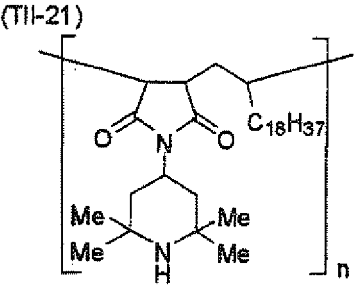

(TII-22)

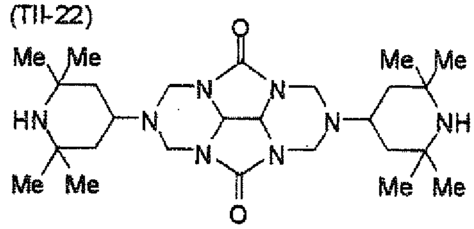

(TII-23)

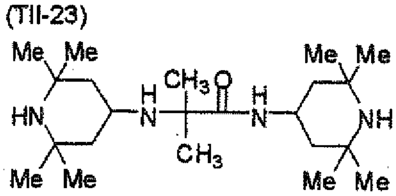

(TII-24)

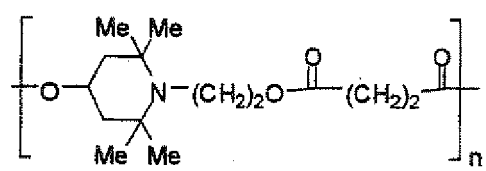

(TII-25)

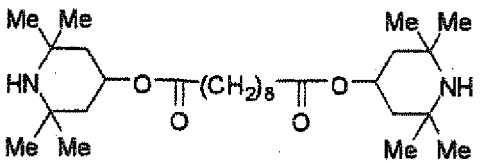

(TII-26)

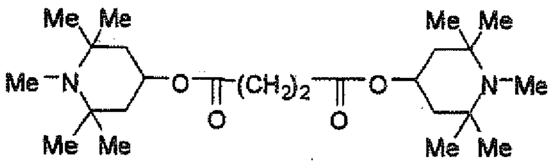

(TII-27)

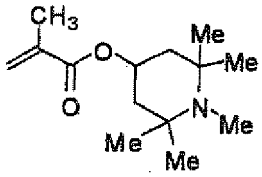

(TII-28)

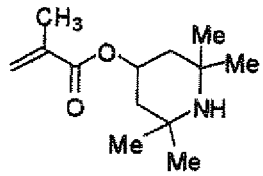

(TII-29)

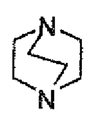

(TII-30)

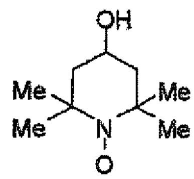

(TII-31)

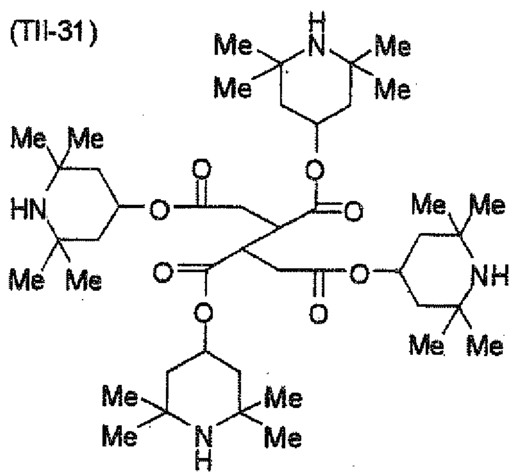

(TII-32)

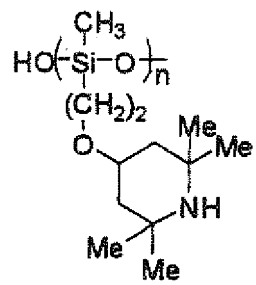

(TII-33)

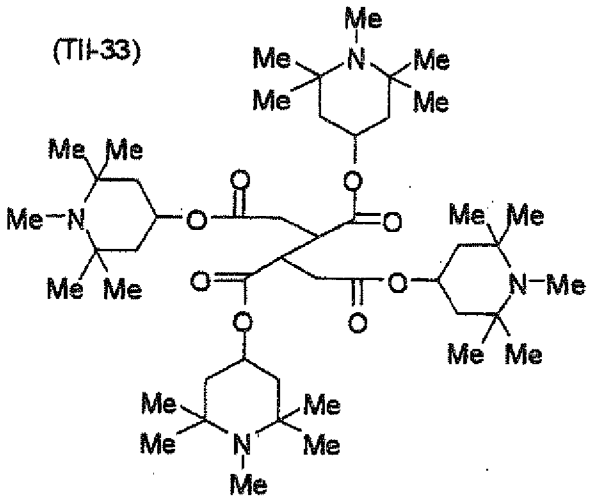

(TII-34)

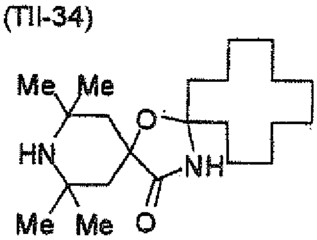

(TII-35)

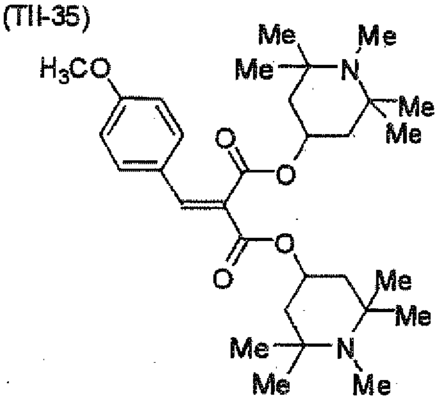

(TII-36)

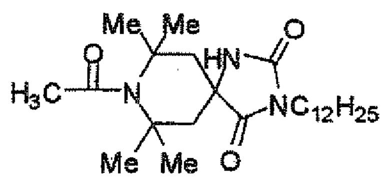

(TIII-1)

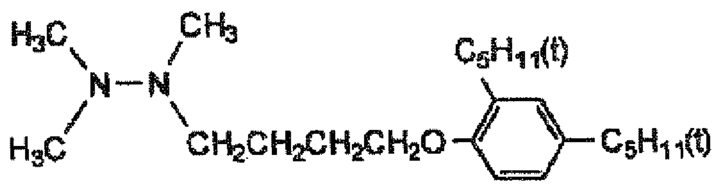

(TIII-2)

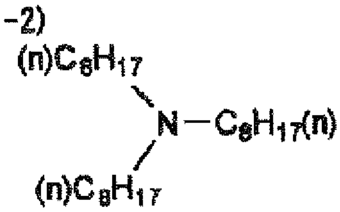

(TIII-3)

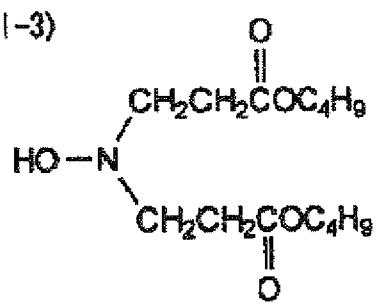

(TIII-4)

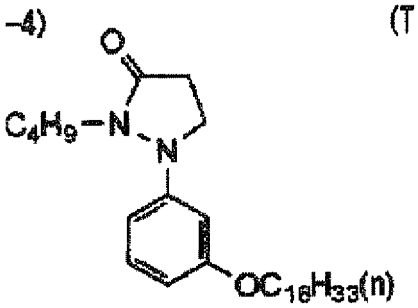

(TIII-5)

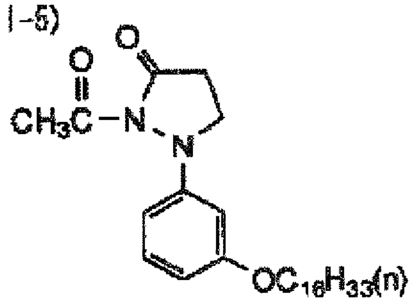

(TIII-6)

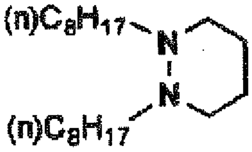

(TIII-7)

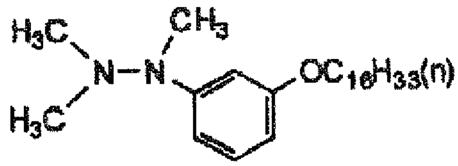

(TIII-8)

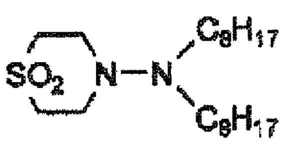

(TIII-9)

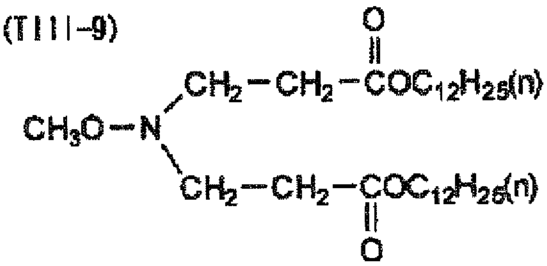

(TIII-10)

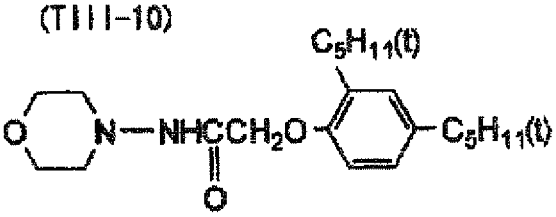

(TIII-11)

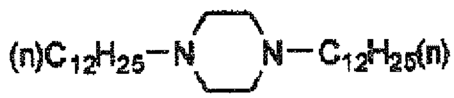

(TIII-12)

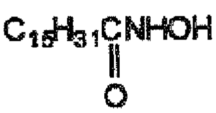

(TIII-13)

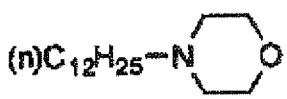

(TIV-1)

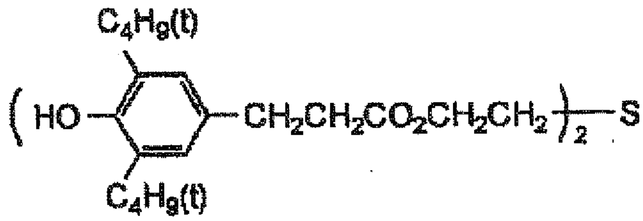

(TIV-2)

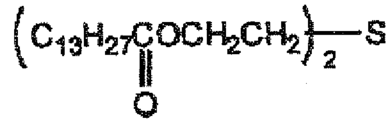

(TIV-3)

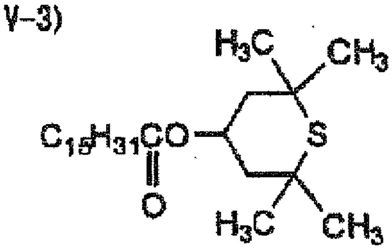

(TIV-4)

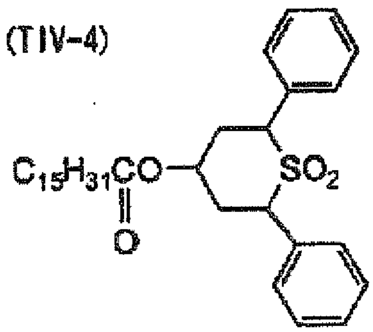

(TIV-5)

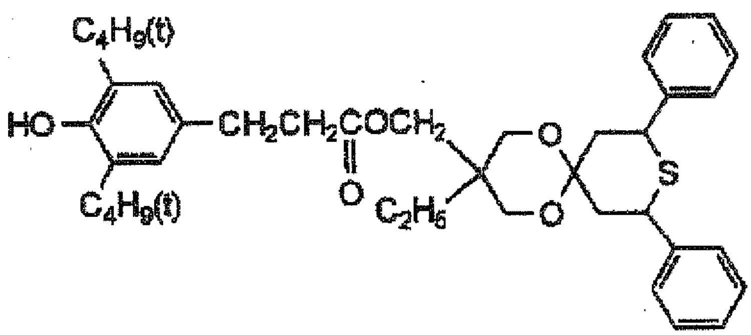

(TIV-6)

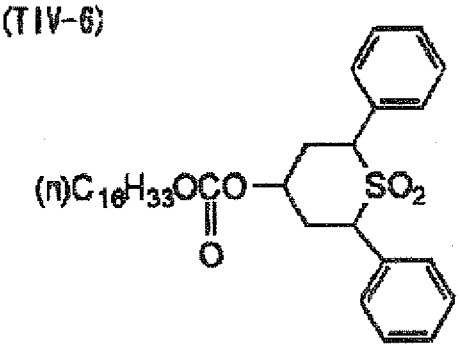

(TV-1)

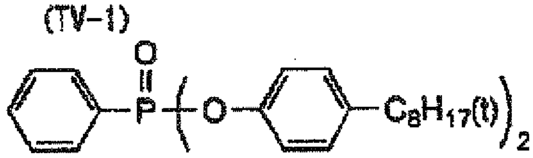

(TV-2)

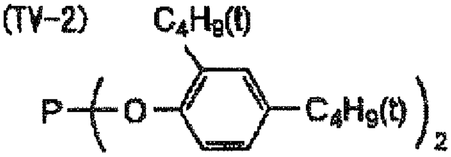

(TV-3)

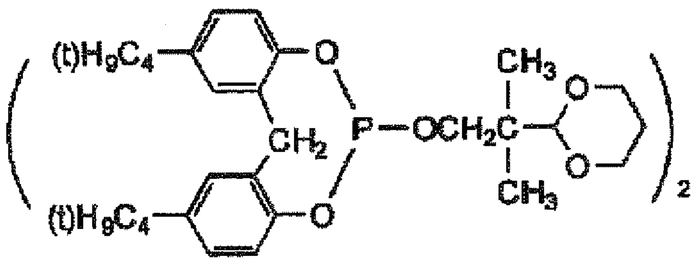

(TV-4)

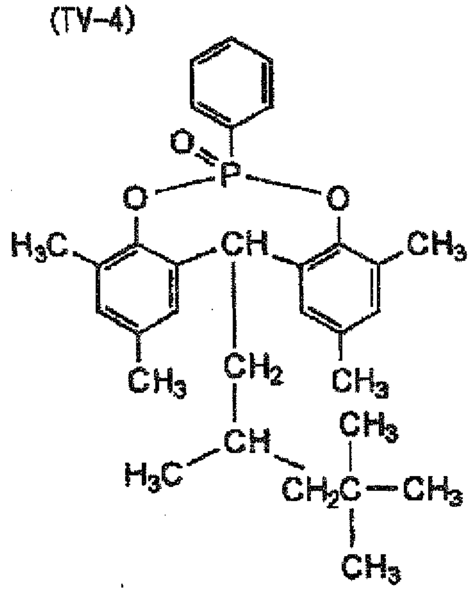

(TV-5)

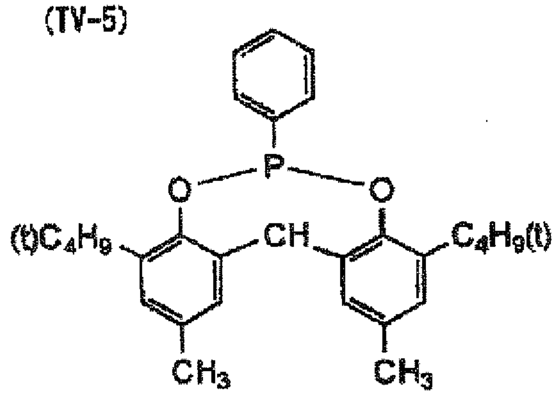

(TV-6)

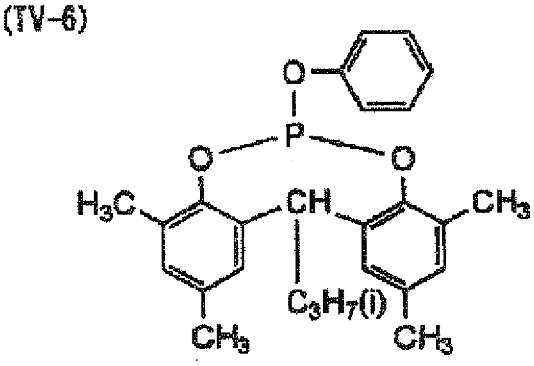

(TV-7)

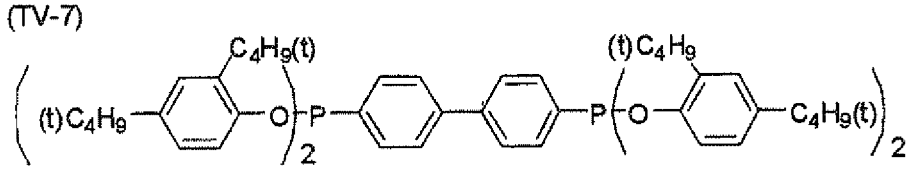

(TV-8)

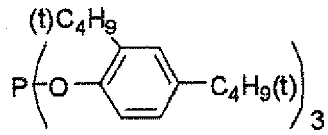

Among the compounds represented by any one of formulae (TS-I) to (TS-V), compounds represented by any one of formulae (TS-I), (TS-II) and (TS-V) are preferable; compounds represented by formula (TS-I) or (TS-II) are more preferable.

When the resin composition contains two or more compounds represented by any one of formulae (TS-I) to (TS-V), the two or more compounds may be selected from the same family (for example, that is the case where the two compounds represented by formula (TS-II) are used), or alternatively each of the two or more compounds may be selected from different families (for example, that is the case where one compound represented by formula (TS-I) and another compound represented by formula (TS-II) are used in combination). It is preferable that the two or more compounds, each of which is selected from different families, are used in combination.

In the present specification, the term "aliphatic group" means an alkyl group, a substituted alkyl group, an alkenyl group, a substituted alkenyl group, an alkynyl group, a substituted alkynyl group, an aralkyl group, and a substituted aralkyl group. The aforementioned alkyl group may have a branch or may form a ring. The alkyl group preferably has 1 to 20 carbon atoms, and more preferably 1 to 18 carbon atoms. The alkyl moiety in the aforementioned substituted alkyl group is the same as that of the above mentioned alkyl group. The aforementioned alkenyl group may have a branch or may form a ring. The alkenyl group has preferably 2 to 20 carbon atoms, and more preferably 2 to 18 carbon atoms. The alkenyl moiety in the aforementioned substituted alkenyl group is the same as that of the above mentioned alkenyl group. The aforementioned alkynyl group may have a branch or may form a ring. The alkynyl group has preferably 2 to 20 carbon atoms, and more preferably 2 to 18 carbon atoms. The alkynyl moiety in the aforementioned substituted alkynyl group is the same as that of the above mentioned alkynyl group. The alkyl moiety in the aforementioned aralkyl group and substituted aralkyl group is the same as that of the above mentioned alkyl group. The aryl moiety in the aforementioned aralkyl group and substituted aralkyl group is the same as that of the aryl group mentioned below.

Examples of the substituent in the alkyl moiety of the substituted alkyl group, the substituted alkenyl group, the substituted alkynyl group, and the substituted aralkyl group include: a halogen atom (e.g. a chlorine atom, a bromine atom, or an iodine atom); an alkyl group [which represents a substituted or unsubstituted linear, branched, or cyclic alkyl group, and which includes an alkyl group (preferably an alkyl group having 1 to 30 carbon atoms, e.g. methyl, ethyl, n-propyl, isopropyl, t-butyl, n-octyl, eicosyl, 2-chloroethyl, 2-cyanoethyl, or 2-ethylhexyl), a cycloalkyl group (preferably a substituted or unsubstituted cycloalkyl group having 3 to 30 carbon atoms, e.g. cyclohexyl, cyclopentyl, or 4-n-dodecylcyclohexyl), a bicycloalkyl group (preferably a substituted or unsubstituted bicycloalkyl group having 5 to 30 carbon atoms, i.e. a monovalent group obtained by removing one hydrogen atom from a bicycloalkane having 5 to 30 carbon atoms, e.g. bicyclo[1,2,2]heptan-2-yl or bicyclo[2,2,2]octan-3-yl), and a substituent having three or more ring structures such as tricyclo structure; and an alkyl group in the substituents described below (e.g. an alkyl group in an alkylthio group) represents such an alkyl group of the above concept];

an alkenyl group [which represents a substituted or unsubstituted linear, branched, or cyclic alkenyl group, and which includes an alkenyl group (preferably a substituted or unsubstituted alkenyl group having 2 to 30 carbon atoms, e.g. vinyl, allyl, prenyl, geranyl, or oleyl), a cycloalkenyl group (preferably a substituted or unsubstituted cycloalkenyl group having 3 to 30 carbon atoms, i.e. a monovalent group obtained by removing one hydrogen atom from a cycloalkene having 3 to 30 carbon atoms, e.g. 2-cyclopenten-1-yl or 2-cyclohexen-1-yl), and a bicycloalkenyl group (which represents a substituted or unsubstituted bicycloalkenyl group, preferably a substituted or unsubstituted bicycloalkenyl group having

5 to 30 carbon atoms, i.e. a monovalent group obtained by removing one hydrogen atom from a bicycloalkene having one double bond, e.g. bicydo[2,2,1]hept-2-en-1-yl or bicyclo[2,2,2]oct-2-en-4-yl)]; an alkynyl group (preferably a substituted or unsubstituted alkynyl group having 2 to 30 carbon atoms, e.g. ethynyl, propargyl, or trimethylsilylethynyl group);

an aryl group (preferably a substituted or unsubstituted aryl group having 6 to 30 carbon atoms, e.g. phenyl, p-tolyl, naphthyl, m-chlorophenyl, or o-hexadecanoylaminophenyl); a heterocyclic group (preferably a monovalent group obtained by removing one hydrogen atom from a substituted or unsubstituted 5- or 6-membered aromatic or nonaromatic heterocyclic compound; more preferably a 5- or 6-membered aromatic heterocyclic group having 3 to 30 carbon atoms, e.g. 2-furyl, 2-thienyl, 2-pyrimidinyl, 2-benzothiazolyl); a cyano group; a hydroxyl group; a nitro group; a carboxyl group; an alkoxy group (preferably a substituted or unsubstituted alkoxy group having 1 to 30 carbon atoms, e.g, methoxy, ethoxy, isopropoxy, t-butoxy, n-octyloxy, or 2-methoxyethoxy); an aryloxy group (preferably a substituted or unsubstituted aryloxy having 6 to 30 carbon atoms, e.g. phenoxy, 2-methylphenoxy, 4-t-butylphenoxy, 3-nitrophenoxy, or 2-tetradecanoylaminophenoxy);

a silyloxy group (preferably a silyloxy group having 3 to 20 carbon atoms, e.g. trimethylsilyloxy or t-butyldimethylsilyloxy); a heterocyclic oxy group (preferably a substituted or unsubstituted heterocyclic oxy group having 2 to 30 carbon atoms, e.g. 1-phenyltetrazol-5-oxy or 2-tetrahydropyranyloxy); an acyloxy group (preferably a formyloxy group, a substituted or unsubstituted alkylcarbonyloxy group having 2 to 30 carbon atoms, or a substituted or unsubstituted arylcarbonyloxy group having 6 to 30 carbon atoms, e.g. formyloxy, acetyloxy, pivaloyloxy, stearoyloxy, benzoyloxy, or p-methoxyphenylcarbonyloxy); a carbamoyloxy group (preferably a substituted or unsubstituted carbamoyloxy group having 1 to 30 carbon atoms, e.g. N,N-dimethylcarbamoyloxy, N,N-diethylcarbamoyloxy, morpholinocarbonyloxy, N,N-di-n-octylaminocarbonyloxy, or N-n-octylcarbamoyloxy);

an alkoxycarbonyloxy group (preferably a substituted or unsubstituted alkoxycarbonyloxy group having 2 to 30 carbon atoms, e.g. methoxycarbonyloxy, ethoxycarbonyloxy, t-butoxycarbonyloxy, or n-octylcarbonyloxy); an aryloxycarbonyloxy group (preferably a substituted or unsubstituted aryloxycarbonyloxy group having 7 to 30 carbon atoms, e.g. phenoxycarbonyloxy, p-methoxyphenoxycarbonyloxy, or p-n-hexadecyloxyphenaxycarbonyloxy); an amino group (preferably an amino group, a substituted or unsubstituted alkylamino group having 1 to 30 carbon atoms, or a substituted or unsubstituted anilino group having 6 to 30 carbon atoms, e.g. amino, methylamino, dimethylamino, anilino, N-methyl-anilino, or diphenylamino); an acylamino group (preferably a formylamino group, a substituted or unsubstituted alkylcarbonylamino group having 1 to 30 carbon atoms, or a substituted or unsubstituted arylcarbonylamino group having 6 to 30 carbon atoms, e.g. formylamino, acetylamino, pivaloylamino, lauroylamino, benzoylamino, or 3,4,5-tri-n-octyloxyphenylcarbonylamino);

an aminocarbonylamino group (preferably a substituted or unsubstituted aminocarbonylamino having 1 to 30 carbon atoms, e.g. carbamoylamino, N,N-dimethylaminocarbonylamino, N,N-diethylaminocarbonylamino, or morpholinocarbonylammo); an alkoxycarbonylamino group (preferably a substituted or unsubstituted alkoxycarbonylamino group having 2 to 30 carbon atoms, e.g. methoxycarbonylamino, ethoxycarbonylamino, t-butoxycarbonylamino, n-octadecyloxycarbonylamino, or N-methyl-methaxycarbonylamino); an aryloxycarbonylamino group (preferably a substituted or unsubstituted aryloxycarbonylamino group having 7 to 30 carbon atoms, e.g. phenoxycarbonylamino, p-chlorophenoxycarbonylamino, or m-n-octyloxyphenoxycarbonylamino); a sulfamoylamino group (preferably a substituted or unsubstituted sulfamoylamino group having 0 to 30 carbon atoms, e.g. sulfamoylamino, N,N-dimethylaminosulfonylamino, or N-n-octylaminosulfonylamino);

an alkyl- or aryl-sulfonylamino group (preferably a substituted or unsubstituted alkylsulfonylamino having 1 to 30 carbon atoms, or a substituted or unsubstituted arylsulfonylamino having 6 to 30 carbon atoms, e.g. methylsulfonylamino, butylsulfonylamino, phenylsulfonylamino, 2,3,5-trichlorophenylsulfonylamino, or p-methylphenylsulfonylamino); a mercapto group; an alkylthio group (preferably a substituted or unsubstituted alkylthio group having 1 to 30 carbon atoms, e.g. methylthio, ethylthio, or n-hexadecylthio); an arylthio group (preferably a substituted or unsubstituted arylthio having 6 to 30 carbon atoms, e.g. phenylthio, p-chlorophenylthio, or m-methoxyphenylthio); a heterocyclic thio group (preferably a substituted or unsubstituted heterocyclic thio group having 2 to 30 carbon atoms, e.g. 2-benzothiazolylthio or 1-phenyltetrazol-5-ylthio); a sulfamoyl group (preferably a substituted or unsubstituted sulfamoyl group having 0 to 30 carbon atoms, e.g. N-ethylsulfamoyl, N-(3-dodecyloxypropyl)sulfamoyl, N,N-dimethylsulfamoyl, N-acetylsulfamoyl, N-benzoylsulfamoyl, or N-(N'-phenylcarbamoyl)sulfamoyl); a sulfo group;

an alkyl- or aryl-sulfinyl group (preferably a substituted or unsubstituted alkylsulfinyl group having 1 to 30 carbon atoms, or a substituted or unsubstituted arylsulfinyl group having 6 to 30 carbon atoms, e.g. methylsulfinyl, ethylsulfinyl, phenylsulfinyl, or p-methylphenylsulfinyl); an alkyl- or aryl-sulfonyl group (preferably a substituted or unsubstituted alkylsulfonyl group having 1 to 30 carbon atoms, or a substituted or unsubstituted arylsulfonyl group having 6 to 30 carbon atoms, e.g. methylsulfonyl, ethylsulfonyl, phenylsulfonyl, or p-methylphenylsulfonyl); an acyl group (preferably a formyl group, a substituted or unsubstituted alkylcarbonyl group having 2 to 30 carbon atoms, a substituted or unsubstituted arylcarbonyl group having 7 to 30 carbon atoms, or a substituted or unsubstituted heterocyclic carbonyl group having 4 to 30 carbon atoms, in which the heterocycle binds to said carbonyl group through a carbon atom, e.g. acetyl, pivaloyl, 2-chloroacetyl, stearoyl, benzoyl, p-n-octyloxyphenylcarbonyl, 2-pyridylcarbonyl, or 2-furylcarbonyl);

an aryloxycarbonyl group (preferably a substituted or unsubstituted aryloxycarbonyl group having 7 to 30 carbon atoms, e.g. phenoxycarbonyl, o-chlorophenoxycarbonyl, m-nitrophenoxycarbonyl, or p-t-butylphenoxycarbonyl); an alkoxycarbonyl group (preferably a substituted or unsubstituted alkoxycarbonyl group having 2 to 30 carbon atoms, e.g. methoxycarbonyl, ethoxycarbonyl, t-butoxycarbonyl, or n-octadecyloxycarbanyl); a carbamoyl group (preferably a substituted or unsubstituted carbamoyl group having 1 to 30 carbon atoms, e.g. carbamoyl, N-methylcarbamoyl, N,N-dimethylcarbamoyl, N,N-di-n-octylcarbamoyl, or N-(methylsulfonyl)carbamoyl); an aryl- or heterocyclic-azo group (preferably a substituted or unsubstituted aryl azo group having 6 to 30 carbon atoms, or a substituted or unsubstituted heterocyclic azo group having 3 to 30 carbon atoms, e.g. phenylazo, p-chlorophenylazo, or 5-ethylthio-1,3,4-thiadiazol-2-ylazo); an imido group (preferably N-succinimido or N-phthalimido);

a phosphino group (preferably a substituted or unsubstituted phosphino group having 2 to 30 carbon atoms, e.g. dimethylphosphino, diphenylphosphino, or methylphenoxyphosphino); a phosphinyl group (preferably a substituted or unsubstituted phosphinyl group having 2 to 30 carbon atoms, e.g. phosphinyl, dioctyloxyphosphinyl, or diethoxyphosphinyl); a phosphinyloxy group (preferably a substituted or unsubstituted phosphinyloxy group having 2 to 30 carban atoms, e.g. diphenoxyphosphinyloxy or dioctyloxyphosphinyloxy); a phosphinylamino group (preferably a substituted or unsubstituted phosphinylamino group having 2 to 30 carbon atoms, e.g. dimethoxyphosphinylamino or dimethylaminophosphinylamino); and a silyl group (preferably a substituted or unsubstituted silyl group having 3 to 30 carbon atoms, e.g. trimethylsilyl, t-butyldimethylsilyl, or phenyldimethylsilyl).

Among the functional groups, with respect to one having a hydrogen atom, the hydrogen atom may be removed and said one may be further substituted by any of the above-mentioned substituents. Examples thereof include: an alkylcarbonylaminosulfbnyl group, an arylcarbonylaminosulfonyl group, an alkylsulfonylaminocarbonyl group, and an arylsulfonylaminocarbonyl group. Examples thereof include a methylsulfonylaminocarbonyl group, a p-methylphenylsulfonylaminocarbonyl group, an acetylaminosulfonyl group, and a benzoylaminosulfonyl group.

Examples of a substituent of the aryl portion of the substituted aralkyl group are similar to the examples of a substituent of the substituted aryl groups mentioned later.

In this specification, the term "aromatic group" means an aryl group and a substituted aryl group. To the aromatic groups, an aliphatic ring, another aromatic ring, or a heterocycle may be condensed. The aromatic group preferably has 6 to 40 carbon atoms, more preferably 6 to 30 carbon atoms, and even more preferably 6 to 20 carbon atoms. Among them, phenyl or naphthyl is preferable as the aryl group, and phenyl is particularly preferable.

The aryl portion of the substituted aryl group is similar to the above-mentioned aryl groups. Examples of a substituent of the substituted aryl groups are similar to the above-mentioned examples of the substituent of the alkyl portions of the substituted alkyl group, the substituted alkenyl group, the substituted alkynyl group, and the substituted aralkyl group.

In this specification, the heterocyclic groups preferably contain a 5-membered or 6-membered, saturated or unsaturated heterocycle. To the heterocycle, an aliphatic ring, an aromatic ring, or another heterocycle may be condensed. Examples of a heteroatom of the heterocycle include B, N, O, S, Se, and Te. As the heteroatom, N, O, and S are preferable. It is preferable that a carbon atom of the heterocycle has a free valence (monovalent) (the heterocyclic group is preferably to be bonded at a carbon atom thereof). The heterocyclic group preferably has 1 to 40 carbon atoms, more preferably 1 to 30 carbon atoms, and even more preferably 1 to 20 carbon atoms. Examples of the saturated heterocycle include a pyrrolidine ring, a morpholine ring, a 2-bora-1,3-dioxolane ring, and 1,3-thiazolidine ring. Examples of the unsaturated heterocycles include an imidazole ring, a thiazole ring, a benzothiazole ring, a benzoxazole ring, a benzotriazole ring, a benzoselenazole ring, a pyridine ring, a pyrimidine ring, and a quinoline ring. The heterocyclic groups may have a substituent. Examples of the substituent are similar to the previously-mentioned examples of the substituent of the alkyl portions of the substituted alkyl group, the substituted alkenyl group, the substituted alkynyl group, and the substituted aralkyl group.

Hereinafter, the bluing agent D used in the present invention is explained.
The term "bluing agent" in the present invention means a coloring agent which absorbs light within the range from orange to yellow to show blue to purple. In order to further reduce a yellow tint in the resin composition used in the present invention and to give a natural transparent feeling to a molded product, use of the bluing agent is very effective. As the bluing agent D used in the present invention, various compounds can be used and examples thereof include an anthraquinone-based compound, a phthalocyanin-based compound, a monoazo-based compound, a diazo-based compound, and a triallylmethane-based compound. Specific examples of the bluing agent include Solvent Violet 13 (generic name) [CA. No (color index number) 60725; Microlex Violet B manufactured by Bayer Holding Ltd., Dia Resin Blue G manufactured by Mitsubishi Chemical Co., Ltd., Sumiplast Violet B manufactured by Sumitomo Chemical Co., Ltd.; Plast Violet 8840 manufactured by Arimoto Chemical Co., Ltd.; KP Plast Violet 2R manufactured by Kiwa Chemical Industry Co., Ltd.; and Quinizarin Blue manufactured by Tokyo Chemical Industry Co., Ltd., each trade names], Solvent Violet 31 (generic name) [CA. No. 68210; trade name: Dia Resin Violet D manufactured by Mitsubishi Chemical Co., Ltd.], Solvent Violet 33 (generic name) [CA. No. 60725; trade name: Dia Resin Blue J manufactured by Mitsubishi Chemical Co., Ltd.], Solvent Blue 94 (generic name) [CA. No. 61500; trade name: Dia Resin Blue N manufactured by Mitsubishi

Chemical Co., Ltd.], Solvent Violet 36 (generic name) [CA. No. 68210; trade name: MicroiexVinlet 3R manufactured by Bayer Holding Ltd.], Solvent Blue 97 (generic name) [trade name: Microlex Blue RR manufactured by Bayer Holding Ltd.], and Solvent Blue 45 (generic name) [CA. No. 61110; trade name: Polysynthren Blue RLS manufactured by Sand Co., Ltd.].

The content of the ultraviolet absorbent A to the resin in the resin composition of the present invention is not particularly limited. However, relative to 100 parts by mass of the resin, the content is preferably 0.01 to 20 parts by mass, more preferably 0.01 to 10 parts by mass, and still more preferably 0.01 to 5 parts by mass.

The resin composition of the present invention preferably contains three or less kinds of the ultraviolet absorbent B, more preferably two kinds the ultraviolet absorbent B, and particularly preferably one kind of the ultraviolet absorbent B. Plural kinds of the compound C can be contained.

In the ultraviolet absorbent composition of the present invention, each of the mixing ratios of the ultraviolet absorbents A and B as well as the compound C can be any ratio. The content ratio of the ultraviolet absorbent A to the ultraviolet absorbent B (A:B) may be arbitrary excluding 0:1 1 and 1:0. It is preferably 1:10 to 10:1, more preferably 6: 1 to 1:4, most preferably 4:1 to 1:4. The content ratio in the present invention is expressed by molar ratio. It is possible to convert the molar ratio to the mass ratio, when the molecular weight of the ultraviolet absorbent is known, and thus, a person ordinary skilled in the art can mix the ingredients based on the mass ratio. The content of the compound C in the resin composition of the present invention is preferably 0 to 10, more preferably 0 to 7, still more preferably 0 to 5 and furthermore preferably 0 to 2 in terms of the mole number ratio of the compound C when the smaller value, among the mole number of the ultraviolet absorbent A and the mole number of the ultraviolet absorbent B, is set to 1 (standard value).

The content of the bluing agent D in the resin composition of the present invention is not particularly limited. However, relative to 100 parts by mass of the ultraviolet absorbent A, it is preferably 0.00001 to parts by mass. The content is more preferably 0.01 to 1 parts by mass, still more preferably 0.03 to 1 parts by mass, even still more preferably 0.05 to 0.8 parts by mass, and most preferably 0.05 to 0.5 parts by mass.

Hereinafter, a polymer substance used in the resin composition of the present invention is described in detail. In the present invention, the polymer substance used in the present invention is not particularly limited, but it includes, for example, a natural or synthetic polymer or copolymer. Examples thereof include the followings.

<1> Monoolefinic and diolefinic polymers such as polypropylene, polyisobutylene, polybut-1-ene, poly-4-methyl pent-1-ene, polyvinylcyclohexane, polyisoprene, and polybutadiene; cycloolefin polymers such as of cyclopentene or norbornene; polyethylenes (crosslinkable as needed) such as high-density polyethylene (HDPE), high-density and high-molecular weight polyethylene (HDPE-HMW), high-density and ultrahigh molecular weight polyethylene (HDPE-UHMW), medium-density polyethylene (MDPE), low-density polyethylene (LDPE), and linear low-density polyethylene (LLDPE), (VLDPE) and (ULDPE).

Polyolefins (that is, polymers of the monoolefins exemplified in the paragraph above), preferably polyethylene and polypropylene, may be prepared by various methods, particularly by the following methods:

a) Radical polymerization (normally under high pressure and elevated temperature); and

b) Catalytic polymerization normally by using a catalyst including one or more metals in the group IVb, Vb, VIb or VIII of the Periodic Table. These metals normally have one or more ligand, typical examples thereof include ligands capable of $\pi$- or $\sigma$-coordinating, such as oxide, halide, alcoholate, ester, ether, amine, alkyl, alkenyl and/or aryl. The metal complex is in the free state or may be immobilized on a base material such as activated magnesium chloride, titanium (III) chloride, alumina, or silicon oxide. The catalyst may be soluble or insoluble in the polymerization medium. The catalyst may be used as it is in polymerization or in combination with another activating agent, such as metal alkyl, metal hydride, metal alkyl halide, metal alkyl oxide, or metal alkyloxane, whose metal moiety is an element in the groups Ia, IIa and/or IIIa of the Periodic Table. The activating agent may be modified properly with another ester, ether, amine, or silylether group. Such a catalyst system is normally called Philips, Standard Oil-Indiana, Ziegler (Natta), TNZ (Du Pont), metallocene or single site catalyst (SSC).

<2> Mixture of the polymers described in <1> above such as polypropylene/polyisobutylene mixture, polypropylene/polyethylene mixture (such as PP/HDPE and PP/LDPE), and mixture of different type of polyethylenes (such as LDPE/HDPE).

<3> Copolymers of a monoolefin and a diolefin or a monoolefin or diolefin with another vinyl monomer such as ethylene/propylene copolymer, mixture of linear low-density polyethylene (LLDPE) and its low-density polyethylene (LDPE), propylene/but-1-ene copolymer, propylenelisobutylene copolymer, ethylenelbut-1-ene copolymer, ethylene/hexene copolymer, ethylene/methylpentene copolymer, ethylene/heptene copolymer, ethylene/octene copolymer, ethylene/vinylcyclohexane copolymer, ethylene/cycloolefin copolymer (for example, COC (Cyclo-Olefin Copolymer) of ethylenelnorbornene), ethylene/1-olefin copolymer producing 1-olefin in situ, propylene/butadiene copolymer, isobutylene/isoprene copolymer, ethylene/vinylcyclohexene copolymer, ethylene/alkyl acrylate copolymer, ethylene/alkyl methacrylate copolymer, ethylene/vinyl acetate copolymer or ethylene/acrylic acid copolymer and the salts thereof (ionomers); and terpolymers of ethylene and propyrene with diene such as hexadiene, dicyclopentadiene or ethylidene-norbornene; and mixtures of such copolymers and the polymer described in the above 1), for example, polypropylene/ethylene-propylene copolymer, LDPE/ethylene-vinyl acetate copolymer (EVA), LDPE/ethylene-acrylic acid copolymer (EAA), LLDPE/EVA, LLDPE/EAA and alternating or random polyalkylene/carbon monooxide copolymer and the mixture thereof with other polymer such as polyamide.

<4> Mixtures of hydrocarbon resins (for example, a hydrocarbon resin having 5 to 9 carbon atoms) containing hydrogenated derivatives (for example, tackifier) and polyalkylene and starch.

The homopolymers and copolymers described in <1> to <4> above may have any steric structure such as, syndiotactic structure, isotactic structure, hemiisotactic structure, or atactic structure; and atactic polymers are preferable. Stereoblock polymers are also included.

<5> Polystyrene, poly(p-methylstyrene), and poly($\alpha$-methylstyrene).

<6> Aromatic homopolymer and copolymers derived from aromatic vinyl monomers including all isomers of styrene, $\alpha$-methylstyrene, and vinyltoluene, particularly all isomers of p-vinyltoluene, ethylstyrene, propylstyrene, vinyl biphenyl, vinylnaphthalene, and vinylanthracene, and the mixture thereof. The homopolymers and copolymers may have any steric structure such as syndiotactic structure, isotactic structure, hemiisotactic structure, or atactic structure; and atactic polymers are preferable. Stereoblock polymers are also included.

<6a> Copolymers of the aromatic vinyl monomers or comonomers selected from ethylene, propylene, diene, nitriles, acids, maleic anhydride, maleimide, vinyl acetate and vinyl chloride or its acryl derivative and the mixture thereof, such as styrene/butadiene, styrene/acrylonitrile, styrene/ethylene (copolymer), styrene/alkyl methacrylate, styrene/butadiene/alkyl acrylate, styrene/butadiene/alkyl methacrylate, styrene/maleic anhydride, and styrene/acrylonitrile/methyl acrylate; styrene copolymers and other polymers including high shock-resistant mixtures such as polyacrylate, diene polymer, and ethylene/propylene/diene terpolymer; and styrene block copolymers such as styrene/butadiene/styrene, styrene/isoprene/styrene, styrene/ethylene/butylene/styrene and styrene/ethylene/propylene/styrene.

<6b> Hydrogenated aromatic polymers derived from the hydrogenated polymers described in <6> above, particularly polycyclohexylethylene (PCHE), often called as polyvinylcyclohexane (PVCH), prepared by hydrogenating atactic polystyrene.

<6c> Hydrogenated aromatic polymers prepared by hydrogenating the polymers described in <6a> above.

The homopolymers and copolymers may have any steric structure such as syndiotactic structure, isotactic structure, hemiisotactic structure, or atactic structure, and atactic polymers are preferable. Stereoblock polymers are also included.

<7> Graft copolymers of an aromatic vinyl monomer such as styrene or $\alpha$-methylstyrene, including graft copolymers of polybutadiene/styrene; polybutadienestyrene or polybutadiene-acrylonitrile copolymer/styrene; polybutadiene/styrene and acrylonitrile (or methacrylonitrile); polybutadiene/styrene, acrylonitrile and methyl methacrylate; polybutadiene/styrene and maleic anhydride; polybutadiene/styrene, acrylonitrile and maleic anhydride or maleimide; polybutadiene/styrene and maleimide; polybutadiene/styrene and alkyl acrylate or methacrylate; ethylene/propylene/diene terpolymer/styrene and acrylonitrile; polyalkyl acrylate or polyalkyl methacrylate/styrene and acrylonitrile; acrylate/butadiene copolymer/styrene and acrylonitrile; and mixtures thereof with the copolymers described in <6> above such as known copolymer mixtures of ABS, SAN, MBS, ASA and AES polymer.

<8> Halogen-containing polymers such as polychloroprene, chlorinated rubber, chlorinated or brominated copolymers of isobutylene-isaprene (halobutyl rubbers), chlorinated or sulfochlorinated polyethylene, ethylene-chlorinated ethylene copolymer, and epichlorohydrin homopolymer and copolymers; particularly, polymers of a halogen-containing vinyl compound, for example, polyvinyl chloride, polyvinylidene chloride, polyvinyl fluoride, polyvinylidene fluoride, and copolymers thereof such as polyvinyl chloride/vinylidene chloride, polyvinyl chloride/vinyl acetate or vinylidene chloride/vinyl acetate copolymer.

<9> Polymers derived from $\alpha,\beta$-unsaturated acid and the derivatives thereof such as polyacrylates and polymethacrylates; and polymethyl methacrylate, polyacrylamide and polyacrylonitrile, whose resistance to shock is improved by butyl acrylate.

<10> Copolymers of the monomers described in <9> above or with another unsaturated monomer such as acrylonitrile/butadiene copolymer, acrylonitrile/alkyl acrylate copolymer, acrylonitrile/alkoxyalkyl acrylate, acrylonitrile/vinyl halide copolymer and acrylonitrile/alkyl methacrylate/butadiene terpolymer.

<11> Polymers derived from an unsaturated alcohol and an amine, and acyl derivatives or acetals thereof such as polyvinylalcohol, polyvinyl acetate, polyvinyl stearate, polyvinyl benzoate, polyvinyl maleate, polyvinylbutyral, polyallyl phthalate and polyallylmelamine; and copolymers thereof with the olefin described in <1> above.

<12> Homopolymers and copolymers of cyclic ether such as polyalkylene glycols, polyethyleneoxide, polypropyleneoxide, bisglycidylether, and the copolymers thereof.

<13> Polyacetals such as polyoxymethylene and polyoxymethylene containing ethyleneoxide as a comonomer; thermoplastic polyurethane and polyacetals modified with acrylate or MOBS.

<14> Mixtures of polyphenyleneoxide and sulfide, and those of polyphenyleneoxide and styrene polymer or polyamide.

<15> Polyurethanes derived from a polyether, polyester, or polybutadiene having a hydroxyl group terminal and an aliphatic or aromatic polyisocyanate, and the precursors thereof.

<16> Polyamides and copolyamides derived from a diamine and a dicarboxylic acid and/or aminocarboxylic acid or the corresponding lactam, for example, polyamide 4, polyamide 6, polyamides 6/6, 6/10, 6/9, 6/12, 4/6 and 12/12, polyamide 11, polyamide 12, and an aromatic polyamide from m-xylenediamine and adipic acid; polyamides prepared from hexamethylenediamine and isophthalic and/or terephthalic acid, in the presence or absence of a modifying agent elastomer, for example, poly-2,4,4-trimethylhexamethylene terephthalamide and poly-m-phenylene isophthalamide; block copolymers of the polyamides above with polyolefin, olefin copolymer, ionomer or chemically bonded or grafted elastomer; block copolymers of the polyamides above with polyether such as polyethylene glycol, polypropylene glycol, or polytetramethylene glycol; polyamides or copolyamides modified with EPDM or ABS; and polyamides condensed during processing (RIM polyamides).

<17> Polyurea, polyimide, polyamide-imide, polyether imide, polyester-imide, polyhydantoin, and polybenzimidazole.

<18> Polyesters derived from a dicarboxylic acid and a diol and/or a hydroxycarboxylic acid or the corresponding lactone, for example, polyethylene terephthalate, polybutylene terephthalate, poly-1,4-dimethylolcyclohexane terephthalate, polyalkylene naphthalate (PAN), and polyhydroxybenzoate; block copolyether esters derived from hydroxyl terminal polyethers; and polyesters modified with polycarbonate or MBS; the polyesters and polyester copolymers specified in U.S. Patent No. 5,807,932 (2nd column, line 53) are also incorporated herein by reference.

<19> Polycarbonates and polyester carbonates.

<20> Polyketones.

<21> Polysulfones, polyether sulfones, and polyether ketones.

<22> Crosslinked polymers derived from an aldehyde component and another phenol component and also from urea and melamine, for example, phenol/formaldehyde resin, urea/formaldehyde resin, and melamine/formaldehyde resin.

<23> Dry and non-dry alkyd resins.

<24> Unsaturated polyester resins derived from saturated and unsaturated dicarboxylic acids, a polyvalent alcohol, and a vinyl compound as a crosslinking agent, and less flammable halogen-containing derivatives thereof.

<25> Substituted acrylates, for example, crosslinkable acrylic resins derived from epoxy acrylate, urethane acrylate, or polyester acrylate.

<26> Alkyd resin, polyester resin, and acrylate resin crosslinked with a melamine resin, urea resin, isocyanate, isocyanurate, polyisocyanate, or epoxy resin.

<27> Crosslinked epoxy resins derived from an aliphatic, alicyclic, heterocyclic, or aromatic glycidyl compound, for example, glycidyl ether products of bisphenol A or bisphenol F crosslinked with a common curing agent such as anhydride or amine in the presence or absence of an accelerator.

<28> Natural polymers such as cellulose, rubber, gelatin, and the chemically-modified homologous derivatives thereof such as cellulose acetate, cellulose propionate, cellulose butyrate, and cellulose ethers such as methylcellulose; and rosins and the derivatives thereof.

<29> Blends (polyblends) of the polymers described above such as PP/EPDM, polyamide/EPDM or ABS, PVC/EVA, PVC/ABS, PVC/MBS, PC/ABS, PBTP/ABS, PC/ASA, PC/PBT, PVC/CPE, PVC/acrylate, POM/thermoplastic PUR, PC/thermoplastic PUR, POM/acrylate, POM/MBS, PPO/HIPS, PPO/PA6.6 and copolymer, PA/HDPE, PA/PP, PA/PPO, PBT/PC/ABS and PBT/PET/PC.

<30> Natural and synthetic organic materials of a pure monomer compound or a mixture of the compounds, for example, mineral oils, animal and vegetable fats, oils and waxes, or synthetic ester (for example, phthalate, adipate, phosphate or trimellitate)-based oils, fats and waxes, and mixtures thereof with a synthetic ester and mineral oil at any mass ratio, mixtures typically used as a fiber-spinning composition, and the aqueous emulsions thereof.

<31> Aqueous emulsions of natural or synthetic rubber, for example, a natural latex or latexes of a carboxylated styrene/butadiene copolymer.

<32> Polysiloxanes, for example, the soft hydrophilic polysiloxane described in U.S. Patent No. 4,259,467 and the hard polyorganosiloxane described in U.S. Patent No. 4,355,147.

<33> Polyketimines in combination with an unsaturated acrylpolyacetoacetate resin or an unsaturated acrylic resin including urethane acrylate, polyester acrylate, vinyl or acryl copolymers having a pendant unsaturated group, and acrylated melamines. The polyketimine is prepared from a polyamine and a ketone in the presence of an acid catalyst.

<34> Radiant ray-hardening compositions containing an ethylenically unsaturated monomer or oligomer and a polyunsaturated aliphatic oligomer.

<35> Epoxy melamine resins such as photostabilized epoxy resins crosslinked with a coetherified high-solid content melamine resin sensitive to epoxy groups, such as LSE-4103 (trade name, manufactured by Monsanto company).

The polymer substance used in the present invention is preferably a synthetic polymer, more preferably a polyolefin, an acrylic polymer, polyester, polycarbonate, or a cellulose ester. Among them, polyethylene, polypropylene, poly(4-methylpentene), polymethyl methacrylate, polycarbonate, polyethylene terephthalate, polyethylene naphthalate, polybutylene terephthalate, and triacetylcellulose are particularly preferable.
The polymer substance used in the present invention is preferably a thermoplastic resin.

As the acrylic resin used in the present invention, various resins including commercially available products can be used. For example, HI-PET HBS-000 (trade name) manufactured by MITSUBISHI RAYON CO., LTD. and ACRYPET VH001 (trade name) manufactured by MITSUBISHI RAYON CO., LTD. can be used. In addition, as the polycarbonate resin used in the present invention, various resins including commercially available products can be used. For example, PANLITE L-1125Y (trade name) manufactured by Teijin Chemicals Ltd. can be used. In addition, as the polyester resin used in the present invention, various resins including commercially available products can be used. For example, PETMAX PET resin (trade name) manufactured by TOYOBO CO., LTD. can be used. In addition, as the polyolefin resin used in the present invention, various resins including commercially available products can be used. For example, NOVATEC LD (trade name) manufactured by Japan Polyethylene Corporation can be used.

It is particularly preferable that the resin composition of the present invention contains at least one resin selected from the group consisting of an acrylic resin, a polycarbonate resin, a polyester resin, and a polyolefin resin.

According to the necessary, various optional additives can be added to the resin composition of the present invention by any method within a range in which the performance of the resin composition is not compromised. Examples of the additives include a coloring agent such as a dye and a pigment; a spreading agent or a dispersing agent to improve the dispersability of the pigment or a light diffusing agent; a modifying agent for impact resistance represented by a rubber-type polymer having a core-shell form graft structure, in which acrylic acid esters or methacrylic acid esters are included as a main component; a flame retardant such as phosphoric acid ester; a lubricating agent such as palmitic acid and stearyl alcohol; an organic and inorganic antimicrobial agent; and an anti-static agent. According to the necessary, a plurality of these can be used in combination.

The resin composition of the present invention can be produced by a known method such as a method including the steps of mechanically mixing a predetermined amount of each component using a mixing apparatus such as Henschel mixer and tumbler, melt-kneading a resin sufficiently at an appropriate temperature for the resin, for example, temperature of 200 to 260˚C, using a monoaxial, biaxial screw extruder or Banbury mixer, or the like, and pellitizing by granulation.

The method of producing the lighting unit cover (a molded product of the resin composition described above) of the present invention is not particularly limited. After molding into sheet form by a known method such as injection molding, extrusion molding, blow molding, and compression molding or by extrusion molding, a thermal molding method including vacuum molding, pressure molding, vacuum pressure molding, and press molding can be used to obtain a desired shape.

The lighting unit cover of the present invention may a light for cover to be installed to cover the front side of a lamp or may have a hollow tube shape to have a light source installed therein. In addition, various shapes such as a square shape, horseshoe shape, and a glove shape are also possible. The thickness of the molded product is preferably 4 mm or less, and more preferably 3 mm or less. Practically, it is 0.1 mm or more.

The lighting unit cover of the present invention may be transparent, or may be milk white and a type which softens a lamp image by softening light from the lamp by diffusion of light. Thus, the whole light transmittance of the lighting unit cover of the present invention is preferably 40% or more, and more preferably 50% or more. When the whole light transmittance is 40% or more, excessive darkness is prevented, and therefore light from the lamp can be appropriately relaxed. Furthermore, the value obtained by subtracting the smallest transmittance value within 550 nm to 600 nm from the largest transmittance value within 430 nm to 480 nm is preferably 15% or less, more preferably 14% or less, still more preferably 8% or less, and most preferably 6% or less.

The lighting unit cover can be favorably used for a cover for various kinds of lighting instruments, for example, a ceiling light, a pendant-shaped light, a light for sink, a light for bathroom, a chandelier, a desk or floor lamp, a bracket, a paper-covered lamp stand a light for garage, a light under eaves, a gatepost light, a porch light, a garden light, an entrance light, footlights, a light for stairs, a guidance light, a security light, a down light, a base light, a signboard with lights, and a sign light, and a cover for lighting instruments for a vehicle such as an automobile and a motor bicycle.
In addition, by applying the resin composition containing the UV agent of formula (1) to a lens for glasses or the like, it is possible to indirectly cut off UV light from the lighting instrument. In such the case, it is expected to have an effect of saving eye strain or the like.

The lighting unit cover of the present invention can be used for various light sources. Preferably, it is a fluorescent light or HID lamps (High Intensity Discharge Lamps: a generic name of a metal halide lamp, a high pressure sodium lamp and a mercury lamp which emits light by discharge in metal vapor, and also referred to as high luminance discharge lamp (a high luminance discharge light)). The fluorescent lamp is most preferable.

The lighting unit cover of the present invention is used for the application of removing light having a wavelength to which an insect is apt to be attracted.
The lighting unit cover of the present invention can effectively cut off light having wavelength to which an insect such as a flying insect is apt to be attracted, including light in the long-wavelength range, and thus, can make it difficult for the insect to approach. Further, by using the resin molded product which is high in the degree of a feeling of transparency and has no yellow tint, the appearance of the lighting unit cover is not damaged at the time of the lighting unit turned on. Further, the lighting unit cover is excellent in light fastness, no coloration of yellow occurs even when the lighting unit cover is used for a long period of time.
The present invention is described in more detail based on the following examples, but the invention is not intended to be limited thereby.

EXAMPLES

Example and comparative example

(Preparation of ultraviolet absorbent sample)

As shown in the following Table 1, the ultraviolet absorbents A and B as well as the compound C were combined with each other. As the bluing agent D, "Quinizarin Blue" (trade name) manufactured by Tokyo Chemical Industry Co., Ltd., whose generic name is Solvent Violet 13 as described above, was added to prepare the ultraviolet absorbent samples 0 to 18. In Table 1 below, the ratio among the ultraviolet absorbent A, the ultraviolet absorbent B, and the compound C (A:B:C) is represented in molar ratio. In the samples 1 to 15, the bluing agent D was added in an amount of 0.173 parts by mass, relative to 100 parts by mass of the compound A. No bluing agent was added to the sample 0 and the samples 14 to 18.
Table 1

| Sample No. | Ultraviolet absorbent A | Ultraviolet absorbent B | Compound C | A:B:C | Bluing agent D |
|---|---|---|---|---|---|
| 0 | (1) | None | None | 1:0:0 | None |
| 1 | (1) | None | None | 1:0:0 | Added |
| 2 | (1) | II-2 | TII-25 | 1:2:0.06 | Added |
| 3 | (1) | II-10 | TII-25 | 1:1.5:0.06 | Added |
| 4 | (1) | II-10 | TII-25 | 1:2:0.06 | Added |
| 5 | (1) | II-10 | None | 1:3:0 | Added |
| 6 | (1) | III-1 | TII-25 | 1:2:0.06 | Added |
| 7 | (17) | III-1 | TII-25 | 1:2:0.5 | Added |
| 8 | (1) | III-3 | TI-53 | 1:2:0.06 | Added |
| 9 | (1) | III-4 | TI-53 | 1:2:0.06 | Added |
| 10 | (17) | IV-2 | TII-25 | 1:2:0.06 | Added |
| 11 | (1) | IV-3 | TII-25 | 1:2:0.06 | Added |
| 12 | (1) | V-1 | None | 1:2:0 | Added |
| 13 | (17) | V-1 | TII-25 | 2:1:0.06 | Added |
| 14 | None | III- 1 | None | 0:1:0 | None |
| 15 | None | III- 1 | TII-25 | 0:1:0.01 | None |
| 16 | X-1 | None | None | 1:0:0 | None |
| 17 | X-1 | II-10 | None | 1:2:0.06 | None |
| 18 | X-1 | II-10 | TII-25 | 1:0:0.06 | None |

The maximum absorption wavelength and the absorbance at 320 nm relative to that at the maximum absorption wavelength of each compound used as the ultraviolet absorbent A or B were determined by preparing a solution in ethyl acetate of each compound at a concentration of approximately 5 × $10^{-5}$ mol·$dm^{-3}$ and measuring the UV spectrum of the solution in a 1-cm quartz cell by using a spectrophotometer UV-3600 (trade name) manufactured by Shimadzu Corporation. The maximum absorption wavelength, the half value width, and the absorbance at 320 nm relative to that at the n maximum absorption wavelength were calculated form the spectral chart obtained. Results are summarized in the following Table 2. All of the ultraviolet absorbent B satisfied the condition A.

Table 2

| Compound | Classification of ultraviolet absorbent | Classification of B | Maximum Absorption wavelength (nm) | Absorbance at 320 nm relative to that at maximum absorption wavelength (%) |
|---|---|---|---|---|
| (1) | (A) | - | 376 | 33 |
| (17) | (A) | - | 375 | 50 |
| II-2 | (B) | B-1 | 350 | 83 |
| II-3 | (B) | B-1 | 339 | 77 |
| II-10 | (B) | B-1 | 349 | 84 |
| III-1 | (B) | B-1 | 346 | 58 |
| III-3 | (B) | B-2 | 288 | 50 |
| III-4 | (B) | B-2 | 274 | 41 |
| III-5 | (B) | B-1 | 348 | 61 |
| IV-2 | (B) | B-1 | 346 | 50 |
| IV-3 | (B) | B-2 | 286 | 63 |
| V-1 | (B) | B-1 | 346 | 64 |
| X-1 | Compound corresponding to (A) | - | 371 | 25 |

The compound X-1 is a compound known in JP-A-2005-206830 that it can effectively cut off the long-wavelength ultraviolet light up to 410 nm. The structure of the compound X-1 is shown below.

(X-1)

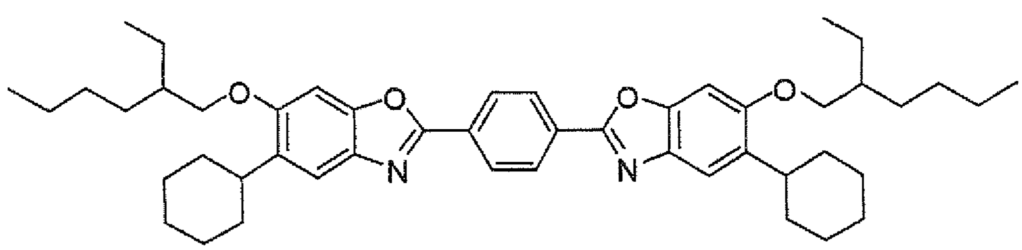

(Preparation of polycarbonate sample plates 200 to 218)

To 3 kg of a polycarbonate resin (PC) [trade name: PANLITE, manufactured by Teijin Chemicals Ltd.], the sample 0 was added to have 3 g of the ultraviolet absorbent A, and stirred for one hour by a stainless tumbler. The mixture was melt and mixed at 300°C using a biaxial extruding and kneading apparatus. According to a common method,

a pellet for molding was produced. By using this pellet, a sample plate 200 having a thickness of 1 mm was produced by an injection molding machine.
Except that the sample 0 was change to the samples 1 to 13, respectively, sample plates 201 to 213 were produced, respectively, in the same manner as the production of the sample plate 200. Sample plates 216 to 218 were produced, respectively, in the same manner as the production of the sample plate 200, except that the samples 16 to 18 were added, respectively, in an amount to have 9 g of the ultraviolet absorbent A, instead of adding the sample 0 in an amount to have 3 g of the ultraviolet absorbent A. Sample plate 214 and sample plate 215 were produced, respectively, in the same manner as the production of the sample plate 200, except that the sample 14 or the sample 15 was added in an amount to have 3 g of the ultraviolet absorbent B.
Observing the thus-obtained sample plates with naked eyes, all of them had almost no color. The transmittance spectra of the thus-obtained sample plates 200 and 201 are given in Figs. 2 and 3, respectively.
In the sample plates within the scope of the present invention, the value obtained by subtracting the smallest transmittance value in 550 nm to 600 nm from the largest transmittance value in 430 nm to 480 nm was 15% or less and the whole light transmittance was 40% or more.

(Preparation of polyethylene terephthalate sample plates 300 to 318)

Sample plates 300 to 318 were produced, respectively, in the same manner as the production of the polycarbonate resin plate in the example 2, except that the polycarbonate resin was changed to a polyethylene terephthalate resin (PET) and the temperature of biaxially extruding and kneading was changed from 300˚C to 270˚C. Observing the thus-obtained sample plates with naked eyes, all of them had almost no color.

<Evaluation>

(Evaluation of light fastness of sample plate)

With respect to the obtained sample plates 200 to 218 and 300 to 318, by using U-4100 spectrophotometer (trade name, manufactured by Hitachi High-Technologies Corporation), measurement of transmittance was conducted. As a result, it was found that the sample plates 200 to 213, 216 to 218, 300 to 313 and 316 to 318 sufficiently cut off the long-wavelength ultraviolet light up to 410 nm. On the other hand, the sample plates 214, 215, 314 and 315 did not cut off light up to 410 nm and the long-wavelength ultraviolet light was insufficiently cut off.
Then, light illumination was carried out for 150 hours with luminance of 170,000 lux using a Xenon lamp (manufactured by Eagle Engineering). After that, by using U-4100 spectrophotometer (trade name, manufactured by Hitachi High-Technologies Corporation), measurement of b* value was carried out. With the each measured value, Δb* was calculated based on the following equation.

$$\Delta b^* = (b^* \text{ value after the Xenon illumination}) - (b^* \text{ value before the Xenon illumination})$$

Herein, b* value is defined by JIS Z 8729.
Regarding the values obtained, those having Δb* of less than 4 were evaluated as o, those having Δb* of 4 or more and less than 5 were evaluated as Δ, and those having Δb* of 5 or more were evaluated as x. The results are shown in Table 3.
Table 3

| Sample plate | Light fastness | | Remarks |
|---|---|---|---|
| | Δb* | Evaluation | |
| 200 | 3.8 | ○ | This invention |
| 201 | 3.7 | ○ | This invention |
| 202 | 3.8 | ○ | This invention |
| 203 | 3.8 | ○ | This invention |

(continued)

| Sample plate | Light fastness | | Remarks |
|---|---|---|---|
| | Δb* | Evaluation | |
| 204 | 3.8 | ○ | This invention |
| 205 | 3.8 | ○ | This invention |
| 206 | 3.7 | ○ | This invention |
| 207 | 3.7 | ○ | This invention |
| 208 | 3.8 | ○ | This invention |
| 209 | 3.8 | ○ | This invention |
| 210 | 3.8 | ○ | This invention |
| 211 | 3.7 | ○ | This invention |
| 212 | 3.8 | ○ | This invention |
| 213 | 3.8 | ○ | This invention |
| 214 | 4.1 | Δ | Comparative example |
| 215 | 4.1 | Δ | Comparative example |
| 216 | 5.1 | × | Comparative example |
| 217 | 5.1 | × | Comparative example |
| 218 | 5.2 | × | Comparative example |

As is apparent from the results of Table 3, the molded plates obtained by using the resin compositions which constituted the lighting unit cover of the present invention showed very small degree of yellowing by Xenon illumination (sample plates 200 to 213). On the other hand, the sample plates which did not contain the ultraviolet absorbent A of the present invention and did not sufficiently cut off the long-wavelength ultraviolet light (sample plates 214 and 215) and the sample plates which contained the ultraviolet absorbent A that was not included in the present invention (sample plates 216 to 218) exhibited significant yellowing.
Thus, the lighting unit cover of the present invention does not damage the appearance of the light at the time of lighting, is excellent in light fastness, and no coloration of yellow occurs even when it is used for a long period of time.

(Evaluation of the effect of preventing a flying insect from being attracted)

At the center of a room with a size of 10 m × 10 m, a light box NEW5000 (trade name, manufactured by FUJIFILM Corporation) was lighted as a light source. The sample plates 300 to 318 were installed to cover the light illuminating surface of the light box and used as a lighting unit cover. In addition, at the height of 20 cm above the light illuminating surface, an adhesive sheet having a size of 30 cm × 30 cm was placed. Five hundred Chironomidae were released in the room, and a property of attracting flying insects was evaluated based on the number of the insects attracted by light and trapped in the adhesive sheet. When the number of the trapped insects was less than 50, it was evaluated as ○ while the number of 50 or more was evaluated as ×. In addition, in the case where any one of the sample plates was used as the lighting unit cover, it was found that the appearance of the light was not damaged since the lighting unit cover was colorless. The results are shown in Table 4.
Table 4

| Sample plate | Preventing property of attracting insect | | Remarks |
|---|---|---|---|
| | Number of insect trapped | Evaluation | |
| 300 | 47 | ○ | This invention |
| 301 | 42 | ○ | This invention |
| 302 | 38 | ○ | This invention |

(continued)

| Sample plate | Preventing property of attracting insect | | Remarks |
|---|---|---|---|
| | Number of insect trapped | Evaluation | |
| 303 | 40 | ○ | This invention |
| 304 | 42 | ○ | This invention |
| 305 | 40 | ○ | This invention |
| 306 | 41 | ○ | This invention |
| 307 | 46 | ○ | This invention |
| 308 | 36 | ○ | This invention |
| 309 | 35 | ○ | This invention |
| 310 | 41 | ○ | This invention |
| 311 | 44 | ○ | This invention |
| 312 | 39 | ○ | This invention |
| 313 | 38 | ○ | This invention |
| 314 | 224 | × | Comparative example |
| 315 | 215 | × | Comparative example |
| 316 | 41 | ○ | Comparative example |
| 317 | 49 | ○ | Comparative example |
| 318 | 38 | ○ | Comparative example |

(Results of property of attracting insect)

As is apparent from the results of Table 4, the lighting unit cover of the present invention (i.e., a molded plate obtained by using the resin composition) was able to prevent the attraction of flying insects by light (sample plates 300 to 313). On the other hand, the sample plates which did not contain the ultraviolet absorbent A of the present invention and did not sufficiently cut off the long-wavelength ultraviolet light showed insufficient effect of preventing the attraction of flying insects (sample plates 314 and 315). The sample plates 316 to 318 were able to prevent the attraction of the flying insects by light since it contained a known compound to cut off the long-wavelength ultraviolet light up to 410 nm. However, since light fastness was poor as described above, a significant coloration of yellow was observed.
Thus, the lighting unit cover of the present invention effectively cuts off light having the wavelength to which an insect such as a flying insect is apt to be attracted, including light in the long-wavelength range, and thus can make it difficult for the insects to approach.

As is apparent from the above results, the ultraviolet absorbent A used in the present invention has an absorbing ability of ultraviolet light in broad wavelength range, cuts off ultraviolet light in long-wavelength rang, and is excellent in a colorless property. Thus, the present invention can provide a lighting unit cover being capable of preventing the gathering of flying insects without damaging the appearance of the light at the time of lighting, and is excellent in light fastness.

Having described our invention as related to the present embodiments, it is our intention that the invention not be limited by any of the details of the description, unless otherwise specified, but rather be construed broadly within its spirit and scope as set out in the accompanying claims.

This application claims priority on Patent Application No. 2008-232472 filed in Japan on September 10, 2008, which is entirely herein incorporated by reference.

## Claims

1. A lighting unit cover, comprising a resin composition including ultraviolet absorbent A, the ultraviolet absorbent A being a compound represented by formula (1):

Formula (1)

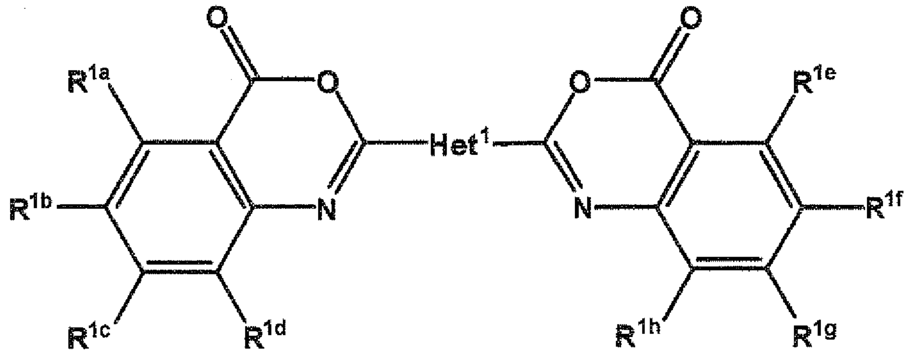

wherein, $Het^1$ represents a bivalent five- or six-membered aromatic heterocyclic residue; the aromatic heterocyclic residue may have a substitutent; and
$R^{1a}$, $R^{1b}$, $R^{1c}$, $R^{1d}$ $R^{1e}$, $R^{1f}$, $R^{1g}$ and $R^{1h}$ each independently represent a hydrogen atom or a monovalent substituent.

**2.** The lighting unit cover according to Claim 1, wherein the compound represented by formula (1) is a compound represented by formula (2):

Formula (2)

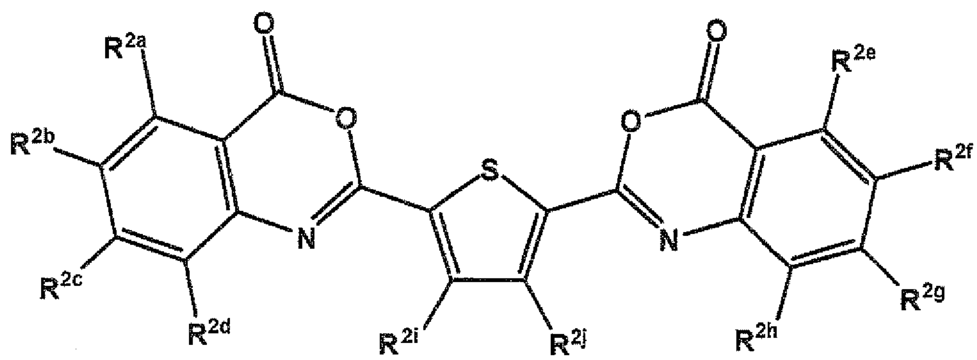

wherein, $R^{2a}$, $R^{2b}$, $R^{2c}$, $R^{2d}$, $R^{2e}$, $R^{2f}$, $R^{2g}$ and $R^{2h}$ have the same meanings as those of $R^{1a}$, $R^{1b}$, $R^{1c}$, $R^{1d}$, $R^{1e}$, $R^{1f}$, $R^{1g}$ and $R^{1h}$ in formula (1), respectively, and $R^{2i}$ and $R^{2j}$ each independently represent a hydrogen atom or a monovalent substituent.

**3.** The lighting unit cover according to Claim 1 or 2,
wherein the resin composition includes ultraviolet absorbent B, and
wherein the ultraviolet absorbent B is a compound, in which an absorbance at 320 nm is 20% or more of an absorbance at a maximum absorption wavelength within a range from 270 to 400 nm and the maximum absorption wavelength is 380 nm or less.

**4.** The lighting unit cover according to any one of Claims 1 to 3, wherein the resin composition contains 0.00001 to 1 mass part of bluing agent D, relative to 100 mass parts of the ultraviolet absorbent A.

**5.** The lighting unit cover according to any one of Claims 1 to 4, wherein the resin composition includes at least one kind of compound C represented by any one of formulae (TS-I) to (TS-V):

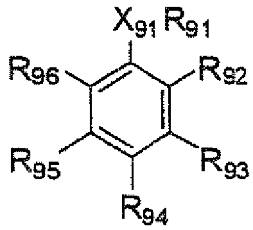

(TS-I)

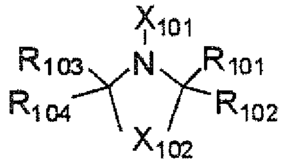

(TS-II)

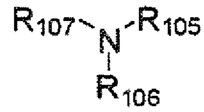

(TS-III)

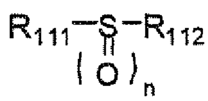

(TS-IV)

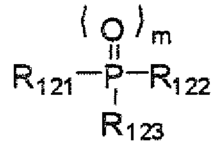

(TS-V)

wherein, in formula (TS-I), $R_{91}$ represents a hydrogen atom, an alkyl group, an alkenyl group, an aryl group, an heterocyclic group, an acyl group, an alkyloxcarbonyl group, an alkenyloxycarbonyl group, an aryloxycarbonyl group, an alkyl sulfonyl group, an arylsulfonyl group, a phosphinotolyl group, a phosphinyl group, or $-Si(R_{97})(R_{98})(R_{99})$, in which $R_{97}$, $R_{98}$ and $R_{99}$, which may be the same as or different from each other, each independently represent an alkyl group, an alkenyl group, an aryl group, an alkoxy group, an alkenyloxy group, or an aryloxy group; $-X_{91}-$ represents -O-, -S-, or $-N(-R_{100})-$, in which $R_{100}$ has the same meaning as that of $R_{91}$; $R_{92}$, $R_{93}$, $R_{94}$, $R_{95}$ and $R_{96}$, which may be the same as or different from each other, each independently represent a hydrogen atom or a substituent; $R_{91}$ and $R_{92}$, $R_{100}$ and $R_{96}$, and $R_{91}$ and $R_{100}$, respectively, may bind to each other to form any of 5- to 7-membered rings; $R_{92}$ and $R_{93}$, and $R_{93}$ and $R_{94}$, respectively, may bind together with each other to form any of 5- to 7-membered rings, a spiro ring or a bicyclo ring; and all of $R_{91}$, $R_{92}$, $R_{93}$, $R_{94}$, $R_{95}$, $R_{96}$ and $R_{100}$ cannot simultaneously represent a hydrogen atom, respectively, and the total number of carbon atoms of $R_{91}$, $R_{92}$, $R_{93}$, $R_{94}$, $R_{95}$, $R_{96}$ and $R_{100}$ is 10 or more;
wherein, in formula (TS-II), $R_{101}$, $R_{102}$, $R_{103}$ and $R_{104}$ each independently represent a hydrogen atom, an alkyl group, or an alkenyl group; $R_{101}$ and $R_{102}$, and $R_{103}$ and $R_{104,}$ respectively, may bind to each other to form any of 5- to 7-membered rings; $X_{101}$ represents a hydrogen atom, an alkyl group, an alkenyl group, an alkyloxy group, an alkenyloxy group, an alkyloxycarbonyl group, an alkenyloxycarbonyl group, an aryloxycarbonyl group, an acyl group, an acyloxy group, an alkyloxycarbonyloxy group, an alkenyloxycarbonyloxy group, an aryloxycarbonyloxy group, an alkylsulfonyl group, an alkenylsulfonyl group, an arylsulfonyl group, an alkylsulfinyl group, an alkenylsulfinyl group, an arylsulfinyl group, a sulfamoyl group, a carbamoyl group, a hydroxy group, or an oxy radical group; and $X_{102}$ represents a group of non-metal atoms necessary for forming any of 5- to 7-membered rings;
wherein, in formula (TS-III), $R_{105}$ and $R_{106}$ each independently represent a hydrogen atom, an aliphatic group, an acyl group, an aliphatic oxycarbonyl group, an aromatic oxycarbonyl group, an aliphatic sulfonyl group, or an aromatic sulfonyl group; $R_{107}$ represents an aliphatic group, an aliphatic oxy group, an aromatic oxy group, an aliphatic thio group, an aromatic thio group, an acyloxy group, an aliphatic oxyaarbonyloxy group, an aromatic oxycarbonyloxy group, a substituted amino group, a heterocyclic group, or a hydroxyl group; $R_{105}$ and $R_{106}$, $R_{106}$ and $R_{107}$, and $R_{105}$ and $R_{107}$, respectively, may bind to each other to form any of 5- to 7-membered rings except of forming a 2,2,6,6-tetraalkylpiperidine residue; and both $R_{105}$ and $R_{106}$ are not hydrogen atoms at the same time, and the total number of carbon atoms of $R_{105}$ and $R_{106}$ is 7 or more;
wherein, in formula (TS-IV), $R_{111}$ and $R_{112}$, each independently represent an aliphatic group; $R_{111}$ and $R_{112}$ may bind to each other to form any of 5- to 7-membered rings; n represents 0, 1 or 2; and the total number of carbon

atoms of $R_{111}$ and $R_{112}$ is 10 or more; and
wherein, in formula (TS-V), $R_{121}$ and $R_{122}$ each independently represent an aliphatic oxy group or an aromatic oxy group; $R_{123}$ represents an aliphatic group, an aromatic group, an aliphatic oxy group, or an aromatic oxy group; m represents 0 or 1; $R_{121}$ and $R_{122}$, and $R_{121}$ and $R_{123}$, respectively, may bind to each other to form any of 5-to 8-membered rings; and the total number of carbon atoms of $R_{121}$, $R_{122}$ and $R_{123}$ is 10 or more.

**6.** The lighting unit cover according to any one of Claims 1 to 5, wherein a content of the ultraviolet absorbent A in the resin composition is 0.01 to 20 mass parts, relative to 100 mass parts of the resin.

**7.** The lighting unit cover according to any one of Claims 3 to 6, wherein the molar ratio of the ultraviolet absorbent A and the ultraviolet absorbent B in the resin composition is within a range of 1: 10 to 10: 1.

**8.** The lighting unit cover according to any one of Claims 1 to 7, wherein the resin composition includes at least one kind of an acrylic resin, a polycarbonate resin, a polyester resin, and a polyolefin resin.

**9.** The lighting unit cover according to any one of Claims 1 to 8, wherein a value obtained by subtracting a smallest transmittance value in 550 nm to 600 nm from a largest transmittance value in 430 nm to 480 nm is 15% or less of the largest transmittance value in 430 nm to 480 nm.

**10.** The lighting unit cover according to any one of Claims 1 to 9, wherein an entire light transmittance is 40% or more.

**11.** The lighting unit cover according to any one of Claims 1 to 10, wherein the cover is adapted to an application for removing light defined in the wavelength that is apt to attract an insect.

{Fig. 1}

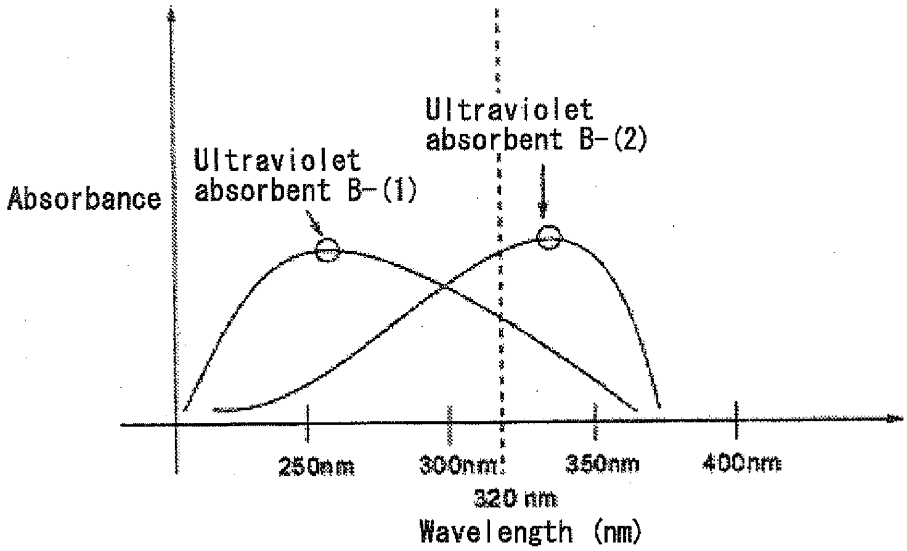


{Fig. 2}

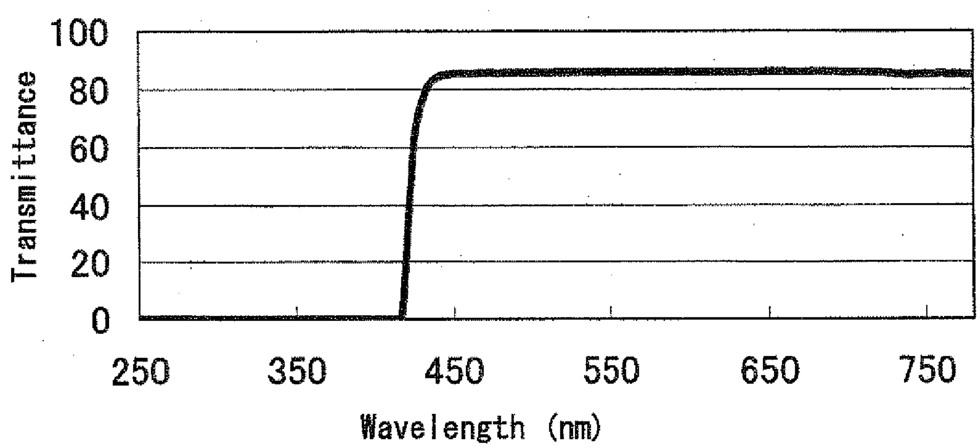


{Fig. 3}

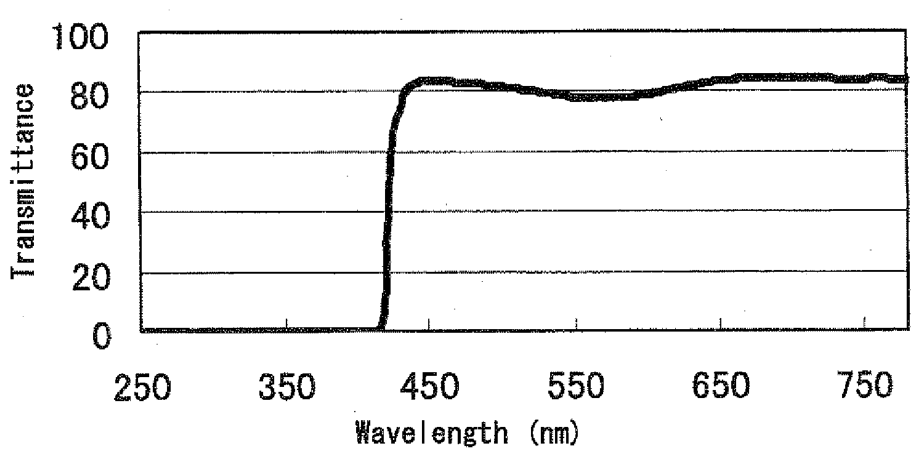

**INTERNATIONAL SEARCH REPORT**

International application No.
PCT/JP2009/065691

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C08L101/00, C08K5/00, C08K5/357, C08K5/45, C09K3/00, F21V3/04, F21V9/06, C07D249/20, C07D251/22, C07D265/22, C07D413/14, F21Y101/00, F21Y103/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-195830 A (Fujifilm Corp.), 28 August 2008 (28.08.2008), entire text (Family: none) | 1-11 |
| A | JP 2002-225195 A (Fuji Photo Film Co., Ltd.), 14 August 2002 (14.08.2002), entire text; all drawings (Family: none) | 1-11 |
| A | JP 2007-119613 A (Tsutsunaka Plastic Industry Co., Ltd.), 17 May 2007 (17.05.2007), entire text (Family: none) | 1-11 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>02 December, 2009 (02.12.09) | Date of mailing of the international search report<br>15 December, 2009 (15.12.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/065691

C (Continuation). DOCUMENTS CONSIDERED TO BE RELEVANT

| Category* | Citation of document, with indication, where appropriate, of the relevant passages | Relevant to claim No. |
|---|---|---|
| E,X | WO 2009/122968 A1 (Fujifilm Corp.),<br>08 October 2009 (08.10.2009),<br>entire text; all drawings<br>(Family: none) | 1-11 |
| P,X | JP 2008-273927 A (Fujifilm Corp.),<br>13 November 2008 (13.11.2008),<br>entire text<br>(Family: none) | 1-11 |

Form PCT/ISA/210 (continuation of second sheet) (April 2007)

INTERNATIONAL SEARCH REPORT

International application No.
PCT/JP2009/065691

Continuation of A. CLASSIFICATION OF SUBJECT MATTER
(International Patent Classification (IPC))

*C08L101/00*(2006.01)i, *C08K5/00*(2006.01)i, *C08K5/357*(2006.01)i, *C08K5/45*(2006.01)i, *C09K3/00*(2006.01)i, *F21V3/04*(2006.01)i, *F21V9/06*(2006.01)i, *C07D249/20*(2006.01)n, *C07D251/22*(2006.01)n, *C07D265/22*(2006.01)n, *C07D413/14*(2006.01)n, *F21Y101/00*(2006.01)n, *F21Y103/00*(2006.01)n

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001161253 A
- JP 5339033 A
- JP 5345639 A
- JP 6056466 A
- JP 2003177235 A
- JP 2005517787 T
- JP 7285927 A
- JP 2005206830 A
- JP 2000264879 A
- JP 2003155375 A
- JP 1005345 B
- JP 61189530 A
- EP 27242 A
- JP 2037575 B
- JP 2050457 B
- JP 5067220 B
- JP 5070809 B
- JP 6019534 B
- JP 62227889 A
- JP 62244046 A
- JP 2066541 A
- JP 2139544 A
- JP 2194062 A
- JP 2212836 A
- JP 3200758 A
- JP 3048845 A
- JP 3266836 A
- JP 3969440 A
- JP 4330440 A
- JP 5297541 A
- JP 6130602 A
- WO 91111749 A
- DE 4008785 A1
- US 4931382 A
- EP 203746 B1
- EP 264730 B1
- JP 2032298 B
- JP 3039296 B
- JP 3040373 B
- JP 2049762 A
- JP 2208653 A
- JP 2217845 A
- US 4906555 A
- EP 309400 A2
- EP 309401 A1
- EP 309402 A1
- JP 6097332 B
- JP 6097334 B
- JP 2148037 A
- JP 2150841 A
- JP 2181145 A
- JP 4350854 A
- JP 5061166 A
- JP 2044052 B
- JP 3048242 A
- JP 5323545 A
- JP 6148837 A
- US 4933271 A
- JP 3025437 A
- JP 3142444 A
- US 4749645 A
- US 4980275 A
- US 5807932 A
- US 4259467 A
- US 4355147 A
- JP 2008232472 A


### Non-patent literature cited in the description


- *Bioorganic & Medicinal Chemistry,* 2000, vol. 8, 2095-2103
- *Bioorganic & Medicinal Chemistry Letters,* 2003, vol. 13, 4077-4080
- *Fine Chemical,* May 2004, 28-38
- Kobunshi-yo Kinoseitenkazai no Shin Tenkai. Toray Research Center, 1999, 96-140
- Kobunshi Tenkazai no Kaihatsu to kankyo Taisaku. C M C Shuppan, 2003, 54-64
- Kobunshi no Rekka · Henshoku mekanizumu to sono Anteika Gijutsu-Nohausyu. Kabushiki kaisha Gijutsu Jyoho Kyokai, 2006